# NEW EUROPEAN PATENT SPECIFICATION

(11) **EP 2 298 301 B2**
(45) Date of publication and mention of the opposition decision: **16.11.2022**
(45) Mention of the grant of the patent: 04.07.2018
(21) Application number: 10181796.3
(22) Date of filing: 06.12.2002
(51) Int. Cl.: A61K 31/47, A61K 31/4738, A61K 31/63, A61K 38/17, A61K 31/00, C07K 14/47, C07K 14/505, A61P 7/06, G01N 33/74, C12N 15/09, C12Q 1/34, C12Q 1/56, A61K 31/472, A61K 31/4745, A61K 31/17, A61K 31/4375, A61K 31/44, A61K 31/4418, A61K 31/496

(54) **MEDICAMENTS FOR TREATING ANEMIA ASSOCIATED WITH KIDNEY DISEASE**
MEDIKAMENTE ZUR BEHANDLUNG DER ANÄMIE IM ZUSAMMENHANG MIT NIERENERKRANKUNGEN
MÉDICAMENTS POUR LE TRAITEMENT D'UNE ANÉMIE ASSOCIÉE À UNE MALADIE RÉNALE

(30) Priority: 06.12.2001 US 337082 P; 16.01.2002 US 349659 P; 25.02.2002 US 359683 P; 05.06.2002 US 386488 P
(43) Date of publication of application: 23.03.2011
(62) Divisional of application: 02805548.1
(73) Proprietor: Fibrogen, Inc., San Francisco, CA 94158 (US)
(72) Inventor: KLAUS, Stephen J., San Francisco, CA 94109 (US); LIN, Al Y., Castro Valley, CA 94552 (US); NEFF, Thomas B., Atherton, CA 94027 (US); WANG, Qingjian, Davis, CA 95616 (US); GUENZLER-PUKALL, Volkmar, San Leandro, CA 94577 (US); AREND, Michael P., San Mateo, CA 94403 (US); FLIPPIN, Lee A., Woodside, CA 94062 (US)
(74) Representative: Carpmaels & Ransford LLP

(56) References cited:
- EP-A1- 0 765 871
- EP-A1- 0 765 871
- EP-A1- 1 633 333
- EP-A2- 0 911 340
- EP-A2- 0 911 340
- EP-A2- 1 463 823
- EP-B1- 0 650 960
- EP-B1- 0 650 961
- WO-A1-01/64652
- WO-A1-97/41103
- WO-A1-97/41103
- WO-A2-02/074981
- WO-A2-03/049686
- WO-A2-03/053997
- WO-A2-2008/002576
- CA-A1- 2 134 866
- DE-A1- 10 010 423
- DE-A1- 10 010 423
- DE-A1- 10 010 425
- DE-A1- 10 010 426
- DE-A1- 10 010 430
- DE-A1- 10 010 430
- US-A- 5 607 954
- US-A- 5 610 172
- US-A- 5 620 995
- US-A- 5 620 996
- US-A- 5 658 933
- US-A- 5 719 164
- US-A- 5 726 305
- US-A- 6 020 350
- US-A- 6 093 730
- BRUICK RICHARD K ET AL: "A conserved family of prolyl-4-hydroxylases that modify HIF", SCIENCE, AMERICAN ASSOCIATION FOR THE ADVANCEMENT OF SCIENCE, WASHINGTON, DC; US, vol. 294, no. 5545, 9 November 2001 (2001-11-09), pages 1337-1340, XP002433273, ISSN: 0036-8075, DOI: 10.1126/SCIENCE.1066373
- P. JAAKKOLA ET AL: "Targeting of HIF-alpha to the von Hippel-Lindau Ubiquitylation Complex by O2-Regulated Prolyl Hydroxylation", SCIENCE, vol. 292, no. 5516, 20 April 2001 (2001-04-20), pages 468-472, XP55040289, ISSN: 0036-8075, DOI: 10.1126/science.1059796
- EPSTEIN A C R ET AL: "C. ELEGANS EGL-9 AND MAMMALIAN HOMOLOGS DEFINE A FAMILY OF DIOXYGENASES THAT REGULATE HIF BY PROLYL HYDROXYLATION", CELL, CELL PRESS, US, vol. 107, no. 1, 5 October 2001 (2001-10-05), pages 43-54, XP008046407, ISSN: 0092-8674, DOI: 10.1016/S0092-8674(01)00507-4
- IVAN MIRCEA ET AL: "HIFalpha targeted for VHL-mediated destruction by proline hydroxylation: Implications for O2 sensing", SCIENCE, AMERICAN ASSOCIATION FOR THE ADVANCEMENT OF SCIENCE, WASHINGTON, DC; US, vol. 292, no. 5516, 20 April 2001 (2001-04-20), pages 464-468, XP002207795, ISSN: 0036-8075, DOI: 10.1126/SCIENCE.1059817
- DATABASE MEDLINE [Online] US NATIONAL LIBRARY OF MEDICINE (NLM), BETHESDA, MD, US; October 1996 (1996-10), JELKMANN W ET AL: "Erythropoietin in the control of red cell production.", XP002684805, Database accession no. NLM8931850 & ANNALS OF ANATOMY = ANATOMISCHER ANZEIGER : OFFICIAL ORGAN OF THE ANATOMISCHE GESELLSCHAFT OCT 1996 LNKD- PUBMED:8931850, vol. 178, no. 5, October 1996 (1996-10), pages 391-403, ISSN: 0940-9602
- DATABASE MEDLINE [Online] US NATIONAL LIBRARY OF MEDICINE (NLM), BETHESDA, MD, US; July 1997 (1997-07), WENGER R H ET AL: "Oxygen(es) and the hypoxia-inducible factor-1.", XP002684806, Database accession no. NLM9278140 & BIOLOGICAL CHEMISTRY JUL 1997 LNKD- PUBMED:9278140, vol. 378, no. 7, July 1997 (1997-07), pages 609-616, ISSN: 1431-6730
- MASSON N ET AL: "Independent function of two destruction domains in hypoxia-inducible factor-alfa chains activated by prolyl hydroxylaTIOn", EMBO JOURNAL, OXFORD UNIVERSITY PRESS, SURREY, GB, vol. 20, no. 18, 1 January 2001 (2001-01-01), pages 5197-5206, XP002253782, ISSN: 0261-4189, DOI: 10.1093/EMBOJ/20.18.5197
- KALLIO PEKKA J ET AL: "Regulation of the hypoxia-inducible transcription factor 1alpha by the ubiquitin-proteasome pathway", JOURNAL OF BIOLOGICAL CHEMISTRY, AMERICAN SOCIETY FOR BIOCHEMISTRY AND MOLECULAR BIOLOGY, US, vol. 274, no. 10, 5 March 1999 (1999-03-05), pages 6519-6525, XP002146028, ISSN: 0021-9258, DOI: 10.1074/JBC.274.10.6519
- S. SALCEDA: "Hypoxia-inducible Factor 1alpha (HIF-1alpha ) Protein Is Rapidly Degraded by the Ubiquitin-Proteasome System under Normoxic Conditions. ITS STABILIZATION BY HYPOXIA DEPENDS ON REDOX-INDUCED CHANGES", JOURNAL OF BIOLOGICAL CHEMISTRY, vol. 272, no. 36, 5 September 1997 (1997-09-05), pages 22642-22647, XP55040278, ISSN: 0021-9258, DOI: 10.1074/jbc.272.36.22642
- WARSHAKOON N C ET AL: "Design and synthesis of substituted pyridine derivatives as HIF-1alpha prolyl hydroxylase inhibitors", BIOORGANIC & MEDICINAL CHEMISTRY LETTERS, PERGAMON, AMSTERDAM, NL, vol. 16, no. 21, 1 November 2006 (2006-11-01), pages 5616-5620, XP027966145, ISSN: 0960-894X [retrieved on 2006-11-01]
- HOFER T ET AL: "Oxygen sensing, HIF-1[alpha] stabilization and potential therapeutic strategies", PFLUEGERS ARCHIV: EUROPEAN JOURNAL OF PHYSIOLOGY, SPRINGER VERLAG, BERLIN, DE, vol. 443, no. 4, 30 April 2014 (2014-04-30), pages 503-507, XP036392387, ISSN: 0031-6768, DOI: 10.1007/S00424-001-0759-8 [retrieved on 2014-04-30]
- English translation of DE 100 10 430 A1 (Espacenet)
- G: Bruick, R. K. and McKnight, S.L. (November 9, 2001), A conserved family of Prolyl-4-Hydroxylases that modify HIF, Science, 294: 1337-1340.
- Jaakkola et al., ?Targeting of HIF-a to the von Hippel-Lindau Ubiquitylation Complex by O2-Regulated Prolyl Hydroxylation?, Science, 20 April 2001, Vol. 292, 468-472.
- Epstein, A.C.R. et al. (2001). C. elegans EGL-9 and mammalian homologs define a family of dioxygenases that regulate HIF by prolyl hydroxylation, Cell, 107, 43-54
- Ivan et al., ?HIFalpha Targeted for VHL-Mediated Destruction by Proline Hydroxylation: Implications for 02 Sensing", Science, vol 292, 20 April 2001, pages 464-8.
- Jelkmann W. et al: "Erythropoietin in the control of red cell production.", Annals of Anatomy vol. 178, no. 5, October 1996(1996-10), pages 391-403
- Wenger et al: "Oxygen(es), and the hypoxia-inducible factor-1?, Biol. Chem. July 1997, Vol. 378, pp. 609-616
- Masson et al: "Independent function of two destruction domains in hypoxiainducible factor-alfa chains activated by prolyl hydroxylation", EMBO Journal, vol. 20, no. 18, 1 January 2001, pages 5197-5206
- Kallio Pekka et. al.: "Regulation of the hypoxia-inducible transcription factor 1 alpha by the ubiquitin-proteasome pathway", Journal of Biological Chemistry, vol. 274, no. 10, 5, March 1999, pages 6519-6525
- S. Salceda: "Hypoxia-inducible Factor 1 alpha (HIF-1 alpha)Protein is Rapidly Degraded by the Ubiquitin-Proteasome System under Normoxic Conditions. Its Stabilization by hypoxia depends on redox-induced changes", Journal of Biological Chemistry, vol. 272, no. 36, 5 September 1997, pages 22642-22647
- Test data filed by the opponent in the EP '823 case on 05.12.2013 as Annex 022
- Warshakoon et al., ?Design and synthesis of substituted pyridine derivatives as HIF-la prolyl hydroxylase inhibitors", Bioorganic & Medicinal Chermistry Letters, 2006, Vol. 16, 5616-5620
- Poster presentation at Keystone Conference, January 15-20, 2008
- Declaration of J. Patrick Elsevier, Ph.D. and Appendices A to E
- Joint Status Report and Appendices 1,2 and 3
- Opposition Division's decision in EP1633333 (including the main request in that decision, and the list of cited documents)
- Experimental report regarding compounds from D12
- Declaration of Dr. Walkinshaw

### SEQUENCE LISTING

<110> FibroGen Inc.
<120> METHODS FOR INCREASING ENDOGENOUS ERYTHROPOIETIN (EPO)
<130> P055543EP
<150> US 60/349,659
   <151> 2002-01-16
<150> US 60/386,488
   <151> 2002-06-05
<150> US 60/337,082
   <151> 2001-12-06
<150> US 60/359,683
   <151> 2002-02-25
<160> 5
<170> PatentIn version 3.2
<210> 1
   <211> 22
   <212> DNA
   <213> Mus musculus
<400> 1
   acgaacttgc tccccgtcac tg 22
<210> 2
   <211> 20
   <212> DNA
   <213> Mus musculus
<400> 2
   ttctggcccc gaggatgtca 20
<210> 3
   <211> 23
   <212> DNA
   <213> Rattus norvegicus
<400> 3
   cggcggcttt ggtgactcta gat 23
<210> 4
   <211> 22
   <212> DNA
   <213> Rattus norvegicus
<400> 4
   taggcacggc gactaccatc ga 22
<210> 5
   <211> 19
   <212> PRT
   <213> Artificial
<220>
   <223> synthetic peptide
<400> 5
   Asp Leu Asp Leu Glu Ala Leu Ala Pro Tyr Ile Pro Ala Asp Asp Asp
   1 5 10 15
   Phe Gln Leu
<400> 5

## Description

### FIELD OF THE INVENTION

The present invention relates to compounds that inhibit HIF prolyl hydroxylase enzyme activity for use in increasing endogenous erythropoietin in the treatment or prevention of anemia associated with kidney disease.

### BACKGROUND OF THE INVENTION

Erythropoietin (EPO), a naturally occurring hormone, stimulates the production of red blood cells (erythrocytes), which carry oxygen throughout the body. EPO is normally secreted by the kidneys, and endogenous EPO is increased under conditions of reduced oxygen (hypoxia). All types of anemia are characterized by the blood's reduced capacity to carry oxygen, and thus are associated with similar signs and symptoms, including pallor of the skin and mucous membranes, weakness, dizziness, easy fatigability, and drowsiness, leading to a decrease in quality of life. Subjects with severe cases of anemia show difficulty in breathing and heart abnormalities. Anemia is typically associated with a condition in which the blood is deficient in red blood cells or in hemoglobin.

Common causes of anemia include deficiencies of iron, vitamin B₁₂, and folic acid. Anemia can also develop in association with chronic diseases, e.g., in inflammatory disorders, including disorders with consequent inflammatory suppression of marrow. Anemia may be caused by loss of blood, for example, due to accidents, surgery, or gastrointestinal bleeding caused by medications such as aspirin and ibuprofen. Excessive blood loss can also be seen in women with heavy menstrual periods, and in people with stomach ulcers, duodenal ulcers, hemorrhoids, or cancer of the stomach or large intestine.

Various conditions can cause the destruction of erythrocytes (hemolysis), thus leading to anemia. For example, allergic-type reactions to bacterial toxins and various chemical agents such as sulfonamides and benzene can cause hemolysis. Hemolytic anemia is often caused by chemical poisoning, parasites, infection, or sickle-cell anemia. In addition, there are unusual situations in which the body produces antibodies against its own erythrocytes, resulting in hemolysis. Any disease or injury to the bone marrow can cause anemia, since that tissue is the site of erythropoiesis, i.e. erythrocyte synthesis. Irradiation, disease, or various chemical agents can also cause bone marrow destruction, producing aplastic anemia. Cancer patients undergoing chemotherapy often have aplastic anemia. Anemia is also associated with renal dysfunction, the severity of the anemia correlating highly with the extent of the dysfunction. Most patients with renal failure undergoing dialysis suffer from chronic anemia.

In addition to being produced in the kidney, erythropoietin is produced by astrocytes and neurons in the central nervous system (CNS), and EPO and EPO receptors are expressed at capillaries of the brain-periphery interface. Furthermore, systemically administered EPO crosses the blood-brain barrier and reduces neuronal cell loss in response to cerebral and spinal chord ischemia, mechanical trauma, epilepsy, excitotoxins, and neuroinflammation. (Sakanaka (1998) Proc Natl Acad Sci U S A 95:4635-4640; Celik et al. (2002) Proc Natl Acad Sci USA 99:2258-2263; Brines et al. (2000) Proc Natl Acad Sci USA 97:10526-10531; Calapai et al. (2000) Eur J Pharmacol 401:349-356; and Siren et al. (2001) Proc Natl Acad Sci USA 98:4044-404.)

In the late 1980s, Amgen introduced a genetically engineered EPO for the treatment of anemia in chronic renal failure patients. EPO is also administered to cancer patients undergoing radiation and/or chemotherapy, decreasing the need for blood transfusions. EPO is used to treat anemia associated with HIV infection or azidothymidine (AZT) therapy. Although the market for EPO therapy is increasing, future sales are adversely affected by the high cost of the product. In addition, recombinant EPO therapy requires intravenous administration of EPO one to three times per week for up to twelve weeks, a treatment regimen that limits self-administration and is inconvenient for the patient. Further, human serum EPO shows size heterogeneity due to extensive and varied glycosylation not reproduced in any recombinant human EPO.

WO97/41103 describes 3-alkoxypyridine-2-carboxylic acid amide esters and their use in inhibiting collagen biosynthesis and as drugs for treating fibrotic diseases. EP0765871 describes 3-hydroxyquinoline-2-carboxamide derivatives and EP0911340 describes isoquinoline-3-carboxylic acid amide derivatives. Hofer et al. (European Journal of Physiology, vol. 443, no.4, pp. 503-507) describes HIF-1α stabilization and potential therapeutic strategies.

Due to deficiencies in current production and use of recombinant EPO, there remains a need for methods and compounds effective in the treatment of erythropoietin-associated conditions such as anemia, including anemia associated with diabetes, ulcers, kidney failure, cancer, infection, dialysis, surgery, and chemotherapy. Specifically, there is a need in the art for methods and compounds that increase endogenous erythropoietin.

### SUMMARY OF THE INVENTION

The present invention relates to compounds of Formula (I) as defined herein that inhibit HIF prolyl hydroxylase enzyme activity for use in increasing endogenous erythropoietin in treating or preventing anemia associated with kidney disease in a subject. In one aspect, the present disclosure provides a method of increasing endogenous erythropoietin (EPO) in a subject, the method comprising stabilizing the alpha subunit of hypoxia inducible factor (HIFα). In another aspect, the present disclosure provides a method of increasing endogenous EPO in a subject, the method comprising inhibiting hydroxylation of HIFα. In yet another aspect, a method of increasing endogenous EPO in a subject, the method comprising inhibiting 2-oxoglutarate dioxygenase enzyme activity, is disclosed.

The subject can be, in various embodiments, an animal, a mammal, a human, a cell, a tissue, an organ.

In one aspect, the disclosure provides a method of increasing endogenous EPO, the method comprising stabilizing HIFα, wherein the stabilizing takes place *in vivo.*

In particular embodiments of the invention in which stabilizing endogenous HIFα is contemplated, the HIFα is selected from the group consisting of HIF-1α, HIF-2α, HIF-3α, and any fragment thereof. In one embodiment, the HIFα is endogenous to the subject.

In methods of the disclosure relating to inhibition of 2-oxoglutarate dioxygenase enzyme activity, various embodiments are provided in which the 2-oxoglutarate dioxygenase enzyme is selected from the group consisting of EGLN1, EGLN2, EGLN3, procollagen prolyl 4-hydroxylase, procollagen prolyl 3-hydroxylase, procollagen lysyl hydroxylase, PHD4, FIH-1, and any subunit or fragment thereof. With respect to methods for increasing endogenous EPO which comprise inhibiting HIF prolyl hydroxylase enzyme activity, embodiments in which the HIF prolyl hydroxylase enzyme is selected from the group consisting of EGLN1, EGLN2, EGLN3, and any subunit or fragment thereof are contemplated.

A preferred method for increasing endogenous EPO according to the present disclosure comprises administering to the subject a compound that increases endogenous EPO. In one aspect of this method, the compound stabilizes HIFα. In another aspect of this method, the compound inhibits hydroxylation of HIFα. In a further aspect of this method, the compound inhibits 2-oxoglutarate dioxygenase enzyme activity. In a particular aspect of this method, the compound inhibits HIF prolyl hydroxylase enzyme activity.

In certain embodiments, the present disclosure provides a method for increasing endogenous EPO in a subject, the method comprising administering to the subject a compound selected from the group consisting of heterocyclic carboxamides, phenanthrolines, hydroxamates, and physiologically active salts and prodrugs derived therefrom. In a particular embodiment of the disclosure, the compound is a heterocyclic carboxamide selected from the group consisting of pyridine carboxamides, quinoline carboxamides, isoquinoline carboxamides, cinnoline carboxamides, and beta-carboline carboxamides. In a preferred embodiment, the compound is delivered to the subject in the form of an oral formulation. In another preferred embodiment, the compound is delivered in a transdermal formulation.

Various methods for treating, preventing, or pretreating an EPO-associated disorder in a subject are disclosed. In one aspect, the present disclosure provides a method for treating, preventing, or pretreating an EPO-associated disorder, the method comprising increasing endogenous EPO. In another aspect of the disclosure, a method for treating, preventing, or pretreating an EPO-associated disorder in a subject, the method comprising stabilizing HIFα, is provided. In another method according to the present disclosure, a method of treating, preventing, or pretreating an EPO-associated disorder in a subject comprises inhibiting hydroxylation of HIFα. In yet another aspect, the disclosure provides a method of treating, preventing, or pretreating an EPO-associated disorder in a subject, the method comprising inhibiting 2-oxoglutarate dioxygenase enzyme activity. In a preferred aspect of the present disclosure, a method for treating, preventing, or pretreating an EPO-associated disorder in a subject, the method comprising inhibiting HIF prolyl hydroxylase enzyme activity, is contemplated.

The present invention relates to compounds of Formula (I) as defined herein that inhibit HIF prolyl hydroxylase enzyme activity for use in increasing endogenous erythropoietin in treating or preventing anemia associated with kidney disease in a subject.

In one aspect of the disclosure, the anemia is associated with abnormal hemoglobin or erythrocytes. In a further aspect of the disclosure, the anemia is associated with a condition selected from the group consisting of diabetes, cancer, ulcers, kidney disease, immunosuppressive disease, infection, and inflammation. In yet another aspect of the disclosure, the anemia is associated with a procedure or treatment selected from the group consisting of radiation therapy, chemotherapy, dialysis, and surgery. In another aspect of the disclosure the anemia is associated with blood loss, e.g. blood loss associated with bleeding disorders, trauma, injury, surgery. It is contemplated in specific embodiments of the disclosure that the anemia can be associated with defects in iron transport, processing, or utilization. Preventing or treating anemia associated with kidney disease by increasing endogenous EPO, and further comprising administering to the subject a compound selected from the group consisting of, e.g., an iron supplement, vitamin B₁₂, folic acid, exogenous erythropoietin, and granulocyte-colony stimulating factor is contemplated.

Methods for identifying compounds that increase endogenous EPO in a subject are also disclosed. In one embodiment, a method of identifying a compound that increases endogenous EPO, the method comprising administering a compound to a subject; measuring EPO in the subject or in a sample from the subject; and comparing the EPO in the subject or in the sample to a standard, wherein an increase in the EPO in the subject or in the sample relative to the standard is indicative of a compound that increases endogenous EPO, is disclosed.

The invention can be applied to an animal. Preferably, the animal is a mammal, e.g., a cat or dog, and, more preferably, the animal is a human. In certain embodiments, the invention increases synthesis of endogenous erythropoietin in tissues including renal, hepatic, hematopoietic, and/or neural tissues. The compounds of the invention that inhibit HIF prolyl hydroxylase enzyme activity are used in increasing endogenous erythropoietin to prevent or treat anemia associated with kidney disease. Erythropoietin-associated conditions associated with anemia include polycystic kidney disease, chronic renal failure, diabetes, cancer, ulcers, and immunosuppressive conditions such as AIDS. In further embodiments, the compounds of the invention are used to treat anemia associated with procedures or treatments including radiation therapy, chemotherapy, kidney dialysis, or surgery. In specific embodiments, the compounds of the invention are used to increase endogenous erythropoietin levels in an HIV-infected anemic subject being treated with zidovudine or other reverse transcriptase inhibitors. In other embodiments, the methods are used to increase endogenous erythropoietin levels in an anemic cancer patient receiving cyclic cisplatin- or non-cisplatin-containing chemotherapy. In further embodiments, the compounds are used to increase endogenous erythropoietin levels in an anemic patient scheduled to undergo elective, noncardiac, nonvascular surgery, thereby reducing the need for allogenic blood transfusions or to facilitate banking of blood prior to surgery. In one specific embodiment, the compounds are used to increase endogenous erythropoietin levels in a subject prior to procedures such as, e.g., surgery requiring aortic clamping such as thoracoabdominal aortic surgery.

In one aspect, the disclosure provides compounds that increase endogenous erythropoietin plasma levels. In one embodiment of the disclosure, a therapeutically effective amount of the compound or a pharmaceutically acceptable salt thereof, alone or in combination with a pharmaceutically acceptable excipient, is administered to a subject having an erythropoietin-associated condition. In another aspect of the disclosure, a therapeutically effective amount of the compound or a pharmaceutically acceptable salt thereof, alone or in combination with a pharmaceutically acceptable excipient, is administered to a patient having anemia.

Preferred embodiments of the invention comprise oral and transdermal delivery mechanisms. Such mechanisms could provide advantages over current therapies, e.g., increased ease of administration, self-administration by patient, reduced cost, fewer physician visits, and reduced risks due to infection and immunogenic complications, minimizing the adverse reactions some subjects develop in response to dosing with recombinant EPO. In one preferred embodiment, the present invention involves oral administration. Thus, the present invention also provides an oral formulation comprising a compound of the invention. In another preferred embodiment, the present compounds are for use in methods involving transdermal administration. Thus, the present invention may involve a transdermal patch or pad comprising a compound of the invention.

The compounds of the invention are of the formula (I) 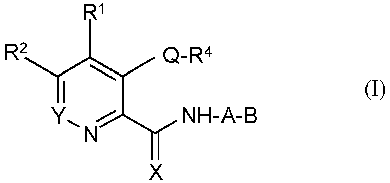 wherein
A is (C₁-C₄)-alkylene.
B is -CO₂H, -NH₂, -NHSO₂CF₃, tetrazolyl, imidazolyl, 3-hydroxyisoxazolyl, -CONHCOR'", -CONHSOR"', CONHSO₂R"', where R'" is aryl, heteroaryl, (C₃-C₇)-cycloalkyl, or (C₁-C₄)-alkyl, optionally monosubstituted by (C₆-C₁₂)-aryl, heteroaryl, OH, SH, (C₁-C₄)-alkyl, (C₁-C₄)-alkoxy, (C₁-C₄)-thioalkyl, (C₁-C₄)-sulfinyl, (C₁-C₄)-sulfonyl, CF₃, Cl, Br, F, I, NO2, -COOH, (C₂-C₅)-alkoxycarbonyl, NH₂, mono-(C₁-C₄-alkyl)-amino, di-(C₁-C₄-alkyl)-amino, or (C₁-C₄)-perfluoroalkyl; or wherein B is a CO₂-G carboxyl radical, where G is a radical of an alcohol G-OH in which G is selected from (C₁-C₂₀)-alkyl radical, (C₃-C₈) cycloalkyl radical, (C₂-C₂₀)-alkenyl radical, (C₃-C₈)-cycloalkenyl radical, retinyl radical, (C₂-C₂₀)-alkynyl radical, (C₄-C₂₀)-alkenynyl radical, where the alkenyl, cycloalkenyl, alkynyl, and alkenynyl radicals contain one or more multiple bonds; (C₆-C₁₆)-carbocyclic aryl radical, (C₇-C₁₆)-carbocyclic aralkyl radical, heteroaryl radical, or heteroaralkyl radical, wherein a heteroaryl radical or heteroaryl moiety of a heteroaralkyl radical contains 5 or 6 ring atoms; and wherein radicals defined for G are substituted by one or more hydroxyl, halogen, cyano, trifluoromethyl, nitro, carboxyl, (C₁-C₁₂)-alkyl, (C₃-C₈)-cycloalkyl, (C₅-C₈)-cycloalkenyl, (C₆-C₁₂)-aryl, (C₇-C₁₆)-aralkyl, (C₂-C₁₂)-alkenyl, (C₂-C₁₂)-alkynyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, (C₆-C₁₂)-aryloxy, (C₇-C₁₆)-aralkyloxy, (C₁-C₈)-hydroxyalkyl, -O-[CH₂]ₓ-C_{f}H_{(2f+1-g)}-F_{g}, -OCF₂Cl, -OCF₂-CHFCl, (C₁-C₁₂)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₂-C₁₂)-alkenylcarbonyl, (C₂-C₁₂)-alkynylcarbonyl, (C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, acyloxy, (C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆) aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy, carbamoyl, N-(C₁-C₁₂)-alkylcarbamoyl, N.N-di(C₁-C₁₂)-alkylcarbamoyl, N-(C₃-C₈)-cycloalkyl-carbamoyl, N-(C₆-C₁₆)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₀)-alkoxy-(C₁-C₁₀)-alkyl)-carbamoyl, N-((C₆-C₁₂)-aryloxy-(C₁-C₁₀)alkyl)-carbamoyl, N-((C₇-C₁₆)-aralkyloxy-(C₁-C₁₀)-alkyl)-carbamoyl, N-(C₁-C₁₀)-alkyl-N-((C₁-C₁₀)-alkoxy-(C₁-C₁₀)-alkyl)-carbamoyl, N-(C₁-C₁₀)-alkyl-N-((C₆-C₁₆)-aryloxy-(C₁-C₁₀)-alkyl)-carbamoyl, N-(C₁-C₁₀)-alkyl-N-((C₇-C₁₆)-aralkyloxy-(C₁-C₁₀)-alkyl)-carbamoyl, carbamoyloxy, N-(C₁-C₁₂)-alkylcarbamoyloxy, N.N-di-(C₁-C₁₂)-alkylcarbamoyloxy, N-(C₃-C₈)-cycloalkylcarbamoyloxy, N-(C₆-C₁₂)-arylcarbamoyloxy, N-(C₇-C₁₆)-aralkylcarbamoyloxy, N-(C₁-C₁₀)-alkyl-N-(C₆-C₁₂)-arylcarbamoyloxy, N(C₁-C₁₀)-alkyl-N-(C₇-C₁₆)-aralkylcarbamoyloxy, N-((C₁-C₁₀)-alkyl)-carbamoyloxy, N-((C₆-C₁₂)-aryloxy-(C₁-C₁₀)-alkyl)-carbamoyloxy, N-((C₇-C₁₆)-aralkyloxy-(C₁-C₁₀)-alkyl)-carbamoyloxy, N-(C₁-C₁₀)-alkyl-N-((C₁-C₁₀)-alkoxy-(C₁-C₁₀)-alkyl)-carbamoyloxy, N-(C₁-C₁₀)-alkyl-N-((C₆-C₁₂)-aryloxy-(C₁-C₁₀)-alkyl)-carbamoyloxy, N-(C₁-C₁₀)-alkyl-N-((C₇-C₁₆)-aralkyloxy-(C₁-C₁₀)-alkyl)-carbamoyloxy, amino, (C₁-C₁₂)-alkylamino, di-(C₁-C₁₂)-alkylamino, (C₃-C₈)-cycloalkylamino, (C₂-C₁₂)-alkenylamino, (C₂-C₁₂)-alkynylamino, N-(C₆-C₁₂)-arylamino, N-(C-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino, (C₁-C₁₂)-alkylcarbonylamino, (C₃-C₈)-cycloalkylcarbonylamino, (C₆-C₁₂) arylcarbonylamino, (C₇-C₁₆)-aralkylcarbonylamino, (C₁-C₁₂)-alkylcarbonyl-N-(C₁-C₁₀)-alkylamino, (C₃-C₈)-cycloalkylcarbonyl-N-(C₁-C₁₀)-alkylamino, (C₆-C₁₂)-arylcarbonyl-N-(C₁-C₁₀)alkylamino, (C₇-C₁₁)-aralkylcarbonyl-N-(C₁-C₁₀)-alkylamino, (C₁-C₁₂)-alkylcarbonylamino-(C₁-C₈)-alkyl, (C₃-C₈)-cycloalkylcarbonylamino-(C₁-C₈)alkyl, (C₆-C₁₂)-arylcarbonylamino-(C₁-C₈)-alkyl, (C₇-C₁₂)-aralkylcarbonylamino(C₁-C₈)-alkyl, amino-(C₁-C₁₀)-alkyl, N-(C₁-C₁₀) alkylamino-(C₁-C₁₀)-alkyl, N.N-di-(C₁-C₁₀)-alkylamino-(C₁-C₁₀)-alkyl, (C₃-C₈)cycloalkylamino-(C₁-C₁₀)-alkyl, (C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₂)-arylsulfonyl, (C₇-C₁₆)-aralkylmercapto, (C₇-C₁₆)-aralkylsulfinyl, (C₇-C₁₆)-aralkylsulfonyl, sulfamoyl, N-(C₁-C₁₀)-alkylsulfamoyl, N.N-di(C₁-C₁₀)-alkylsulfamoyl, (C₃-C₈)-cycloalkylsulfamoyl, N-(C₆-C₁₂)-alkylsulfamoyl, N-(C₇-C₁₆)-aralkylsulfamoyl, N-(C₁-C₁₀)-alkyl-N-(C₆-C₁₂)-arylsulfamoyl, N-(C₁-C₁₀)-alkyl-N-(C₇-C₁₆)-aralkylsulfamoyl, (C₁-C₁₀)-alkylsulfonamido, N-((C₁-C₁₀)-alkyl)-(C₁-C₁₀)-alkylsulfonamido, (C₇-C₁₆)-aralkylsulfonamido, or N-((C₁-C₁₀)-alkyl-(C₇-C₁₆)-aralkylsulfonamido; wherein radicals which are aryl or contain an aryl moiety, may be substituted on the aryl by one to five identical or different hydroxyl, halogen, cyano, trifluoromethyl, nitro, carboxyl, (C₁-C₁₂)-alkyl, (C₃-C₈)-cycloalkyl, (C₆-C₁₂)-aryl, (C₇-C₁₆)-aralkyl, (C₁-C₁₂)-alkoxy, (C₁-C₁₂)-alkoxy-(C₁-C₁₂)alkyl, (C₁-C₁₂)-alkoxy-(C₁ C₁₂)alkoxy, (C₆-C₁₂)-aryloxy, (C₇-C₁₆)-aralkyloxy, (C₁-C₈)-hydroxyalkyl, (C₁-C₁₂)-alkylcarbonyl, (C₃-C₈)-cycloalkyl-carbonyl, (C₆-C₁₂)-arylcarbonyl, (C₇-C₁₆) aralkylcarbonyl, (C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, (C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy, (C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy, carbamoyl, N-(C₁-C₁₂)-alkylcarbamoyl, N.N-di-(C₁-C₁₂)-alkylcarbamoyl, N-(C₃-C₈)-cycloalkylcarbamoyl, N-(C₆-C₁₂)-arylcarbamoyl, N-(C₇-C₁₆)-aralkylcarbamoyl, N-(C₁-C₁₀)-alkyl-N-(C₆-C₁₂)-arylcarbamoyl, N-(C₁-C₁₀)-alkyl-N-(C₇-C₁₆)-aralkylcarbamoyl, N-((C₁-C₁₀)-alkoxy-(C₁-C₁₀)-alkyl)-carbamoyl, N-((C₆-C₁₂)-aryloxy-(C₁-C₁₀)-alkyl)-carbamoyl, N-((C₇-C₁₆)-aralkyloxy-(C₁-C₁₀)-alkyl)-carbamoyl, N-(C₁-C₁₀)-alkyl-N-((C₁-C₁₀)-alkoxy-(C₁-C₁₀)-alkyl)-carbamoyl, N-(C₁-C₁₀)-alkyl-N-((C₆-C₁₂)-aryloxy-(C₁-C₁₀)-alkyl)-carbamoyl, N-(C₁-C₁₀)-alkyl-N-((C₇-C₁₆)-aralkyloxy-(C₁-C₁₀)-alkyl)-carbamoyl, carbamoyloxy, N-(C₁-C₁₂)-alkylcarbamoyloxy, N.N-di-(C₁-C₁₂)-alkylcarbamoyloxy, N-(C₃-C₈)-cycloalkylcarbamoyloxy, N-(C₆-C₁₂)-arylcarbamoyloxy, N-(C₇-C₁₆)-aralkylcarbamoyloxy, N-(C₁-C₁₀)-alkyl-N-(C₆-C₁₂)-arylcarbamoyloxy, N(C₁-C₁₀)-alkyl-N-(C₇-C₁₆)-aralkylcarbamoyloxy, N-((C₁-C₁₀)-alkyl)-carbamoyloxy, N-((C₆-C₁₂)-aryloxy-(C₁-C₁₀)-alkyl)-carbamoyloxy, N-((C₇-C₁₆)-aralkyloxy-(C₁-C₁₀)-alkyl)-carbamoyloxy, N-(C₁-C₁₀)-alkyl-N-((C₁-C₁₀)-alkoxy-(C₁-C₁₀)-alkyl)-carbamoyloxy, N-(C₁-C₁₀)-alkyl-N-((C₆-C₁₂)-aryloxy-(C₁-C₁₀)-alkyl)-carbamoyloxy, N-(C₁-C₁₀)-alkyl-N-((C₇-C₁₆)-aralkyloxy-(C₁-C₁₀)-alkyl)-carbamoyloxy, amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkylaralkylamino, N-alkyl-arylamino, (C₁-C₁₂)-alkoxyamino, (C₁-C₁₂)-alkoxy-N-(C₁-C₁₀)-alkylamino, (C₁-C₁₂)-alkylcarbonylamino, (C₃-C₈)-cycloalkylcarbonylamino, (C₆-C₁₂)-arylcarbonylamino, (C₇-C₁₆)-alkylcarbonylamino, (C₁-C₁₂)-alkylcarbonyl-N-(C₁-C₁₀)-alkylamino, (C₃-C₈)-cycloalkylcarbonyl-N-(C₁-C₁₀)-alkylamino, (C₆-C₁₂)-arylcarbonyl-N-(C₁-C₁₀)-alkylamino, (C₇-C₁₁)-aralkylcarbonyl-N-(C₁-C₁₀)-alkylamino, (C₁-C₁₂)-alkylcarbonylamino-(C₁-C₈)-alkyl, (C₃-C₈)-cycloalkylcarbonylamino-(C₁-C₈)-alkyl, (C₆-C₁₂)-arylcarbonylamino-(C₁-C₈)-alkyl, (C₇-C₁₆)-aralkylcarbonylamino-(C₁-C₈)-alkyl, amino-(C₁-C₁₀)-alkyl, N-(C₁-C₁₀)-alkylamino-(C₁-C₁₀)alkyl, N.N-di-(C₁-C₁₀)-alkylamino-(C₁-C₁₀)-alkyl, (C₃-C₈)-cycloalkylamino-(C₁-C₁₀)-alkyl, (C₁-C₁₂)-alkylmercapto, (C₁-C₁₂)-alkylsulfinyl, (C₁-C₁₂)-alkylsulfonyl, (C₆-C₁₂)-arylmercapto, (C₆-C₁₂)-arylsulfinyl, (C₆-C₁₂)-arylsulfonyl, (C₇-C₁₆)-aralkylmercapto, (C₇-C₁₆)-aralkylsulfinyl, or (C₇-C₁₆)-aralkylsulfonyl;
X is O or S;
Q is O, S, NR', or a bond;
where, if Q is a bond, R⁴ is halogen, nitrile, or trifluoromethyl;
or where, if Q is O, S, or NR', R⁴ is hydrogen, (C₁-C₁₀)-alkyl radical, (C₂-C₁₀)-alkenyl radical, (C₂-C₁₀)-alkynyl radical, wherein alkenyl or alkynyl radical contains one or two C-C multiple bonds; unsubstituted fluoroalkyl radical of the formula -[CH₂]ₓ-C_{f}H_{(2f+1-g)}-F_{g}, (C₁-C₈)-alkoxy-(C₁-C₆)-alkyl radical, (C₁-C₆)-alkoxy-(C₁-C₄)-alkoxy-(C₁-C₄)-alkyl radical, aryl radical, heteroaryl radical, (C₇-C₁₁)-aralkyl radical, or a radical of the formula Z

   -[CH₂]ᵥ-[O]_{w}-[CH₂]ₜ-E (Z)

   where
   E is a heteroaryl radical, a (C₃-C₈)-cycloalkyl radical, or a phenyl radical of the formula F 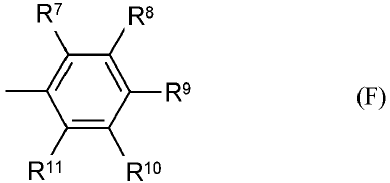
      v is 0-6,
      w is 0 or 1,
      t is 0-3, and
      R⁷, R⁸, R⁹, R¹⁰, and R¹¹ are identical or different and are hydrogen, halogen, cyano, nitro, trifluoromethyl, (C₁-C₆)-alkyl, (C₃-C₈)-cycloalkyl, (C₁-C₆)-alkoxy, -O-[CH₂]ₓ-C_{f}H_{(2f+1-g)}-F_{g}, -OCF₂-Cl, -O-CF₂-CHFCl, (C₁-C₆)-alkylmercapto, (C₁-C₆)-hydroxyalkyl, (C₁-C₆)-alkoxy-(C₁-C₆)-alkoxy, (C₁-C₆)-alkoxy-(C₁-C₆)-alkyl, (C₁-C₆)-alkylsulfinyl, (C₁-C₆)-alkylsulfonyl, (C₁-C₆)-alkylcarbonyl, (C₁-C₈)-alkoxycarbonyl, carbamoyl, N-(C₁-C₈)-alkylcarbamoyl, N,N-di-(C₁-C₈)-alkylcarbamoyl, or (C₇-C₁₁)-aralkylcarbamoyl, optionally substituted by fluorine, chlorine, bromine, trifluoromethyl, (C₁-C₆)-alkoxy, N-(C₃-C₈)-cycloalkylcarbamoyl, N-(C₃-C₈)-cycloalkyl-(C₁-C₄)-alkylcarbamoyl, (C₁-C₆)-alkylcarbonyloxy, phenyl, benzyl, phenoxy, benzyloxy, NR^{Y}R^{Z} wherein R^{y} and R^{z} are independently selected from hydrogen, (C₁-C₁₂)-alkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₃-C₁₀)-cycloalkyl, (C₃-C₁₂)-alkenyl, (C₃-C₁₂)-alkynyl, (C₆-C₁₂)-aryl, (C₇-C₁₁)-aralkyl, (C₁-C₁₂)-alkoxy, (C₇-C₁₂)aralkoxy, (C₁-C₁₂)-alkylcarbonyl, (C₃-C₈)-cycloalkylcarbonyl, (C₆-C₁₂) arylcarbonyl, (C₇-C₁₆)-aralkylcarbonyl; or further wherein R^{y} and R^{z} together are-[CH2]ₕ, in which a CH₂ group can be replaced by O, S, N-(C₁-C₄)-alkylcarbonylimino, or N-(C₁-C₄)-alkoxycarbonylimino; phenylmercapto, phenylsulfonyl, phenylsulfinyl, sulfamoyl, N-(C₁-C₈)-alkylsulfamoyl, or N, N-di-(C₁-C₈)-alkylsulfamoyl; or alternatively R⁷ and R⁸, R⁸ and R⁹, R⁹ and R¹⁰, or R¹⁰ and R¹¹, together are a chain selected from -[CH₂]ₙ- or -CH=CH-CH=CH-, where a CH₂ group of the chain is optionally replaced by O, S, SO, SO₂, or NR^{Y}; and n is 3, 4, or 5; and if E is a heteroaryl radical, said radical can carry 1-3 substituents selected from those defined for R⁷-R¹¹, or if E is a cycloalkyl radical, the radical can carry one substituent selected from those defined for R⁷-R¹¹;
   or where, if Q is NR', R⁴ is alternatively R", where R' and R" are identical or different and are hydrogen, (C₆-C₁₂)-aryl, (C₇-C₁₁)-aralkyl, (C₁-C₈)-alkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkylcarbonyl, optionally substituted (C₇-C₁₆)-aralkylcarbonyl, or optionally substituted C₆-C₁₂)-arylcarbonyl; or R' and R" together are -[CH₂]ₕ, in which a CH₂ group can be replaced by O, S, N-acylimino, or N-(C₁-C₁₀)-alkoxycarbonylimino, and h is 3 to 7.
   Y is N or CR³;
   R¹, R² and R³ are identical or different and are hydrogen, hydroxyl, halogen, cyano, trifluoromethyl, nitro, carboxyl, (C₁-C₂₀)-alkyl, (C₃-C₈)-cycloalkyl, (C₃-C₈)cycloalkyl-(C₁-C₁₂)-alkyl, (C₃-C₈)-cycloalkoxy, (C₃-C₈)-cycloalkyl-(C₁-C₁₂)-alkoxy, (C₃-C₈)-cycloalkyloxy-(C₁-C₁₂)-alkyl, (C₃-C₈)-cycloalkyloxy-(C₁-C₁₂)-alkoxy, (C₃-C₈)-cycloalkyl-(C₁-C₈)-alkyl-(C₁-C₆)-alkoxy, (C₃-C₈)-cycloalkyl-(C₁-C₈)-alkoxy-(C₁-C₆)-alkyl, (C₃-C₈)-cycloalkyloxy-(C₁-C₈)-alkoxy-(C₁-C₆)-alkyl, (C₃-C₈)-cycloalkoxy-(C₁-C₈)-alkoxy-(C₁-C₈)-alkoxy, (C₆-C₁₂)-aryl, (C₇-C₁₆)-aralkyl, (C₇-C₁₆)-aralkenyl, (C₇-C₁₆)-aralkynyl, (C₂-C₂₀)-alkenyl, (C₂-C₂₀)-alkynyl, (C₁-C₂₀)-alkoxy, (C₂-C₂₀)-alkenyloxy, (C₂-C₂₀)-alkynyloxy, retinyloxy, (C₁-C₂₀)-alkoxy-(C₁-C₁₂)-alkyl, (C₁-C₁₂)-alkoxy-(C₁-C₁₂)-alkoxy, (C₁-C₁₂)-alkoxy-(C₁-C₈)-alkoxy-(C₁-C₈)-alkyl, (C₆-C₁₂)-aryloxy, (C₇-C₁₆)-aralkyloxy, (C₆-C₁₂)-aryloxy-(C₁-C₆)-alkoxy, (C₇-C₁₆)-aralkoxy-(C₁-C₆)-alkoxy, (C₁-C₁₆)-hydroxyalkyl, (C₆-C₁₆)-aryloxy-(C₁-C₈)-alkyl, (C₇-C₁₆)-aralkoxy-(C₁-C₈)-alkyl, (C₆-C₁₂)-aryloxy-(C₁-C₈)-alkoxy-(C₁-C₆)-alkyl, (C₇-C₁₂)-aralkyloxy-(C₁-C₈)-alkoxy-(C₁-C₆)-alkyl, (C₂-C₂₀)-alkenyloxy-(C₁-C₆)-alkyl, (C₂-C₂₀)-alkynyloxy-(C₁-C₆)-alkyl, retinyloxy-(C₁-C₆)-alkyl, -O-[CH₂]ₓCfH_{(2f+1-g)}F_{g}, -OCF₂Cl, -OCF₂-CHFCl, (C₁-C₂₀)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₂-C₂₀)-alkenylcarbonyl, (C₂-C₂₀)-alkynylcarbonyl, (C₁-C₂₀)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₂₀)-alkenyloxycarbonyl, retinyloxycarbonyl, (C₂-C₂₀)-alkynyloxycarbonyl, (C₆-C₁₂)-aryloxy-(C₁-C₆)-alkoxycarbonyl, (C₇-C₁₆)-aralkoxy-(C₁-C₆)-alkoxycarbonyl, (C₃-C₈)-cycloalkyl-(C₁-C₆)-alkoxycarbonyl, (C₃-C₈)-cycloalkoxy-(C₁-C₆)-alkoxycarbonyl, (C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy, (C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy, carbamoyl, N-(C₁-C₁₂)-alkylcarbamoyl, N,N-di-(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)-carbamoyl, N-(C₁-C₆)-alkyl-N-((C₃-C₈)-cycloalkyl-(C₁-C₆)-alkyl)-carbamoyl, N-(+)-dehydroabietylcarbamoyl, N-(C₁-C₆)-alkyl-N-(+)-dehydroabietylcarbamoyl, N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₈)-alkoxy-(C₁-C₁₀)-alkyl)-carbamoyl, N-((C₆-C₁₆)-aryloxy-(C₁-C₁₀)-alkyl)-carbamoyl, N-((C₇-C₁₆)-aralkyloxy-(C₁-C₁₀)-alkyl)-carbamoyl, N-(C₁-C₁₀)-alkyl-N-((C₁-C₁₀)-alkoxy-(C₁-C₁₀)-alkyl)-carbamoyl, N-(C₁-C₁₀)-alkyl-N-((C₆-C₁₂)-aryloxy-(C₁-C₁₀)-alkyl)-carbamoyl, N-(C₁-C₁₀)-alkyl-N-((C₇-C₁₆)-aralkyloxy-(C₁-C₁₀)-alkyl)-carbamoyl; CON(CH₂)ₕ, in which a CH₂ group can be replaced by O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino, N-(C₁-C₄)-alkoxy-(C₁-C₆)-alkylimino, and h is from 3 to 7; a carbamoyl radical of the formula R 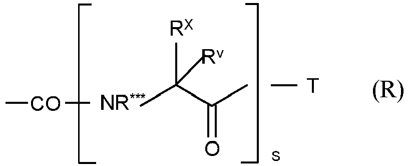 in which
      R^{x} and R^{v} are each independently selected from hydrogen, (C₁-C₆)-alkyl, (C₃-C₇)-cycloalkyl, or aryl,
      s is 1-5,
      T is OH, or NR^{∗}R^{∗∗}, and R^{∗}, R^{∗∗} and R^{∗∗∗} are identical or different and are selected from hydrogen, (C₆-C₁₂)-aryl, (C₇-C₁₁)-aralkyl, (C₁-C₈)-alkyl, (C₃-C₈)-cycloalkyl, (+)-dehydroabietyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkanoyl, optionally substituted (C₇-C₁₆)-aralkanoyl, optionally substituted (C₆-C₁₂)-aroyl; or R^{∗} and R^{∗∗} together are -[CH₂]ₕ, in which a CH₂ group can be replaced by O, S, SO, SO₂, N-acylamino, N-(C₁-C₁₀)-alkoxycarbonylimino, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino, N-(C₁-C₄)-alkoxy-(C₁-C₆)-alkylimino, and h is from 3 to 7; carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₂)-arylcarbamoyloxy, N-(C₇-c₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkyl)-carbamoyloxy, N-((C₆-C₁₂)-aryloxy-(C₁-C₁₀)-alkyl)-carbamoyloxy, N-((C₇-C₁₆)-aralkyloxy-(C₁-C₁₀)-alkyl)-carbamoyloxy, N-(C₁-C₁₀)-alkyl-N-((C₁-C₁₀)-alkoxy-(C₁-C₁₀)-alkyl)-carbamoyloxy, N-(C₁-C₁₀)-alkyl-N-((C₆-C₁₂)-aryloxy-(C₁-C₁₀)-alkyl)-carbamoyloxy, N-(C₁-C₁₀)-alkyl-N-((C₇-C₁₆)-aralkyloxy-(C₁-C₁₀)-alkyl)-carbamoyloxyamino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino, (C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino, (C₁-C₁₂)-alkanoylamino-(C₁-C₈)-alkyl, (C₃-C₈)-cycloalkanoylamino-(C₁-C₈)-alkyl, (C₆-C₁₂)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di(C₁-C₁₀)-alkylamino-(C₁-C₁₀)-alkyl, (C₃-C₈)-cycloalkylamino(C₁-C₁₀)-alkyl, (C₁-C₂₀)-alkylmercapto, (C₁-C₂₀)-alkylsulfinyl, (C₁-C₂₀)-alkylsulfonyl, (C₆-C₁₂)-arylmercapto, (C₆-C₁₂)-arylsulfinyl, (C₆-C₁₂)-arylsulfonyl, (C₇-C₁₆)-aralkylmercapto, (C₇-C₁₆)-aralkylsulfinyl, (C₇-C₁₆)-aralkylsulfonyl, (C₁-C₁₂)-alkylmercapto-(C₁-C₆)-alkyl, (C₁-C₁₂)-alkylsulfinyl-(C₁-C₆)-alkyl, (C₁-C₁₂)-alkylsulfonyl-(C₁-C₆)-alkyl, (C₆-C₁₂)-arylmercapto-(C₁-C₆)-alkyl, (C₆-C₁₂)-arylsulfinyl-(C₁-C₆)-alkyl, (C₆-C₁₂)-arylsulfonyl-(C₁-C₆)-alkyl, (C₇-C₁₆)-aralkylmercapto-(C₁-C₆)-alkyl, (C₇-C₁₆)-aralkylsulfinyl-(C₁-C₆)-alkyl, (C₇-C₁₆)-aralkylsulfonyl-(C₁-C₆)-alkyl, sulfamoyl, N-(C₁-C₁₀)-alkylsulfamoyl, N,N-di-(C₁-C₁₀)-alkylsulfamoyl, (C₃-C₈)-cycloalkylsulfamoyl, N-(C₆-C₁₂)-arylsulfamoyl, N-(C₇-C₁₆)-aralkylsulfamoyl, N-(C₁-C₁₀)-alkyl-N-(C₆-C₁₂)-arylsulfamoyl, N-(C₁-C₁₀)-alkyl-N-(C₇-C₁₆)-aralkylsulfamoyl, (C₁-C₁₀)-alkylsulfonamido, N-((C₁-C₁₀)-alkyl)-(C₁-C₁₀)-alkylsulfonamido, (C₇-C₁₆)-aralkylsulfonamido, and N-((C₁-C₁₀)-alkyl-(C₇-C₁₆)-aralkylsulfonamido; where an aryl radical may be substituted by 1 to 5 substituents selected from hydroxyl, halogen, cyano, trifluoromethyl, nitro, carboxyl, (C₂-C₁₆)-alkyl, (C₃-C₈)-cycloalkyl, (C₃-C₈)-cycloalkyl-(C₁-C₁₂)-alkyl, (C₃-C₈)-cycloalkoxy, (C₃-C₈)-cycloalkyl-(C₁-C₁₂)-alkoxy, (C₃-C₈)-cycloalkyloxy-(C₁-C₁₂)-alkyl, (C₃-C₈)-cycloalkyloxy-(C₁-C₁₂)-alkoxy, (C₃-C₈)-cycloalkyl-(C₁-C₈)-alkyl-(C₁-C₆)-alkoxy, (C₃-C₈)-cycloalkyl(C₁-C₈)-alkoxy-(C₁-C₆)-alkyl, (C₃-C₈)-cycloalkyloxy-(C₁-C₈)-alkoxy-(C₁-C₆)-alkyl, (C₃-C₈)-cycloalkoxy-(C₁-C₈)-alkoxy-(C₁-C₈)-alkoxy, (C₆-C₁₂)-aryl, (C₇-C₁₆)-aralkyl, (C₂-C₁₆)-alkenyl, (C₂-C₁₂)-alkynyl, (C₁-C₁₆)-alkoxy, (C₁-C₁₆)-alkenyloxy, (C₁-C₁₂)-alkoxy-(C₁-C₁₂)-alkyl, (C₁-C₁₂)-alkoxy-(C₁-C₁₂)-alkoxy, (C₁-C₁₂)-alkoxy(C₁-C₈)-alkoxy-(C₁-C₈)-alkyl, (C₆-C₁₂)-aryloxy, (C₇-C₁₆)-aralkyloxy, (C₆-C₁₂)-aryloxy-(C₁-C₆)-alkoxy, (C₇-C₁₆)-aralkoxy-(C₁-C₆)-alkoxy, (C₁-C₈)-hydroxyalkyl, (C₆-C₁₆)-aryloxy-(C₁-C₈)-alkyl, (C₇-C₁₆)-aralkoxy-(C₁-C₈)-alkyl, (C₆-C₁₂)-aryloxy-(C₁-C₈)-alkoxy-(C₁-C₆)-alkyl, (C₇-C₁₂)-aralkyloxy-(C₁-C₈)-alkoxy-(C₁-C₆)-alkyl, -O-[CH₂]ₓC_{f}H_{(2f+1-g)}F_{g}, -OCF₂Cl, -OCF₂-CHFCl, (C₁-C₁₂)-alkylcarbonyl, (C₃-C₈)-cycloalkylcarbonyl, (C₆-C₁₂)-arylcarbonyl, (C₇-C₁₆)-aralkylcarbonyl, (C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, (C₆-C₁₂)-aryloxy-(C₁-C₆)-alkoxycarbonyl, (C₇-C₁₆)-aralkoxy-(C₁-C₆)-alkoxycarbonyl, (C₃-C₈)-cycloalkyl-(C₁-C₆)-alkoxycarbonyl, (C₃-C₈)-cycloalkoxy-(C₁-C₆)-alkoxycarbonyl, (C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy, (C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy, carbamoyl, N-(C₁-C₁₂)-alkylcarbamoyl, N,N-di(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl, N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₆)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₂)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)-carbamoyl, CON(CH₂)ₕ, in which a CH₂ group can be replaced by, O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino, N-(C₁-C₄)-alkoxy-(C₁-C₆)-alkylimino, and h is from 3 to 7; carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₆)-arylcarbamoyloxy, N-(C₇-C₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkyl)carbamoyloxy, N-((C₆-C₁₂)-aryloxy-(C₁-C₁₀)-alkyl)carbamoyloxy, N-((C₇-C₁₆)-aralkyloxy-(C₁-C₁₀)-alkyl)carbamoyloxy, N-(C₁-C₁₀)-alkyl-N-((C₁-C₁₀)-alkoxy-(C₁-C₁₀)-alkyl)carbamoyloxy, N-(C₁-C₁₀)-alkyl-N-((C₆-C₁₂)-aryloxy-(C₁-C₁₀)-alkyl)carbamoyloxy, N-(C₁-C₁₀)-alkyl-N-((C₇-C₁₆)-aralkyloxy-(C₁-C₁₀)-alkyl)carbamoyloxy, amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino, (C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino, (C₁-C₁₂)-alkanoylamino-(C₁-C₈)-alkyl, (C₃-C₈)-cycloalkanoylamino-(C₁-C₈)-alkyl, (C₆-C₁₂)-aroylamino- (C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylamino-(C₁-C₁₀)-alkyl, (C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkylmercapto, (C₇-C₁₆)-aralkylsulfinyl, or (C₇-C₁₆)-aralkylsulfonyl;
      or wherein R¹ and R², or R² and R³ form a chain [CH₂]ₒ, which is saturated or unsaturated by a C=C double bond, in which 1 or 2 CH₂ groups are optionally replaced by O, S, SO, SO₂, or NR', and R' is hydrogen, (C₆-C₁₂)-aryl, (C₁-C₈)-alkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkanoyl, optionally substituted (C₇-C₁₆)-aralkanoyl, or optionally substituted (C6-C12)-aroyl; and ο is 3, 4 or 5;
      or wherein the radicals R¹ and R², or R² and R³, together with the pyridine or pyridazine carrying them, form a 5,6,7,8-tetrahydroisoquinoline ring, a 5,6,7,8-tetrahydroquinoline ring, or a 5,6,7,8-tetrahydrocinnoline ring;
      or wherein R¹ and R², or R² and R³ form a carbocyclic or heterocyclic 5- or 6-membered aromatic ring;
      or where R¹ and R², or R² and R³, together with the pyridine or pyridazine carrying them, form an optionally substituted heterocyclic ring systems selected from thienopyridines, furanopyridines, pyridopyridines, pyrimidinopyridines, imidazopyridines, thiazolopyridines, oxazolopyridines, quinoline, isoquinoline, and cinnoline; where quinoline, isoquinoline or cinnoline preferably satisfy the formulae Ia, Ib and Ic: 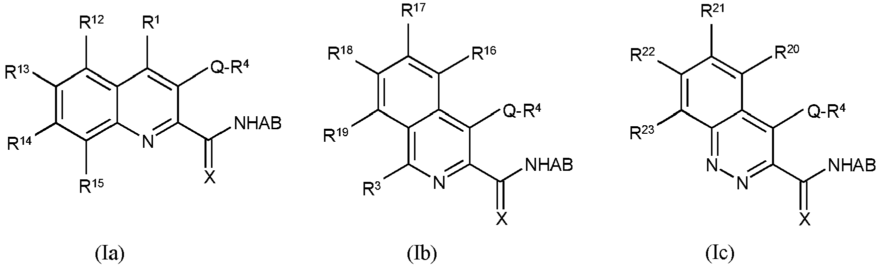
      and the substituents R¹² to R²³ in each case independently of each other have the meaning of R¹, R² and R³;
      or wherein the radicals R¹ and R², together with the pyridine carrying them, form a compound of Formula Id: 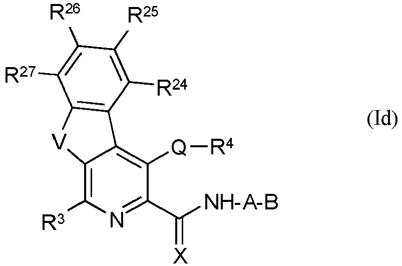
         where V is S, O, or NR^{k}, and R^{k} is selected from hydrogen, (C₁-C₆)-alkyl, aryl, or benzyl; where an aryl radical may be optionally substituted by 1 to 5 substituents as defined above; and
         R²⁴, R²⁵, R²⁶, and R²⁷ in each case independently of each other have the meaning of R¹, R² and R³;
         f is 1 to 8;
         g is 0 or 1 to (2f+1);
         x is 0 to 3; and
         h is 3 to 7;
         including the physiologically active salts derived therefrom.

In some embodiments, compounds of Formula (I) as defined above include, but are not limited to, N-((6-(1-butyloxy)-3-hydroxyquinolin-2-yl)-carbonyl)-glycine; N-((6-chloro-3-hydroxyquinolin-2-yl)-carbonyl)-glycine; N-((3-hydroxy-6-(2-propyloxy)-quinolin-2-yl)-carbonyl)-glycine; and N-((7-chloro-3-hydroxyquinolin-2-yl)-carbonyl)-glycine; [(3-methoxy-pyridine-2-carbonyl)-amino]-acetic acid; 3-methoxypyridine-2-carboxylic acid N-(((hexadecyloxy)-carbonyl)-methyl)-amide hydrochloride, 3-methoxypyridine-2-carboxylic acid N-(((1-octyloxy)-carbonyl)-methyl)-amide, 3-methoxypyridine-2-carboxylic acid N-(((hexyloxy)-carbonyl)-methyl)-amide, 3-methoxypyridine-2-carboxylic acid N-(((butyloxy)-carbonyl)-methyl)-amide, 3-methoxypyridine-2-carboxylic acid N-(((2-nonyloxy)-carbonyl)-methyl)-amide racemate, 3-methoxypyridine-2-carboxylic acid N-(((heptyloxy)-carbonyl)-methyl)-amide, 3-benzyloxypyridine-2-carboxylic acid N-(((octyloxy)-carbonyl)-methyl)-amide, 3-benzyloxypyridine-2-carboxylic acid N-(((butyloxy)-carbonyl)-methyl)-amide, 5-(((3-(1-butyloxy)-propyl)-amino)-carbonyl)-3-methoxypyridine-2-carboxylic acid N-((benzyloxycarbonyl)-methyl)-amide, 5-(((3-(1-butyloxy)-propyl)-amino)-carbonyl)-3-methoxypyridine-2-carboxylic acid N-(((1-butyloxy)-carbonyl)-methyl)-amide, 5-(((3-lauryloxy)-propyl)amino)-carbonyl)-3-methoxypyridine-2-carboxylic acid N-(((benzyloxy)-carbonyl)-methyl)-amide, 3-hydroxypyridine-2-carboxylic acid N-(((hexadecyloxy)-carbonyl)-methyl)-amide hydrochloride, 3-hydroxypyridine-2-carboxylic acid N-(((1-octyloxy)-carbonyl)-methyl)-amide, 3-hydroxypyridine-2-carboxylic acid N-(((hexyloxy)-carbonyl)-methyl)-amide, 3-hydroxypyridine-2-carboxylic acid N-(((butyloxy)-carbonyl)-methyl)-amide, 3-hydroxypyridine-2-carboxylic acid N-(((2-nonyloxy)-carbonyl)-methyl)-amide racemate, 3-hydroxypyridine-2-carboxylic acid N-(((heptyloxy)-carbonyl)-methyl)-amide, 3-benzyloxypyridine-2-carboxylic acid N-(((octyloxy)-carbonyl)-methyl)-amide, 3-benzyloxypyridine-2-carboxylic acid N-(((butyloxy)-carbonyl)-methyl)-amide, 5-(((3-(1-butyloxy)-propyl)-amino)-carbonyl)-3-hydroxypyridine-2-carboxylic acid N-((benzyloxycarbonyl)-methyl)-amide, 5-(((3-(1-butyloxy)-propyl)-amino)-carbonyl)-3-hydroxypyridine-2-carboxylic acid N-(((1-butyloxy)-carbonyl)-methyl)-amide, and 5-(((3-lauryloxy)-propyl)amino)-carbonyl)-3-hydroxypyridine-2-carboxylic acid N-(((benzyloxy)-carbonyl)-methyl)-amide. In other embodiments, compounds of Formula (Ia) as defined above include, but are not limited to, N-((6-(1-butyloxy)-3-hydroxyquinolin-2-yl)-carbonyl)-glycine, N-((6-chloro-3-hydroxyquinolin-2-yl)-carbonyl)-glycine, N-((3-hydroxy-6-(2-propyloxy)-quinolin-2-yl)-carbonyl)-glycine, N-((7-chloro-3-hydroxy-quinoline-2-carbonyl)-amino]-acetic acid, [(3-benzyloxy-7-chloro-quinoline-2-carbonyl)-amino]-acetic acid, [(3-hydroxy-6-isopropoxy-quinoline-2-carbonyl)-amino]-acetic acid, [(3-hydroxy-6-phenoxy-quinoline-2-carbonyl)-amino]-acetic acid, and [(3-hydroxy-6-trifluoromethoxy-quinoline-2-carbonyl)-amino]-acetic acid. In still other embodiments, compounds of Formula (Ib) as defined above include, but are not limited to, N-((1-chloro-4-hydroxy-7-(2-propyloxy) isoquinolin-3-yl)-carbonyl)-glycine, N-((7-bromo-4-hydroxy-isoquinoline-3-carbonyl)-amino)-acetic acid, N-((1-chloro-4-hydroxy-6-(2-propyloxy) isoquinolin-3-yl)-carbonyl)-glycine, N-((1-chloro-4-hydroxy-7-methoxyisoquinolin-3-yl)-carbonyl)-glycine, N-((1-chloro-4-hydroxy-6-methoxyisoquinolin-3-yl)-carbonyl)-glycine, [(7-butoxy-1-chloro-4-hydroxy-isoquinoline-3-carbonyl)-amino]-acetic acid, N-((7-benzyloxy-1-chloro-4-hydroxyisoquinolin-3-yl)-carbonyl)-glycine, N-((6-benzyloxy-1-chloro-4-hydroxyisoquinolin -3-yl)-carbonyl)-glycine, [(1-chloro-4-hydroxy-isoquinoline-3-carbonyl)-amino]-acetic acid, N-((8-chloro-4-hydroxyisoquinolin-3-yl)-carbonyl)-glycine, and [(7-butoxy-4-hydroxy-isoquinoline-3-carbonyl)-amino]-acetic acid.

In one aspect, a compound of the invention increases endogenous erythropoietin plasma levels by increasing synthesis of erythropoietin in tissues, such as renal, hepatic, hematopoietic, and/or neural tissues, *in vivo.* In one embodiment, the compound increases erythropoietin synthesis by inhibiting hydroxylation of the alpha subunit of hypoxia inducible factor (HIFα), thereby stabilizing HIF within a cell. In one specific embodiment, the agent inhibits hydroxylation of the HIF-1α P₅₆₄ residue or a homologous proline in another HIFα isoform. In another specific embodiment, the agent inhibits hydroxylation of the HIF-1α P₄₀₂ residue or a homologous proline in another HIFα isoform. In yet another embodiment, the compound may additionally inhibit hydroxylation of HIFα asparagine residues. In one specific embodiment, the agent inhibits hydroxylation of the HIF-1α N₈₀₃ residue or a homologous asparagine residue in another HIFα isoform.

Disclosed herein for reference only are methods for identifying compounds that increase endogenous erythropoietin plasma levels, the methods comprising administering a compound of interest to, e.g., an animal or to cultured cells and measuring erythropoietin in, e.g., the blood or conditioned culture media, respectively. An increase in EPO in treated animals or cells relative to untreated controls is indicative of a compound that increases endogenous EPO. Alternatively, the methods identify compounds that indirectly increase synthesis of erythropoietin by stabilizing HIFα in cells.

The compounds of the invention can be administered in combination with various other therapeutic approaches. In one embodiment, the compound is administered with an iron supplement, e.g., ferrous sulfate, vitamin B₁₂, and/or folic acid. In another embodiment, the compound is administered in conjunction with administration of exogenous erythropoietin, e.g., recombinant human erythropoietin, and/or granulocyte-colony stimulating factor (G-CSF), e.g., recombinant G-CSF.

### BRIEF DESCRIPTION OF THE DRAWINGS

Figure 1 shows erythropoietin induction *in vitro* in response to compounds of the invention. Cells in culture were treated with compounds at the concentrations indicated. Cell types shown in the figure are human liver cells derived from a hepatocellular carcinoma (Hep3B).
Figures 2A, 2B, and 2C show erythropoietin induction and subsequent hematocrit increase in animals treated with a compound of the invention. Figure 2A shows expression of erythropoietin transcript in the liver and kidney of animals treated for 3 days with either a vehicle control (0 mg compound/kg body weight/day) or a compound of the invention. Figure 2B shows erythropoietin levels in plasma, and Figure 2C shows blood hematocrit, in blood samples collected 4 hours after final treatment from the same animals represented in Figure 2A.
Figures 3A and 3B show increase in plasma erythropoietin and resulting increase in hematocrit in animals treated with compounds of the invention. Figure 3A shows an increase in plasma erythropoietin two days after treatment with compound. Figure 3B shows the increase in hematocrit 2 and 7 days after treatment with various compounds of the invention.
Figures 4A, 4B, 4C, and 4D show changes in serum erythropoietin, circulating blood reticulocytes, blood hemoglobin level, and hematocrit, respectively, in animals treated with variable dosing regimens of a compound of the invention.
Figures 5A and 5B show changes in hematocrit and circulating blood reticulocytes in animals exposed to a single dose of cisplatin and subsequently treated with a compound of the invention.
Figure 6A, 6B, and 6C show expression of erythropoietin transcripts in the brain, liver, and kidney, respectively, in animals treated with a compound of the invention.
Figure 7 shows increases in endogenous erythropoietin levels in sham-operated and bilaterally nephrectomized animals treated with a compound of the invention relative to untreated sham and BN controls.

### DESCRIPTION OF THE INVENTION

Before the present invention is described, it is to be understood that the invention is not limited to the particular methodologies, protocols, cell lines, assays, and reagents described, as these may vary.

It must be noted that as used herein and in the appended claims, the singular forms "a," "an," and "the" include plural references unless context clearly dictates otherwise. Thus, for example, a reference to "a fragment" includes a plurality of such fragments, a reference to an "antibody" is a reference to one or more antibodies and to equivalents thereof known to those skilled in the art, and so forth.

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. All publications cited herein are mentioned for the purpose of describing and disclosing the methodologies, reagents, and tools reported in the publications which might be used in connection with the invention.

The practice of the present invention will employ, unless otherwise indicated, conventional methods of chemistry, biochemistry, molecular biology, cell biology, genetics, immunology and pharmacology, within the skill of the art. Such techniques are explained fully in the literature. (See, e.g., Gennaro, A.R., ed. (1990) Remington's Pharmaceutical Sciences, 18th ed., Mack Publishing Co.; Colowick, S. et al., eds., Methods In Enzymology, Academic Press, Inc.; Handbook of Experimental Immunology, Vols. I-IV (D.M. Weir and C.C. Blackwell, eds., 1986, Blackwell Scientific Publications); Maniatis, T. et al., eds. (1989) Molecular Cloning: A Laboratory Manual, 2nd edition, Vols. I-III, Cold Spring Harbor Laboratory Press; Ausubel, F. M. et al., eds. (1999) Short Protocols in Molecular Biology, 4th edition, John Wiley & Sons; Ream et al., eds. (1998) Molecular Biology Techniques: An Intensive Laboratory Course, Academic Press); PCR (Introduction to Biotechniques Series), 2nd ed. (Newton & Graham eds., 1997, Springer Verlag).)

### DEFINITIONS

The term "anemia" as used herein refers to any abnormality in hemoglobin or erythrocytes that leads to reduced oxygen levels in the blood. Anemia can be associated with abnormal production, processing, or performance of erythrocytes and/or hemoglobin. The term anemia refers to any reduction in the number of red blood cells and/or level of hemoglobin in blood relative to normal blood levels.

Anemia can arise due to conditions such as acute or chronic kidney disease, infections, inflammation, cancer, irradiation, toxins, diabetes, and surgery. Infections may be due to, e.g., virus, bacteria, and/or parasites. Inflammation may be due to infection, autoimmune disorders, such as rheumatoid arthritis. Anemia can also be associated with blood loss due to, e.g., stomach ulcer, duodenal ulcer, hemorrhoids, cancer of the stomach or large intestine, trauma, injury, surgical procedures. Anemia is further associated with radiation therapy, chemotherapy, and kidney dialysis. Anemia is also associated with HIV-infected patients undergoing treatment with azidothymidine (zidovudine) or other reverse transcriptase inhibitors, and can develop in cancer patients undergoing chemotherapy, e.g., with cyclic cisplatin- or non-cisplatin-containing chemotherapeutics. Aplastic anemia and myelodysplastic syndromes are diseases associated with bone marrow failure that result in decreased production of erythrocytes. Further, anemia can result from defective or abnormal hemoglobin or erythrocytes, such as in disorders including microcytic anemia, hypochromic anemia. Anemia can result from disorders in iron transport, processing, and utilization, see, e.g., sideroblastic anemia.

The terms "disorders" and "diseases" and "conditions" are used inclusively and refer to any condition deviating from normal.

The terms "anemic conditions" and "anemic disorders" refer to any condition, disease, or disorder associated with anemia. Such disorders include those disorders listed above. Anemic disorders further include aplastic anemia, autoimmune hemolytic anemia, bone marrow transplantation, Churg-Strauss syndrome, Diamond Blackfan anemia, Fanconi's anemia, Felty syndrome, graft versus host disease, hematopoietic stem cell transplantation, hemolytic uremic syndrome, myelodysplasic syndrome, nocturnal paroxysmal hemoglobinuria, osteomyelofibrosis, pancytopenia, pure red-cell aplasia, purpura Schoenlein-Henoch, sideroblastic anemia, refractory anemia with excess of blasts, rheumatoid arthritis, Shwachman syndrome, sickle cell disease, thalassemia major, thalassemia minor, thrombocytopenic purpura.

The term "erythropoietin-associated conditions" is used inclusively and refers to any condition associated with below normal, abnormal, or inappropriate modulation of erythropoietin. Erythropoietin-associated conditions include any condition wherein an increase in EPO level would provide therapeutic benefit. Levels of erythropoietin associated with such conditions can be determined by any measure accepted and utilized by those of skill in the art. Erythropoietin-associated conditions include anemic conditions such as those described above.

Erythropoietin-associated conditions further include neurological disorders and/or injuries, including cases of stroke, trauma, epilepsy, neurodegenerative disease and the like, wherein erythropoietin may provide a neuroprotective effect. Neurodegenerative diseases contemplated by the invention include Alzheimer's disease, Parkinson's disease, Huntington's disease, and the like.

The term "erythropoietin" refers to any recombinant or naturally occurring erythropoietin including, e.g., human erythropoietin (GenBank Accession No. AAA52400; Lin et al. (1985) Proc Natl Acad Sci USA 82:7580-7584), EPOETIN human recombinant erythropoietin (Amgen, Inc., Thousand Oaks CA), ARANESP human recombinant erythropoietin (Amgen), PROCRIT human recombinant erythropoietin (Ortho Biotech Products, L.P., Raritan NJ).

The term "HIFα" refers to the alpha subunit of hypoxia inducible factor protein. HIFα may be any human or other mammalian protein, or fragment thereof, including human HIF-1α (Genbank Accession No. Q16665), HIF-2α (Genbank Accession No. AAB41495), and HIF-3α (Genbank Accession No. AAD22668); murine HIF-1α (Genbank Accession No. Q61221), HIF-2α (Genbank Accession No. BAA20130 and AAB41496), and HIF-3α (Genbank Accession No. AAC72734); rat HIF-1α (Genbank Accession No. CAA70701), HIF-2α (Genbank Accession No. CAB96612), and HIF-3α (Genbank Accession No. CAB96611); and bovine HIF-1α (Genbank Accession No. BAA78675). HIFα may also be any non-mammalian protein or fragment thereof, including Xenopus laevis HIF-1α (Genbank Accession No. CAB96628), Drosophila melanogaster HIF-1α (Genbank Accession No. JC4851), and chicken HIF-1α (Genbank Accession No. BAA34234). HIFα gene sequences may also be obtained by routine cloning techniques, for example by using all or part of a HIFα gene sequence described above as a probe to recover and determine the sequence of a HIFα gene in another species.

A fragment of HIFα includes any fragment retaining at least one functional or structural characteristic of HIFα. Fragments of HIFα include, e.g., the regions defined by human HIF-1α from amino acids 401 to 603 (Huang et al., *supra*), amino acid 531 to 575 (Jiang et al. (1997) J Biol Chem 272:19253-19260), amino acid 556 to 575 (Tanimoto et al., *supra*), amino acid 557 to 571 (Srinivas et al. (1999) Biochem Biophys Res Commun 260:557-561), and amino acid 556 to 575 (Ivan and Kaelin (2001) Science 292:464-468). Further, HIFα fragments include any fragment containing at least one occurrence of the motif LXXLAP, e.g., as occurs in the human HIF-1α native sequence at L₃₉₇TLLAP and L₅₅₉EMLAP. For example, a HIF peptide for use in the screening assay of Example 9 may comprise [methoxycoumarin]-DLDLEALAPYIPADDDFQL-amide (SEQ ID NO:5).

The terms "amino acid sequence" or "polypeptide" as used herein, e.g., to refer to HIFα and fragments thereof, contemplate an oligopeptide, peptide, or protein sequence, or to a fragment of any of these, and to naturally occurring or synthetic molecules. "Fragments" can refer to any portion of a sequence that retains at least one structural or functional characteristic of the protein. Immunogenic fragments or antigenic fragments are fragments of polypeptides, preferably, fragments of about five to fifteen amino acids in length, that retain at least one biological or immunological activity. Where "amino acid sequence" is used to refer to the polypeptide sequence of a naturally occurring protein molecule, "amino acid sequence" and like terms are not meant to limit the amino acid sequence to the complete native sequence associated with the recited protein molecule.

The term "related proteins" as used herein, for example, to refer to proteins related to HIFα prolyl hydroxylase, encompasses other 2-oxoglutarate dioxygenase enzymes, especially those family members that similarly require Fe²⁺, 2-oxoglutarate, and oxygen to maintain hydroxylase actitity. Such enzymes include e.g., procollagen lysyl hydroxylase, procollagen prolyl 4-hydroxylase, and Factor Inhibiting HIF (FIH), an asparaginyl hydroxylase responsible for regulating transactivation of HIFα. (GenBank Accession No. AAL27308; Mahon et al. (2001) Genes Dev 15:2675-2686; Lando et al. (2002) Science 295:858-861; and Lando et al. (2002) Genes Dev 16:1466-1471. See also Elkins et al. (2002) J Biol Chem C200644200).

The terms "HIF prolyl hydroxylase" and "HIF PH" refer to any enzyme capable of hydroxylating a proline residue in the HIF protein. Preferably, the proline residue hydroxylated by HIF PH includes the proline found within the motif LXXLAP, e.g., as occurs in the human HIF-1α native sequence at L₃₉₇TLLAP and L₅₅₉EMLAP. HIF PH includes members of the Egl-Nine (EGLN) gene family described by Taylor (2001,Gene 275:125-132), and characterized by Aravind and Koonin (2001, Genome Biol 2:RESEARCH0007), Epstein et al. (2001, Cell 107:43-54), and Bruick and McKnight (2001, Science 294:1337-1340). Examples of HIF PH enzymes include human SM-20 (EGLN1) (GenBank Accession No. AAG33965; Dupuy et al. (2000) Genomics 69:348-54), EGLN2 isoform 1 (GenBank Accession No. CAC42510; Taylor, *supra*), EGLN2 isoform 2 (GenBank Accession No. NP_060025), and EGLN3 (GenBank Accession No. CAC42511; Taylor, *supra*); mouse EGLN1 (GenBank Accession No. CAC42515), EGLN2 (GenBank Accession No. CAC42511), and EGLN3 (SM-20) (GenBank Accession No. CAC42517); and rat SM-20 (GenBank Accession No. AAA19321). Additionally, HIF PH may include *Caenorhabditis elegans* EGL-9 (GenBank Accession No. AAD56365) and *Drosophila melanogaster* CG1114 gene product (GenBank Accession No. AAF52050). HIF PH also includes any fragment of the foregoing full-length proteins that retain at least one structural or functional characteristic.

The term "agonist" refers to a molecule that increases or prolongs the duration of the effect of a particular molecule. Agonists may include proteins, nucleic acids, carbohydrates, or any other molecules that increase the effect(s) of the target molecule.

The term "antagonist" refers to a molecule which decreases the extent or duration of the effect of the biological or immunological activity of a particular molecule. Antagonists may include proteins, nucleic acids, carbohydrates, antibodies, or any other molecules that decrease the effect(s) of the target molecule.

The term "microarray" refers to any arrangement of nucleic acids, amino acids, antibodies, etc., on a substrate. The substrate can be any suitable support, e.g., beads, glass, paper, nitrocellulose, nylon, or any appropriate membrane. A substrate can be any rigid or semi-rigid support including, but not limited to, membranes, filters, wafers, chips, slides, fibers, beads, including magnetic or nonmagnetic beads, gels, tubing, plates, polymers, microparticles, capillaries. The substrate can provide a surface for coating and/or can have a variety of surface forms, such as wells, pins, trenches, channels, and pores, to which the nucleic acids, amino acids, may be bound.

The term "excipient" as used herein means an inert or inactive substance used in the production of pharmaceutical products or other tablets, including without limitation any substance used as a binder, disintegrant, coating, compression/encapsulation aid, cream or lotion, lubricant, parenteral, sweetener or flavoring, suspending/gelling agent, or wet granulation agent. Binders include, e.g., carbopol, povidone, xanthan gum; coatings include, e.g., cellulose acetate phthalate, ethylcellulose, gellan gum, maltodextrin; compression/encapsulation aids include, e.g., calcium carbonate, dextrose, fructose dc, honey dc, lactose (anhydrate or monohydrate; optionally in combination with aspartame, cellulose, or microcrystalline cellulose), starch dc, sucrose; disintegrants include, e.g., croscarmellose sodium, gellan gum, sodium starch glycolate; creams and lotions include, e.g., maltodextrin, carrageenans; lubricants include, e.g., magnesium stearate, stearic acid, sodium stearyl fumarate; materials for chewable tablets include, e.g., dextrose, fructose dc, lactose (monohydrate, optionally in combination with aspartame or cellulose); parenterals include, e.g., mannitol, povidone; plasticizers include, e.g., dibutyl sebacate, polyvinylacetate phthalate; suspending/gelling agents include, e.g., carrageenan, sodium starch glycolate, xanthan gum; sweeteners include, e.g., aspartame, dextrose, fructose dc, sorbitol, sucrose dc; and wet granulation agents include, e.g., calcium carbonate, maltodextrin, microcrystalline cellulose.

The term "loading dose" as used herein refers to a single or multiple dose administered initially to rapidly achieve the desired pharmacological level. For example, a loading dose in reference to the methods of the invention refers to an initial dosing regimen that rapidly increases, e.g., the plasma concentration of a compound of the invention to a pharmaceutically active level.

The term "induction dose" as used herein refers to a repeated dose strength administered initially to rapidly achieve the desired physiological response. For example, an induction dose in reference to the methods of the invention refers to an initial dosing regimen that rapidly increases the hematocrit or hemoglobin level to within a target range, which may be at or below normal hematocrit/hemoglobin levels.

The term "maintenance dose" as used herein refers to the dose level administered after a loading or induction dose in order to maintain a desired physiological response. For example, a maintenance dose in reference to the methods of the invention refers to a dosing regimen that maintains hematocrit and/or hemoglobin within a desired target range, which may be at or below normal hematocrit/hemoglobin levels.

The term "sample" is used herein in its broadest sense. Samples may be derived from any source, for example, from bodily fluids, secretions, tissues, cells, or cells in culture including, but not limited to, saliva, blood, urine, serum, plasma, vitreous, synovial fluid, cerebral spinal fluid, amniotic fluid, and organ tissue (e.g., biopsied tissue); from chromosomes, organelles, or other membranes isolated from a cell; from genomic DNA, cDNA, RNA, mRNA; and from cleared cells or tissues, or blots or imprints from such cells or tissues. Samples may be derived from any source, such as, for example, a human subject, or a non-human mammalian subject. Also contemplated are samples derived from any animal model of disease. A sample can be in solution or can be, for example, fixed or bound to a substrate. A sample can refer to any material suitable for testing for the presence of erythropoietin or HIFα or to fragments thereof, or suitable for screening for molecules that increase endogenous levels of erythropoietin or HIFα or to fragments thereof. Methods for obtaining such samples are within the level of skill in the art.

The term "subject" is used herein in its broadest sense. Subjects may include isolated cells, either prokaryotic or eukaryotic, or tissues grown in culture. In certain embodiments, a subject is an animal, particularly an animal selected from a mammalian species including rat, rabbit, bovine, ovine, porcine, canine, feline, murine, equine, and primate, particularly human.

### INVENTION

The present invention relates to compounds of Formula (I) as defined herein that inhibit HIF prolyl hydroxylase enzyme activity for use in increasing endogenous erythropoietin in treating or preventing anemia associated with kidney disease in a subject. Disclosed herein are methods of increasing endogenous erythropoietin (EPO). These methods can be applied *in vivo,* e.g., in blood plasma, or *in vitro,* e.g., in cell culture conditioned media. The invention provides in one aspect compounds for use in methods of treating or preventing conditions associated with the anemia. Conditions associated with the anemia include disorders such as acute or chronic kidney disease, diabetes, cancer, ulcers, infection with virus, e.g., HIV, bacteria, or parasites; inflammation. Anemic conditions can further include those associated with procedures or treatments including, e.g., radiation therapy, chemotherapy, dialysis, and surgery. Disorders associated with the anemia additionally include abnormal hemoglobin and/or erythrocytes, such as found in disorders such as microcytic anemia, hypochromic anemia, aplastic anemia.

The compounds of the invention have been shown to increase endogenous erythropoietin levels in media from cultured cells treated *in vitro* and in blood plasma from animals treated *in vivo.* Although the kidney is the major source of erythropoietin in the body, other organs, including brain, liver, and bone marrow, can and do synthesize erythropoietin upon appropriate stimulation. Using the compounds of the invention, endogenous erythropoietin expression can be increased in various organs of the body, including brain, kidney, and liver. Indeed, compounds of the invention even increase endogenous erythropoietin levels in animals that have undergone bilateral nephrectomy.

The compounds of the invention demonstrate that erythropoietin levels can be increased even when kidney function is compromised. Although the invention is not to be limited by the mechanism by which erythropoietin is produced, the decrease in erythropoietin secretion typically seen during kidney failure may be due to hyperoxia in renal tissue due to increased flowthrough/reperfusion. (Priyadarshi et al. (2002) Kidney Int 61:542-546.)

Further, the compounds of the invention increase the hematocrit and blood hemoglobin level in animals treated *in vivo.* The increases in plasma EPO, hematocrit, and blood hemoglobin in response to the compounds of the invention are dose-sensitive; however, dosing regimes can be established which produce a constant, controlled level of response to the compounds of the invention. Further, treatment with compounds of the invention can correct anemia, for example, induced by a toxic compound such as the chemotherapeutic agent cisplatin, or due to blood loss, e.g., trauma, injury, parasites, or surgery.

The increase in hematocrit and blood hemoglobin in animals treated with compounds of the invention is preceded by an increase in the percentage of circulating immature red blood cells (reticulocytes) within the blood. As such, the disclosure contemplates compounds for use in methods to increase reticulocyte levels in the blood of animals for production of cell-free reticulocyte lysates as described by, e.g., Pelham and Jackson. (1976, Eur J Biochem 67:247-256.) Circulating reticulocyte levels are increased in animals, e.g., rabbits, by treatment with compounds of the invention, alone or in combination with another compound such as, e.g., acetylphenylhydrazine. The blood is collected, and reticulocytes are pelleted by centrifugation and lysed with distilled water. Extracts can be further processed using any appropriate methodology known to those skilled in the art. (See, e.g., Jackson and Hunt (1983) Methods Enzymol 96:50-74.)

The invention also contemplates compounds for use in methods of prevention or treatment of anemia associated with kidney disease, the methods involving increasing iron transport, processing, and utilization. (See, e.g., commonly owned, copending U.S. Patent Application entitled "Stabilization of Hypoxia Inducible Factor (HIF) Alpha," filed of even date.) Specifically, the compounds of the invention may increase enzymes and proteins involved in iron uptake, transport, and processing. Such enzymes and proteins include transferrin and transferrin receptor, which together facilitate iron transport to and uptake by, e.g., erythroid tissue, and ceruloplasmin, a ferroxidase required to oxidize ferrous iron to ferric iron. As transferrin can only bind and transport ferric iron, ceruloplasmin is important for supply of iron to tissues. The ability of the compounds of the invention to increase both endogenous erythropoietin and transport and utilization of iron in a single course of treatment provides benefits not addressed by current anemia therapeutics, such as administration of recombinant erythropoietin, in the treatment of anemic disorders including rheumatoid arthritis, sideroblastic anemia.

The compounds that inhibit HIF prolyl hydroxylase enzyme activity increase synthesis of endogenous erythropoietin by inhibiting hydroxylation of the alpha subunit of hypoxia inducible factor (HIFα). More specifically, the compounds inhibit hydroxylation of HIFα proline residues, e.g., the P₅₆₄ residue in HIF-1α or a homologous proline in another HIFα isoform, or the P₄₀₂ residue in HIF-1α or a homologous proline in another HIFα isoform. Additionally, the compounds may be used to inhibit hydroxylation of HIFα asparagine residues, e.g., the N₈₀₃ residue of HIF-1α or a homologous asparagine residue in another HIFα isoform.

As HIFα is modified by hydroxylation, a reaction requiring oxygen and Fe2⁺, the present invention contemplates in one aspect that the enzyme responsible for HIFα hydroxylation is a member of the 2-oxoglutarate dioxygenase family. Such enzymes include procollagen lysyl hydroxylase, procollagen prolyl 3-hydroxylase, procollagen prolyl 4-hydroxylase α(I) and α(II), thymine 7-hydroxylase, aspartyl (asparaginyl) β-hydroxylase, ε-N-trimethyllysine hydroxylase, γ-butyrobetaine hydroxylase. These enzymes require oxygen, Fe2⁺, 2-oxoglutarate, and ascorbic acid for their hydroxylase activity. (See, e.g., Majamaa et al. (1985) Biochem J 229:127-133; Myllyharju and Kivirikko (1997) EMBO J 16:1173-1180; Thornburg et al. (1993) 32:14023-14033; and Jia et al. (1994) Proc Natl Acad Sci USA 91:7227-7231.)

Several small molecule inhibitors of prolyl 4-hydroxylase have been identified. (See, e.g., Majamaa et al., *supra*; Kivirikko and Myllyharju (1998) Matrix Biol 16:357-368; Bickel et al. (1998) Hepatology 28:404-411; Friedman et al. (2000) Proc Natl Acad Sci USA 97:4736-4741; and Franklin et al. (2001) Biochem J 353:333-338.) The present invention contemplates the use of these compounds that are of Formula (I) as defined herein.

Compounds that can be used in the invention include, e.g., structural mimetics of 2-oxoglutarate of Formula (I) as defined herein. Such compounds may inhibit the target 2-oxoglutarate dioxygenase family member competitively with respect to 2-oxoglutarate and noncompetitively with respect to iron. (Majamaa et al. (1984) Eur J Biochem 138:239-45; and Majamaa et al., *supra*.)

The compounds of the invention are of the formula (I) 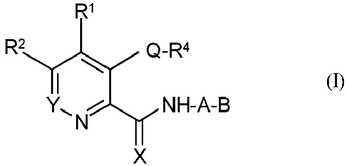 wherein
A is (C₁-C₄)-alkylene.
B is -CO₂H, -NH₂, -NHSO₂CF₃, tetrazolyl, imidazolyl, 3-hydroxyisoxazolyl, -CONHCOR'", -CONHSOR"', CONHSO₂R"', where R'" is aryl, heteroaryl, (C₃-C₇)-cycloalkyl, or (C₁-C₄)-alkyl, optionally monosubstituted by (C₆-C₁₂)-aryl, heteroaryl, OH, SH, (C₁-C₄)-alkyl, (C₁-C₄)-alkoxy, (C₁-C₄)-thioalkyl, (C₁-C₄)-sulfinyl, (C₁-C₄)-sulfonyl, CF₃, Cl, Br, F, I, NO2, -COOH, (C₂-C₅)-alkoxycarbonyl, NH₂, mono-(C₁-C₄-alkyl)-amino, di-(C₁-C₄-alkyl)-amino, or (C₁-C₄)-perfluoroalkyl; or wherein B is a CO₂-G carboxyl radical, where G is a radical of an alcohol G-OH in which G is selected from (C₁-C₂₀)-alkyl radical, (C₃-C₈) cycloalkyl radical, (C₂-C₂₀)-alkenyl radical, (C₃-C₈)-cycloalkenyl radical, retinyl radical, (C₂-C₂₀)-alkynyl radical, (C₄-C₂₀)-alkenynyl radical, where the alkenyl, cycloalkenyl, alkynyl, and alkenynyl radicals contain one or more multiple bonds; (C₆-C₁₆)-carbocyclic aryl radical, (C₇-C₁₆)-carbocyclic aralkyl radical, heteroaryl radical, or heteroaralkyl radical, wherein a heteroaryl radical or heteroaryl moiety of a heteroaralkyl radical contains 5 or 6 ring atoms; and wherein radicals defined for G are substituted by one or more hydroxyl, halogen, cyano, trifluoromethyl, nitro, carboxyl, (C₁-C₁₂)-alkyl, (C₃-C₈)-cycloalkyl, (C₅-C₈)-cycloalkenyl, (C₆-C₁₂)-aryl, (C₇-C₁₆)-aralkyl, (C₂-C₁₂)-alkenyl, (C₂-C₁₂)-alkynyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, (C₆-C₁₂)-aryloxy, (C₇-C₁₆)-aralkyloxy, (C₁-C₈)-hydroxyalkyl, -O-[CH₂]ₓ-C_{f}H_{(2f+1-g)}-F_{g}, -OCF₂Cl, -OCF₂-CHFCl, (C₁-C₁₂)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₂-C₁₂)-alkenylcarbonyl, (C₂-C₁₂)-alkynylcarbonyl, (C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, acyloxy, (C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆) aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy, carbamoyl, N-(C₁-C₁₂)-alkylcarbamoyl, N.N-di(C₁-C₁₂)-alkylcarbamoyl, N-(C₃-C₈)-cycloalkyl-carbamoyl, N-(C₆-C₁₆)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₀)-alkoxy-(C₁-C₁₀)-alkyl)-carbamoyl, N-((C₆-C₁₂)-aryloxy-(C₁-C₁₀)alkyl)-carbamoyl, N-((C₇-C₁₆)-aralkyloxy-(C₁-C₁₀)-alkyl)-carbamoyl, N-(C₁-C₁₀)-alkyl-N-((C₁-C₁₀)-alkoxy-(C₁-C₁₀)-alkyl)-carbamoyl, N-(C₁-C₁₀)-alkyl-N-((C₆-C₁₆)-aryloxy-(C₁-C₁₀)-alkyl)-carbamoyl, N-(C₁-C₁₀)-alkyl-N-((C₇-C₁₆)-aralkyloxy-(C₁-C₁₀)-alkyl)-carbamoyl, carbamoyloxy, N-(C₁-C₁₂)-alkylcarbamoyloxy, N.N-di-(C₁-C₁₂)-alkylcarbamoyloxy, N-(C₃-C₈)-cycloalkylcarbamoyloxy, N-(C₆-C₁₂)-arylcarbamoyloxy, N-(C₇-C₁₆)-aralkylcarbamoyloxy, N-(C₁-C₁₀)-alkyl-N-(C₆-C₁₂)-arylcarbamoyloxy, N(C₁-C₁₀)-alkyl-N-(C₇-C₁₆)-aralkylcarbamoyloxy, N-((C₁-C₁₀)-alkyl)-carbamoyloxy, N-((C₆-C₁₂)-aryloxy-(C₁-C₁₀)-alkyl)-carbamoyloxy, N-((C₇-C₁₆)-aralkyloxy-(C₁-C₁₀)-alkyl)-carbamoyloxy, N-(C₁-C₁₀)-alkyl-N-((C₁-C₁₀)-alkoxy-(C₁-C₁₀)-alkyl)-carbamoyloxy, N-(C₁-C₁₀)-alkyl-N-((C₆-C₁₂)-aryloxy-(C₁-C₁₀)-alkyl)-carbamoyloxy, N-(C₁-C₁₀)-alkyl-N-((C₇-C₁₆)-aralkyloxy-(C₁-C₁₀)-alkyl)-carbamoyloxy, amino, (C₁-C₁₂)-alkylamino, di-(C₁-C₁₂)-alkylamino, (C₃-C₈)-cycloalkylamino, (C₂-C₁₂)-alkenylamino, (C₂-C₁₂)-alkynylamino, N-(C₆-C₁₂)-arylamino, N-(C-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino, (C₁-C₁₂)-alkylcarbonylamino, (C₃-C₈)-cycloalkylcarbonylamino, (C₆-C₁₂) arylcarbonylamino, (C₇-C₁₆)-aralkylcarbonylamino, (C₁-C₁₂)-alkylcarbonyl-N-(C₁-C₁₀)-alkylamino, (C₃-C₈)-cycloalkylcarbonyl-N-(C₁-C₁₀ )-alkylamino, (C₆-C₁₂)-arylcarbonyl-N-(C₁-C₁₀)alkylamino, (C₇-C₁₁)-aralkylcarbonyl-N-(C₁-C₁₀)-alkylamino, (C₁-C₁₂)-alkylcarbonylamino-(C₁-C₈)-alkyl, (C₃-C₈)-cycloalkylcarbonylamino-(C₁-C₈)alkyl, (C₆-C₁₂)-arylcarbonylamino-(C₁-C₈)-alkyl, (C₇-C₁₂)-aralkylcarbonylamino(C-C₈)-alkyl, amino-(C₁-C₁₀)-alkyl, N-(C₁-C₁₀) alkylamino-(C₁-C₁₀)-alkyl, N.N-di-(C₁-C₁₀)-alkylamino-(C₁-C₁₀)-alkyl, (C₃-C₈)cycloalkylamino-(C₁-C₁₀)-alkyl, (C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₂)-arylsulfonyl, (C₇-C₁₆)-aralkylmercapto, (C₇-C₁₆)-aralkylsulfinyl, (C₇-C₁₆)-aralkylsulfonyl, sulfamoyl, N-(C₁-C₁₀)-alkylsulfamoyl, N.N-di(C₁-C₁₀)-alkylsulfamoyl, (C₃-C₈)-cycloalkylsulfamoyl, N-(C₆-C₁₂)-alkylsulfamoyl, N-(C₇-C₁₆)-aralkylsulfamoyl, N-(C₁-C₁₀)-alkyl-N-(C₆-C₁₂)-arylsulfamoyl, N-(C₁-C₁₀)-alkyl-N-(C₇-C₁₆)-aralkylsulfamoyl, (C₁-C₁₀)-alkylsulfonamido, N-((C₁-C₁₀)-alkyl)-(C₁-C₁₀)-alkylsulfonamido, (C₇-C₁₆)-aralkylsulfonamido, or N-((C₁-C₁₀)-alkyl-(C₇-C₁₆)-aralkylsulfonamido; wherein radicals which are aryl or contain an aryl moiety, may be substituted on the aryl by one to five identical or different hydroxyl, halogen, cyano, trifluoromethyl, nitro, carboxyl, (C₁-C₁₂)-alkyl, (C₃-C₈)-cycloalkyl, (C₆-C₁₂)-aryl, (C₇-C₁₆)-aralkyl, (C₁-C₁₂)-alkoxy, (C₁-C₁₂)-alkoxy-(C₁-C₁₂)alkyl, (C₁-C₁₂)-alkoxy-(C₁ C₁₂)alkoxy, (C₆-C₁₂)-aryloxy, (C₇-C₁₆)-aralkyloxy, (C₁-C₈)-hydroxyalkyl, (C₁-C₁₂)-alkylcarbonyl, (C₃-C₈)-cycloalkyl-carbonyl, (C₆-C₁₂)-arylcarbonyl, (C₇-C₁₆) aralkylcarbonyl, (C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, (C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy, (C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy, carbamoyl, N-(C₁-C₁₂)-alkylcarbamoyl, N.N-di-(C₁-C₁₂)-alkylcarbamoyl, N-(C₃-C₈)-cycloalkylcarbamoyl, N-(C₆-C₁₂)-arylcarbamoyl, N-(C₇-C₁₆)-aralkylcarbamoyl, N-(C₁-C₁₀)-alkyl-N-(C₆-C₁₂)-arylcarbamoyl, N-(C₁-C₁₀)-alkyl-N-(C₇-C₁₆)-aralkylcarbamoyl, N-((C₁-C₁₀)-alkoxy-(C₁-C₁₀)-alkyl)-carbamoyl, N-((C₆-C₁₂)-aryloxy-(C₁-C₁₀)-alkyl)-carbamoyl, N-((C₇-C₁₆)-aralkyloxy-(C₁-C₁₀)-alkyl)-carbamoyl, N-(C₁-C₁₀)-alkyl-N-((C₁-C₁₀)-alkoxy-(C₁-C₁₀)-alkyl)-carbamoyl, N-(C₁-C₁₀)-alkyl-N-((C₆-C₁₂)-aryloxy-(C₁-C₁₀)-alkyl)-carbamoyl, N-(C₁-C₁₀)-alkyl-N-((C₇-C₁₆)-aralkyloxy-(C₁-C₁₀)-alkyl)-carbamoyl, carbamoyloxy, N-(C₁-C₁₂)-alkylcarbamoyloxy, N.N-di-(C₁-C₁₂)-alkylcarbamoyloxy, N-(C₃-C₈)-cycloalkylcarbamoyloxy, N-(C₆-C₁₂)-arylcarbamoyloxy, N-(C₇-C₁₆)-aralkylcarbamoyloxy, N-(C₁-C₁₀)-alkyl-N-(C₆-C₁₂)-arylcarbamoyloxy, N(C₁-C₁₀)-alkyl-N-(C₇-C₁₆)-aralkylcarbamoyloxy, N-((C₁-C₁₀)-alkyl)-carbamoyloxy, N-((C₆-C₁₂)-aryloxy-(C₁-C₁₀)-alkyl)-carbamoyloxy, N-((C₇-C₁₆)-aralkyloxy-(C₁-C₁₀)-alkyl)-carbamoyloxy, N-(C₁-C₁₀)-alkyl-N-((C₁-C₁₀)-alkoxy-(C₁-C₁₀)-alkyl)-carbamoyloxy, N-(C₁-C₁₀)-alkyl-N-((C₆-C₁₂)-aryloxy-(C₁-C₁₀)-alkyl)-carbamoyloxy, N-(C₁-C₁₀)-alkyl-N-((C₇-C₁₆)-aralkyloxy-(C₁-C₁₀)-alkyl)-carbamoyloxy, amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkylaralkylamino, N-alkyl-arylamino, (C₁-C₁₂)-alkoxyamino, (C₁-C₁₂)-alkoxy-N-(C₁-C₁₀)-alkylamino, (C₁-C₁₂)-alkylcarbonylamino, (C₃-C₈)-cycloalkylcarbonylamino, (C₆-C₁₂)-arylcarbonylamino, (C₇-C₁₆)-alkylcarbonylamino, (C₁-C₁₂)-alkylcarbonyl-N-(C₁-C₁₀)-alkylamino, (C₃-C₈)-cycloalkylcarbonyl-N-(C₁-C₁₀ )-alkylamino, (C₆-C₁₂)-arylcarbonyl-N-(C₁-C₁₀)-alkylamino, (C₇-C₁₁)-aralkylcarbonyl- N-(C₁-C₁₀ )-alkylamino, (C-C₁₂)-alkylcarbonylamino-(C₁-C₈)-alkyl, (C₃-C₈)-cycloalkylcarbonylamino-(C₁-C₈)-alkyl, (C₆-C₁₂)-arylcarbonylamino-(C₁-C₈)-alkyl, (C₇-C₁₆)-aralkylcarbonylamino-(C₁-C₈)-alkyl, amino-(C₁-C₁₀)-alkyl, N-(C₁-C₁₀)-alkylamino-(C₁-C₁₀)alkyl, N.N-di-(C₁-C₁₀)-alkylamino-(C₁-C₁₀)-alkyl, (C₃-C₈)-cycloalkylamino-(C₁-C₁₀)-alkyl, (C₁-C₁₂)-alkylmercapto, (C₁-C₁₂)-alkylsulfinyl, (C₁-C₁₂)-alkylsulfonyl, (C₆-C₁₂)-arylmercapto, (C₆-C₁₂)-arylsulfinyl, (C₆-C₁₂)-arylsulfonyl, (C₇-C₁₆)-aralkylmercapto, (C₇-C₁₆)-aralkylsulfinyl, or (C₇-C₁₆)-aralkylsulfonyl;
X is O or S;
Q is O, S, NR', or a bond;
where, if Q is a bond, R⁴ is halogen, nitrile, or trifluoromethyl;
or where, if Q is O, S, or NR', R⁴ is hydrogen, (C₁-C₁₀)-alkyl radical, (C₂-C₁₀)-alkenyl radical, (C₂-C₁₀)-alkynyl radical, wherein alkenyl or alkynyl radical contains one or two C-C multiple bonds; unsubstituted fluoroalkyl radical of the formula -[CH₂]ₓ-C_{f}H_{(2f+1-g)}-F_{g}, (C₁-C₈)-alkoxy-(C₁-C₆)-alkyl radical, (C₁-C₆)-alkoxy-(C₁-C₄)-alkoxy-(C₁-C₄)-alkyl radical, aryl radical, heteroaryl radical, (C₇-C₁₁)-aralkyl radical, or a radical of the formula Z

   -[CH₂]ᵥ-[O]_{w}-[CH₂]ₜ-E (Z)

   where
   E is a heteroaryl radical, a (C₃-C₈)-cycloalkyl radical, or a phenyl radical of the formula F 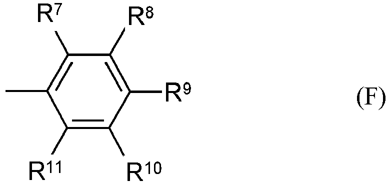
      v is 0-6,
      w is 0 or 1,
      t is 0-3, and
      R⁷, R⁸, R⁹, R¹⁰, and R¹¹ are identical or different and are hydrogen, halogen, cyano, nitro, trifluoromethyl, (C₁-C₆)-alkyl, (C₃-C₈)-cycloalkyl, (C₁-C₆)-alkoxy, -O-[CH₂]ₓ-C_{f}H_{(2f+1-g)}-F_{g}, -OCF₂-Cl, -O-CF₂-CHFCl, (C₁-C₆)-alkylmercapto, (C₁-C₆)-hydroxyalkyl, (C₁-C₆)-alkoxy-(C₁-C₆)-alkoxy, (C₁-C₆)-alkoxy-(C₁-C₆)-alkyl, (C₁-C₆)-alkylsulfinyl, (C₁-C₆)-alkylsulfonyl, (C₁-C₆)-alkylcarbonyl, (C₁-C₈)-alkoxycarbonyl, carbamoyl, N-(C₁-C₈)-alkylcarbamoyl, N,N-di-(C₁-C₈)-alkylcarbamoyl, or (C₇-C₁₁)-aralkylcarbamoyl, optionally substituted by fluorine, chlorine, bromine, trifluoromethyl, (C₁-C₆)-alkoxy, N-(C₃-C₈)-cycloalkylcarbamoyl, N-(C₃-C₈)-cycloalkyl-(C₁-C₄)-alkylcarbamoyl, (C₁-C₆)-alkylcarbonyloxy, phenyl, benzyl, phenoxy, benzyloxy, NR^{Y}R^{Z} wherein R^{y} and R^{z} are independently selected from hydrogen, (C₁-C₁₂)-alkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₃-C₁₀)-cycloalkyl, (C₃-C₁₂)-alkenyl, (C₃-C₁₂)-alkynyl, (C₆-C₁₂)-aryl, (C₇-C₁₁)-aralkyl, (C₁-C₁₂)-alkoxy, (C₇-C₁₂)aralkoxy, (C₁-C₁₂)-alkylcarbonyl, (C₃-C₈)-cycloalkylcarbonyl, (C₆-C₁₂) arylcarbonyl, (C₇-C₁₆)-aralkylcarbonyl; or further wherein R^{y} and R^{z} together are -[CH2]ₕ, in which a CH₂ group can be replaced by O, S, N-(C₁-C₄)-alkylcarbonylimino, or N-(C₁-C₄)-alkoxycarbonylimino; phenylmercapto, phenylsulfonyl, phenylsulfinyl, sulfamoyl, N-(C₁-C₈)-alkylsulfamoyl, or N, N-di-(C₁-C₈)-alkylsulfamoyl; or alternatively R⁷ and R⁸, R⁸ and R⁹, R⁹ and R¹⁰, or R¹⁰ and R¹¹, together are a chain selected from -[CH₂]ₙ- or -CH=CH-CH=CH-, where a CH₂ group of the chain is optionally replaced by O, S, SO, SO₂, or NR^{Y}; and n is 3, 4, or 5; and if E is a heteroaryl radical, said radical can carry 1-3 substituents selected from those defined for R⁷-R¹¹, or if E is a cycloalkyl radical, the radical can carry one substituent selected from those defined for R⁷-R¹¹;
   or where, if Q is NR', R⁴ is alternatively R", where R' and R" are identical or different and are hydrogen, (C₆-C₁₂)-aryl, (C₇-C₁₁)-aralkyl, (C₁-C₈)-alkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkylcarbonyl, optionally substituted (C₇-C₁₆)-aralkylcarbonyl, or optionally substituted C₆-C₁₂)-arylcarbonyl; or R' and R" together are -[CH₂]ₕ, in which a CH₂ group can be replaced by O, S, N-acylimino, or N-(C₁-C₁₀)-alkoxycarbonylimino, and h is 3 to 7.
   Y is N or CR³;
   R¹, R² and R³ are identical or different and are hydrogen, hydroxyl, halogen, cyano, trifluoromethyl, nitro, carboxyl, (C₁-C₂₀)-alkyl, (C₃-C₈)-cycloalkyl, (C₃-C₈)cycloalkyl-(C₁-C₁₂)-alkyl, (C₃-C₈)-cycloalkoxy, (C₃-C₈)-cycloalkyl-(C₁-C₁₂)-alkoxy, (C₃-C₈)-cycloalkyloxy-(C₁-C₁₂)-alkyl, (C₃-C₈)-cycloalkyloxy-(C₁-C₁₂)-alkoxy, (C₃-C₈)-cycloalkyl-(C₁-C₈)-alkyl-(C₁-C₆)-alkoxy, (C₃-C₈)-cycloalkyl-(C₁-C₈)-alkoxy-(C₁-C₆)-alkyl, (C₃-C₈)-cycloalkyloxy-(C₁-C₈)-alkoxy-(C₁-C₆)-alkyl, (C₃-C₈)-cycloalkoxy-(C₁-C₈)-alkoxy-(C₁-C₈)-alkoxy, (C₆-C₁₂)-aryl, (C₇-C₁₆)-aralkyl, (C₇-C₁₆)-aralkenyl, (C₇-C₁₆)-aralkynyl, (C₂-C₂₀)-alkenyl, (C₂-C₂₀)-alkynyl, (C₁-C₂₀)-alkoxy, (C₂-C₂₀)-alkenyloxy, (C₂-C₂₀)-alkynyloxy, retinyloxy, (C₁-C₂₀)-alkoxy-(C₁-C₁₂)-alkyl, (C₁-C₁₂)-alkoxy-(C₁-C₁₂)-alkoxy, (C₁-C₁₂)-alkoxy-(C₁-C₈)-alkoxy-(C₁-C₈)-alkyl, (C₆-C₁₂)-aryloxy, (C₇-C₁₆)-aralkyloxy, (C₆-C₁₂)-aryloxy-(C₁-C₆)-alkoxy, (C₇-C₁₆)-aralkoxy-(C₁-C₆)-alkoxy, (C₁-C₁₆)-hydroxyalkyl, (C₆-C₁₆)-aryloxy-(C₁-C₈)-alkyl, (C₇-C₁₆)-aralkoxy-(C₁-C₈)-alkyl, (C₆-C₁₂)-aryloxy-(C₁-C₈)-alkoxy-(C₁-C₆)-alkyl, (C₇-C₁₂)-aralkyloxy-(C₁-C₈)-alkoxy-(C₁-C₆)-alkyl, (C₂-C₂₀)-alkenyloxy-(C₁-C₆)-alkyl, (C₂-C₂₀)-alkynyloxy-(C₁-C₆)-alkyl, retinyloxy-(C₁-C₆)-alkyl, - O-[CH₂]ₓCfH_{(2f+1-g)}F_{g}, -OCF₂Cl, -OCF₂-CHFCl, (C₁-C₂₀)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₂-C₂₀)-alkenylcarbonyl, (C₂-C₂₀)-alkynylcarbonyl, (C₁-C₂₀)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₂₀)-alkenyloxycarbonyl, retinyloxycarbonyl, (C₂-C₂₀)-alkynyloxycarbonyl, (C₆-C₁₂)-aryloxy-(C₁-C₆)-alkoxycarbonyl, (C₇-C₁₆)-aralkoxy-(C₁-C₆)-alkoxycarbonyl, (C₃-C₈)-cycloalkyl-(C₁-C₆)-alkoxycarbonyl, (C₃-C₈)-cycloalkoxy-(C₁-C₆)-alkoxycarbonyl, (C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy, (C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy, carbamoyl, N-(C₁-C₁₂)-alkylcarbamoyl, N,N-di-(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)-carbamoyl, N-(C₁-C₆)-alkyl-N-((C₃-C₈)-cycloalkyl-(C₁-C₆)-alkyl)-carbamoyl, N-(+)-dehydroabietylcarbamoyl, N-(C₁-C₆)-alkyl-N-(+)-dehydroabietylcarbamoyl, N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₈)-alkoxy-(C₁-C₁₀)-alkyl)-carbamoyl, N-((C₆-C₁₆)-aryloxy-(C₁-C₁₀)-alkyl)-carbamoyl, N-((C₇-C₁₆)-aralkyloxy-(C₁-C₁₀)-alkyl)-carbamoyl, N-(C₁-C₁₀)-alkyl-N-((C₁-C₁₀)-alkoxy-(C₁-C₁₀)-alkyl)-carbamoyl, N-(C₁-C₁₀)-alkyl-N-((C₆-C₁₂)-aryloxy-(C₁-C₁₀)-alkyl)-carbamoyl, N-(C₁-C₁₀)-alkyl-N-((C₇-C₁₆)-aralkyloxy-(C₁-C₁₀)-alkyl)-carbamoyl; CON(CH₂)ₕ, in which a CH₂ group can be replaced by O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino, N-(C₁-C₄)-alkoxy-(C₁-C₆)-alkylimino, and h is from 3 to 7; a carbamoyl radical of the formula R 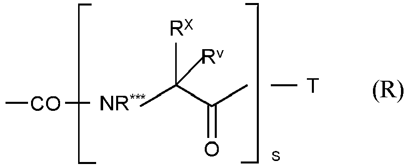 in which
      R^{x} and R^{v} are each independently selected from hydrogen, (C₁-C₆)-alkyl, (C₃-C₇)-cycloalkyl, or aryl,
      s is 1-5,
      T is OH, or NR*R**, and R*, R** and R*** are identical or different and are selected from hydrogen, (C₆-C₁₂)-aryl, (C₇-C₁₁)-aralkyl, (C₁-C₈)-alkyl, (C₃-C₈)-cycloalkyl, (+)-dehydroabietyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkanoyl, optionally substituted (C₇-C₁₆)-aralkanoyl, optionally substituted (C₆-C₁₂)-aroyl; or R* and R** together are -[CH₂]ₕ, in which a CH₂ group can be replaced by O, S, SO, SO₂, N-acylamino, N-(C₁-C₁₀)-alkoxycarbonylimino, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino, N-(C₁-C₄)-alkoxy-(C₁-C₆)-alkylimino, and h is from 3 to 7; carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₂)-arylcarbamoyloxy, N-(C₇-c₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkyl)-carbamoyloxy, N-((C₆-C₁₂)-aryloxy-(C₁-C₁₀)-alkyl)-carbamoyloxy, N-((C₇-C₁₆)-aralkyloxy-(C₁-C₁₀)-alkyl)-carbamoyloxy, N-(C₁-C₁₀)-alkyl-N-((C₁-C₁₀)-alkoxy-(C₁-C₁₀)-alkyl)-carbamoyloxy, N-(C₁-C₁₀)-alkyl-N-((C₆-C₁₂)-aryloxy-(C₁-C₁₀)-alkyl)-carbamoyloxy, N-(C₁-C₁₀)-alkyl-N-((C₇-C₁₆)-aralkyloxy-(C₁-C₁₀)-alkyl)-carbamoyloxyamino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇₋C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino, (C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino, (C₁-C₁₂)-alkanoylamino-(C₁-C₈)-alkyl, (C₃-C₈)-cycloalkanoylamino-(C₁-C₈)-alkyl, (C₆-C₁₂)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di(C₁-C₁₀)-alkylamino-(C₁-C₁₀)-alkyl, (C₃-C₈)-cycloalkylamino(C₁-C₁₀)-alkyl, (C₁-C₂₀)-alkylmercapto, (C₁-C₂₀)-alkylsulfinyl, (C₁-C₂₀)-alkylsulfonyl, (C₆-C₁₂)-arylmercapto, (C₆-C₁₂)-arylsulfinyl, (C₆-C₁₂)-arylsulfonyl, (C₇-C₁₆)-aralkylmercapto, (C₇-C₁₆)-aralkylsulfinyl, (C₇-C₁₆)-aralkylsulfonyl, (C₁-C₁₂)-alkylmercapto-(C₁-C₆)-alkyl, (C₁-C₁₂)-alkylsulfinyl-(C₁-C₆)-alkyl, (C₁-C₁₂)-alkylsulfonyl-(C₁-C₆)-alkyl, (C₆-C₁₂)-arylmercapto-(C₁-C₆)-alkyl, (C₆-C₁₂)-arylsulfinyl-(C₁-C₆)-alkyl, (C₆-C₁₂)-arylsulfonyl-(C₁-C₆)-alkyl, (C₇-C₁₆)-aralkylmercapto-(C₁-C₆)-alkyl, (C₇-C₁₆)-aralkylsulfinyl-(C₁-C₆)-alkyl, (C₇-C₁₆)-aralkylsulfonyl-(C₁-C₆)-alkyl, sulfamoyl, N-(C₁-C₁₀)-alkylsulfamoyl, N,N-di-(C₁-C₁₀)-alkylsulfamoyl, (C₃-C₈)-cycloalkylsulfamoyl, N-(C₆-C₁₂)-arylsulfamoyl, N-(C₇-C₁₆)-aralkylsulfamoyl, N-(C₁-C₁₀)-alkyl-N-(C₆-C₁₂)-arylsulfamoyl, N-(C₁-C₁₀)-alkyl-N-(C₇-C₁₆)-aralkylsulfamoyl, (C₁-C₁₀)-alkylsulfonamido, N-((C₁-C₁₀)-alkyl)-(C₁-C₁₀)-alkylsulfonamido, (C₇-C₁₆)-aralkylsulfonamido, and N-((C₁-C₁₀)-alkyl-(C₇-C₁₆)-aralkylsulfonamido; where an aryl radical may be substituted by 1 to 5 substituents selected from hydroxyl, halogen, cyano, trifluoromethyl, nitro, carboxyl, (C₂-C₁₆)-alkyl, (C₃-C₈)-cycloalkyl, (C₃-C₈)-cycloalkyl-(C₁-C₁₂)-alkyl, (C₃-C₈)-cycloalkoxy, (C₃-C₈)-cycloalkyl-(C₁-C₁₂)-alkoxy, (C₃-C₈)-cycloalkyloxy-(C₁-C₁₂)-alkyl, (C₃-C₈)-cycloalkyloxy-(C₁-C₁₂)-alkoxy, (C₃-C₈)-cycloalkyl-(C₁-C₈)-alkyl-(C₁-C₆)-alkoxy, (C₃-C₈)-cycloalkyl(C₁-C₈)-alkoxy-(C₁-C₆)-alkyl, (C₃-C₈)-cycloalkyloxy-(C₁-C₈)-alkoxy-(C₁-C₆)-alkyl, (C₃-C₈)-cycloalkoxy-(C₁-C₈)-alkoxy-(C₁-C₈)-alkoxy, (C₆-C₁₂)-aryl, (C₇-C₁₆)-aralkyl, (C₂-C₁₆)-alkenyl, (C₂-C₁₂)-alkynyl, (C₁-C₁₆)-alkoxy, (C₁-C₁₆)-alkenyloxy, (C₁-C₁₂)-alkoxy-(C₁-C₁₂)-alkyl, (C₁-C₁₂)-alkoxy-(C₁-C₁₂)-alkoxy, (C₁-C₁₂)-alkoxy(C₁-C₈)-alkoxy-(C₁-C₈)-alkyl, (C₆-C₁₂)-aryloxy, (C₇-C₁₆)-aralkyloxy, (C₆-C₁₂)-aryloxy-(C₁-C₆)-alkoxy, (C₇-C₁₆)-aralkoxy-(C₁-C₆)-alkoxy, (C₁-C₈)-hydroxyalkyl, (C₆-C₁₆)-aryloxy-(C₁-C₈)-alkyl, (C₇-C₁₆)-aralkoxy-(C₁-C₈)-alkyl, (C₆-C₁₂)-aryloxy-(C₁-C₈)-alkoxy-(C₁-C₆)-alkyl, (C₇-C₁₂)-aralkyloxy-(C₁-C₈)-alkoxy-(C₁-C₆)-alkyl, -O-[CH₂]ₓC_{f}H_{(2f+1-g)}F_{g}, -OCF₂Cl, -OCF₂-CHFCl, (C₁-C₁₂)-alkylcarbonyl, (C₃-C₈)-cycloalkylcarbonyl, (C₆-C₁₂)-arylcarbonyl, (C₇-C₁₆)-aralkylcarbonyl, (C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, (C₆-C₁₂)-aryloxy-(C₁-C₆)-alkoxycarbonyl, (C₇-C₁₆)-aralkoxy-(C₁-C₆)-alkoxycarbonyl, (C₃-C₈)-cycloalkyl-(C₁-C₆)-alkoxycarbonyl, (C₃-C₈)-cycloalkoxy-(C₁-C₆)-alkoxycarbonyl, (C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy, (C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy, carbamoyl, N-(C₁-₁₂)-alkylcarbamoyl, N,N-di(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl, N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₆)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₂)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)-carbamoyl, CON(CH₂)ₕ, in which a CH₂ group can be replaced by, O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino, N-(C₁-C₄)-alkoxy-(C₁-C₆)-alkylimino, and h is from 3 to 7; carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₆)-arylcarbamoyloxy, N-(C₇-C₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkyl)carbamoyloxy, N-((C₆-C₁₂)-aryloxy-(C₁-C₁₀)-alkyl)carbamoyloxy, N-((C₇-C₁₆)-aralkyloxy-(C₁-C₁₀)-alkyl)carbamoyloxy, N-(C₁-C₁₀)-alkyl-N-((C₁-C₁₀)-alkoxy-(C₁-C₁₀)-alkyl)carbamoyloxy, N-(C₁-C₁₀)-alkyl-N-((C₆-C₁₂)-aryloxy-(C₁-C₁₀)-alkyl)carbamoyloxy, N-(C₁-C₁₀)-alkyl-N-((C₇-C₁₆)-aralkyloxy-(C₁-C₁₀)-alkyl)carbamoyloxy, amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino, (C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino, (C₁-C₁₂)-alkanoylamino-(C₁-C₈)-alkyl, (C₃-C₈)-cycloalkanoylamino-(C₁-C₈)-alkyl, (C₆-C₁₂)-aroylamino- (C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylamino-(C₁-C₁₀)-alkyl, (C₁-C₁₂)-alkylmercapto, (C₁-₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkylmercapto, (C₇-C₁₆)-aralkylsulfinyl, or (C₇-C₁₆)-aralkylsulfonyl;
or wherein R¹ and R², or R² and R³ form a chain [CH₂]ₒ, which is saturated or unsaturated by a C=C double bond, in which 1 or 2 CH₂ groups are optionally replaced by O, S, SO, SO₂, or NR', and R' is hydrogen, (C₆-C₁₂)-aryl, (C₁-C₈)-alkyl, (C₁-C₈)-alkoxy-(C₁-C₈)-alkyl, (C₇-C₁₂)-aralkoxy-(C₁-C₈)-alkyl, (C₆-C₁₂)-aryloxy-(₁-C₈)-alkyl, (C₁-C₁₀)-alkanoyl, optionally substituted (C₇-C₁₆)-aralkanoyl, or optionally substituted (C6-C12)-aroyl; and ο is 3, 4 or 5;
or wherein the radicals R¹ and R², or R² and R³, together with the pyridine or pyridazine carrying them, form a 5,6,7,8-tetrahydroisoquinoline ring, a 5,6,7,8-tetrahydroquinoline ring, or a 5,6,7,8-tetrahydrocinnoline ring;
or wherein R¹ and R², or R² and R³ form a carbocyclic or heterocyclic 5- or 6-membered aromatic ring;
or where R¹ and R², or R² and R³, together with the pyridine or pyridazine carrying them, form an optionally substituted heterocyclic ring systems selected from thienopyridines, furanopyridines, pyridopyridines, pyrimidinopyridines, imidazopyridines, thiazolopyridines, oxazolopyridines, quinoline, isoquinoline, and cinnoline; where quinoline, isoquinoline or cinnoline preferably satisfy the formulae Ia, Ib and Ic: 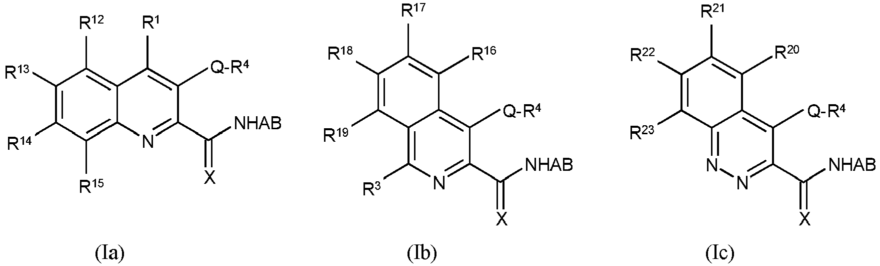
   and the substituents R¹² to R²³ in each case independently of each other have the meaning of R¹, R² and R³;
   or wherein the radicals R¹ and R², together with the pyridine carrying them, form a compound of Formula Id: 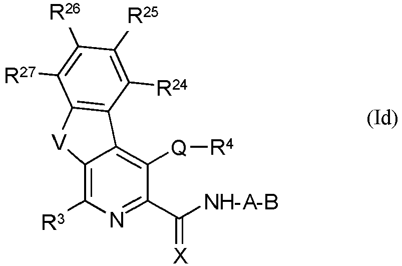
      where V is S, O, or NR^{k}, and R^{k} is selected from hydrogen, (C₁-C₆)-alkyl, aryl, or benzyl; where an aryl radical may be optionally substituted by 1 to 5 substituents as defined above; and
      R²⁴, R²⁵, R²⁶, and R²⁷ in each case independently of each other have the meaning of R¹, R² and R³;
   f is 1 to 8;
   g is 0 or 1 to (2f+1);
   x is 0 to 3; and
   h is 3 to 7;
   including the physiologically active salts derived therefrom.

Exemplary compounds according to Formula (I) are described in European Patent Nos. EP0650960 and EP0650961. All compounds of Formula (I) as defined herein listed in EP0650960 and EP0650961, in particular, those listed in the compound claims and the final products of the working examples, may be used. An exemplary compound contained therein is [(3-methoxy-pyridine-2-carbonyl)-amino]-acetic acid.

Additionally, exemplary compounds according to Formula (I) are described in U.S. Patent No. 5,658,933. All compounds of Formula (I) as defined herein listed in U.S. Patent No. 5,658,933, in particular, those listed in the compound claims and the final products of the working examples, may be used. Exemplary compounds of Formula (I) include, but are not limited to, 3-methoxypyridine-2-carboxylic acid N-(((hexadecyloxy)-carbonyl)-methyl)-amide hydrochloride, 3-methoxypyridine-2-carboxylic acid N-(((1-octyloxy)-carbonyl)-methyl)-amide, 3-methoxypyridine-2-carboxylic acid N-(((hexyloxy)-carbonyl)-methyl)-amide, 3-methoxypyridine-2-carboxylic acid N-(((butyloxy)-carbonyl)-methyl)-amide, 3-methoxypyridine-2-carboxylic acid N-(((2-nonyloxy)-carbonyl)-methyl)-amide racemate, 3-methoxypyridine-2-carboxylic acid N-(((heptyloxy)-carbonyl)-methyl)-amide, 3-benzyloxypyridine-2-carboxylic acid N-(((octyloxy)-carbonyl)-methyl)-amide, 3-benzyloxypyridine-2-carboxylic acid N-(((butyloxy)-carbonyl)-methyl)-amide, 5-(((3-(1-butyloxy)-propyl)-amino)-carbonyl)-3-methoxypyridine-2-carboxylic acid N-((benzyloxycarbonyl)-methyl)-amide, 5-(((3-(1-butyloxy)-propyl)-amino)-carbonyl)-3-methoxypyridine-2-carboxylic acid N-(((1-butyloxy)-carbonyl)-methyl)-amide, and 5-(((3-lauryloxy)-propyl)amino)-carbonyl)-3-methoxypyridine-2-carboxylic acid N-(((benzyloxy)-carbonyl)-methyl)-amide.

Additional compounds acording to Formula (I) are substituted heterocyclic carboxyamides described in U.S. Patent No. 5,620,995; 3-hydroxypyridine-2-carboxamidoesters described in U.S. Patent No. 6,020,350; sulfonamidocarbonylpyridine-2-carboxamides described in U.S. Patent No. 5,607,954; and sulfonamidocarbonyl-pyridine-2-carboxamides and sulfonamidocarbonyl-pyridine-2-carboxesteramides described in U.S. Patent Nos. 5,610,172 and 5,620,996. All compounds of Formula (I) as defined herein listed in these patents, in particular, those compounds listed in the compound claims and the final products of the working examples, may be used. Exemplary compounds of Formula (I) include, but are not limited to, 3-methoxypyridine-2-carboxylic acid N-(((hexadecyloxy)-carbonyl)-methyl)-amide hydrochloride, 3-methoxypyridine-2-carboxylic acid N-(((1-octyloxy)-carbonyl)-methyl)-amide, 3-methoxypyridine-2-carboxylic acid N-(((hexyloxy)-carbonyl)-methyl)-amide, 3-methoxypyridine-2-carboxylic acid N-(((butyloxy)-carbonyl)-methyl)-amide, 3-methoxypyridine-2-carboxylic acid N-(((2-nonyloxy)-carbonyl)-methyl)-amide racemate, 3-methoxypyridine-2-carboxylic acid N-(((heptyloxy)-carbonyl)-methyl)-amide, 3-benzyloxypyridine-2-carboxylic acid N-(((octyloxy)-carbonyl)-methyl)-amide, 3-benzyloxypyridine-2-carboxylic acid N-(((butyloxy)-carbonyl)-methyl)-amide, 5-(((3-(1-butyloxy)-propyl)-amino)-carbonyl)-3-methoxypyridine-2-carboxylic acid N-((benzyloxycarbonyl)-methyl)-amide, 5-(((3-(1-butyloxy)-propyl)-amino)-carbonyl)-3-methoxypyridine-2-carboxylic acid N-(((1-butyloxy)-carbonyl)-methyl)-amide, and 5-(((3-lauryloxy)-propyl)amino)-carbonyl)-3-methoxypyridine-2-carboxylic acid N-(((benzyloxy)-carbonyl)-methyl)-amide.

Additionally, exemplary compounds of Formula (I) include, but are not limited to, 3-hydroxypyridine-2-carboxylic acid N-(((hexadecyloxy)-carbonyl)-methyl)-amide hydrochloride, 3-hydroxypyridine-2-carboxylic acid N-(((1-octyloxy)-carbonyl)-methyl)-amide, 3-hydroxypyridine-2-carboxylic acid N-(((hexyloxy)-carbonyl)-methyl)-amide, 3-hydroxypyridine-2-carboxylic acid N-(((butyloxy)-carbonyl)-methyl)-amide, 3-hydroxypyridine-2-carboxylic acid N-(((2-nonyloxy)-carbonyl)-methyl)-amide racemate, 3-hydroxypyridine-2-carboxylic acid N-(((heptyloxy)-carbonyl)-methyl)-amide, 3-benzyloxypyridine-2-carboxylic acid N-(((octyloxy)-carbonyl)-methyl)-amide, 3-benzyloxypyridine-2-carboxylic acid N-(((butyloxy)-carbonyl)-methyl)-amide, 5-(((3-(1-butyloxy)-propyl)-amino)-carbonyl)-3-hydroxypyridine-2-carboxylic acid N-((benzyloxycarbonyl)-methyl)-amide, 5-(((3-(1-butyloxy)-propyl)-amino)-carbonyl)-3-hydroxypyridine-2-carboxylic acid N-(((1-butyloxy)-carbonyl)-methyl)-amide, and 5-(((3-lauryloxy)-propyl)amino)-carbonyl)-3-hydroxypyridine-2-carboxylic acid N-(((benzyloxy)-carbonyl)-methyl)-amide.

Exemplary compounds according to Formula (Ia) are described in U.S. Patent Nos. 5,719,164 and 5,726,305. All compounds of Formula (I) as defined herein listed in the foregoing patents, in particular, those listed in the compound claims and the final products of the working examples, may be used. Exemplary compounds of Formula (Ia) include, but are not limited to, N-((6-(1-butyloxy)-3-hydroxyquinolin-2-yl)-carbonyl)-glycine, N-((6-chloro-3-hydroxyquinolin-2-yl)-carbonyl)-glycine, N-((3-hydroxy-6-(2-propyloxy)-quinolin-2-yl)-carbonyl)-glycine, N-((7-chloro-3-hydroxy-quinoline-2-carbonyl)-amino]-acetic acid, [(3-benzyloxy-7-chloro-quinoline-2-carbonyl)-amino]-acetic acid (Compound D), [(3-hydroxy-6-isopropoxy-quinoline-2-carbonyl)-amino]-acetic acid (Compound E), [(3-hydroxy-6-phenoxy-quinoline-2-carbonyl)-amino]-acetic acid (Compound F), and [(3-hydroxy-6-trifluoromethoxy-quinoline-2-carbonyl)-amino]-acetic acid (Compound G).

Exemplary compounds according to Formula (Ib) are described in U.S. Patent No. 6,093,730. All compounds of Formula (I) as defined herein listed in U.S. Patent No. 6,093,730, in particular, those listed in the compound claims and the final products of the working examples, may be used. Exemplary compounds of Formula (Ib) include, but are not limited to, N-((1-chloro-4-hydroxy-7-(2-propyloxy) isoquinolin-3-yl)-carbonyl)-glycine, N-((7-bromo-4-hydroxy-isoquinoline-3-carbonyl)-amino)-acetic acid, N-((1-chloro-4-hydroxy-6-(2-propyloxy) isoquinolin-3-yl)-carbonyl)-glycine, N-((1-chloro-4-hydroxy-7-methoxyisoquinolin-3-yl)-carbonyl)-glycine (Compound J), N-((1-chloro-4-hydroxy-6-methoxyisoquinolin-3-yl)-carbonyl)-glycine, [(7-butoxy-1-chloro-4-hydroxy-isoquinoline-3-carbonyl)-amino]-acetic acid, N-((7-benzyloxy-1-chloro-4-hydroxyisoquinolin -3-yl)-carbonyl)-glycine, N-((6-benzyloxy-1-chloro-4-hydroxyisoquinolin-3-yl)-carbonyl)-glycine, [(1-chloro-4-hydroxy-isoquinoline-3-carbonyl)-amino]-acetic acid (Compound C), N-((8-chloro-4-hydroxyisoquinolin-3-yl)-carbonyl)-glycine, [(4-Hydroxy-7-isopropoxy-isoquinoline-3-carbonyl)-amino]-acetic acid (Compound H), [(7-butoxy-4-hydroxy-isoquinoline-3-carbonyl)-amino]-acetic acid (Compound I), and [(1-Chloro-4-hydroxy-7-isopropoxy-isoquinoline-3-carbonyl)-amino]-acetic acid (Compound K).

Additionally, compounds for use in the invention are compounds described by Majamaa et al. (1984, Eur J Biochem 138:239-245; and 1985, Biochem J 229:127-133), Kivirikko and Myllyharju (1998, Matrix Biol 16:357-368), Bickel et al. (1998, Hepatology 28:404-411), Friedman et al. (2000, Proc Natl Acad Sci USA 97:4736-4741), and Franklin et al. (2001, Biochem J 353:333-338), as far as these are covered by the appended claims. Further, the disclosure provides additional exemplary compounds wherein, e.g., position A and B together may be, e.g., hexanoic acid, cyanomethyl, 2-aminoethyl, benzoic acid, 1H-benzoimidazol-2-ylmethyl.

The invention provides compounds for use in treating or preventing erythropoietin-associated conditions that are anemic conditions associated with kidney disease. Further, the invention provides compounds for treating a patient having anemia associated with particular courses of treatment such as, e.g., chemotherapy, dialysis.

### Methods of Using the Compounds of the Invention

The present disclosure provides compounds that are capable of increasing endogenous erythropoietin, thereby increasing erythropoiesis. The compounds can be used to prevent, or treat anemia associated with kidney disease.

For example, a therapeutically effective amount of the compound or a pharmaceutically acceptable salt thereof, alone or in combination with a pharmaceutically acceptable excipient, can be administered to a patient having or at risk of developing anemia associated with kidney disease. The anemia can be due to a condition or disorder including, acute or chronic kidney disease, diabetes, cancer, ulcers; infection with virus, bacteria, or parasites; inflammation; or can be associated with a medical procedure or treatment including, e.g., radiation therapy, chemotherapy, kidney dialysis, surgery.

Preferred routes of administration include oral and transdermal delivery mechanisms. Such mechanisms provide benefit over current EPO replacement therapies by allowing for, e.g., ease of administration, self-administration by patient, reduced cost, fewer physician visits, and reduced risks due to infection and immunogenic complications, minimizing the adverse reactions some patients develop in response to dosing with recombinant EPO.

A compound of the invention that inhibits HIF prolyl hydroxylase enzyme activity may also inhibit one or more 2-oxoglutarate dioxygenase enzymes. In one embodiment, the compound inhibits at least two 2-oxoglutarate dioxygenase family members, e.g., HIF prolyl hydroxylase and HIF asparagine-hydroxylase (FIH-1), with either the same specificity or with differential specificity. In another embodiment, the compound is specific for one 2-oxoglutarate dioxygenase, e.g., HIF prolyl hydroxylase, and shows little to no specificity for other family members.

The compounds of the invention can be administered in combination with various other therapeutic approaches. In one embodiment, the compound is administered with another 2-oxoglutarate dioxygenase inhibitor, wherein the two compounds have differential specificity for individual 2-oxoglutarate dioxygenase family members. The two compounds may be administered at the same time as a ratio of one relative to the other. Determination of a ratio appropriate to a given course of treatment or a particular subject is within the level of skill in the art. Alternatively, the two compounds may be administered consecutively during a treatment time course, e.g., following myocardial infarction. In a particular embodiment, one compound specifically inhibits HIF prolyl hydroxylase enzyme activity, and a second compound specifically inhibits procollagen prolyl 4-hydroxylase enzyme activity. In another specific embodiment, one compound specifically inhibits HIF prolyl hydroxylase enzyme activity, and a second compound specifically inhibits HIF asparaginyl-hydroxylase enzyme activity. Additionally, the compound can be administered with another agent such as an iron supplement, e.g., ferrous sulfate, vitamin B₁₂, and/or folic acid. The compound can also be administered in conjunction with exogenous erythropoietin, e.g., EPOGEN^{®} or ARANESP^{®} recombinant human erythropoietin (Amgen, Inc., Thousand Oaks CA), and/or granulocyte-colony stimulating factor (G-CSF), e.g., NEUPOGEN^{®} or NEULASTA^{®} recombinant G-CSF (Amgen).

### Pharmaceutical Formulations And Routes Of Administration

The compounds of the present invention can be delivered directly or in pharmaceutical compositions along with suitable carriers or excipients, as is well known in the art. Compounds of the present invention can be for use in methods of treatment comprising administration of an effective amount of a compound of the invention to a subject having or at risk for anemia due to, e.g., chronic renal failure, diabetes, cancer, AIDS, radiation therapy, chemotherapy, kidney dialysis, or surgery. In a preferred embodiment, the subject is a mammalian subject, and in a most preferred embodiment, the subject is a human subject.

An effective amount of such agents can readily be determined by routine experimentation, as can the most effective and convenient route of administration and the most appropriate formulation. Various formulations and drug delivery systems are available in the art. (See, e.g., Gennaro, A.R., ed. (1995) Remington's Pharmaceutical Sciences, *supra*.)

Suitable routes of administration may, for example, include oral, rectal, transmucosal, nasal, or intestinal administration and parenteral delivery, including intramuscular, subcutaneous, intramedullary injections, as well as intrathecal, direct intraventricular, intravenous, intraperitoneal, intranasal, or intraocular injections. The agent or composition thereof may be administered in a local rather than a systemic manner. For example, a suitable agent can be delivered via injection or in a targeted drug delivery system, such as a depot or sustained release formulation.

The pharmaceutical compositions of the present invention may be manufactured by any of the methods well-known in the art, such as by conventional mixing, dissolving, granulating, dragee-making, levigating, emulsifying, encapsulating, entrapping, or lyophilizing processes. As noted above, the compositions of the present invention can include one or more physiologically acceptable carriers such as excipients and auxiliaries that facilitate processing of active molecules into preparations for pharmaceutical use.

Proper formulation is dependent upon the route of administration chosen. For injection, for example, the composition may be formulated in aqueous solutions, preferably in physiologically compatible buffers such as Hanks' solution, Ringer's solution, or physiological saline buffer. For transmucosal or nasal administration, penetrants appropriate to the barrier to be permeated are used in the formulation. Such penetrants are generally known in the art. In a preferred embodiment of the present invention, the present compounds are prepared in a formulation intended for oral administration. For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions, for oral ingestion by a subject. The compounds may also be formulated in rectal compositions such as suppositories or retention enemas, e.g., containing conventional suppository bases such as cocoa butter or other glycerides.

Pharmaceutical preparations for oral use can be obtained as solid excipients, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Also, wetting agents such as sodium dodecyl sulfate may be included.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations for oral administration include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

In one embodiment, the compounds of the present invention can be administered transdermally, such as through a skin patch, or topically. In one aspect, the transdermal or topical formulations of the present invention can additionally comprise one or multiple penetration enhancers or other effectors, including agents that enhance migration of the delivered compound. Transdermal or topical administration could be preferred, for example, in situations in which location specific delivery is desired.

For administration by inhalation, the compounds for use according to the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide, or any other suitable gas. In the case of a pressurized aerosol, the appropriate dosage unit may be determined by providing a valve to deliver a metered amount. Capsules and cartridges of, for example, gelatin, for use in an inhaler or insufflator may be formulated. These typically contain a powder mix of the compound and a suitable powder base such as lactose or starch.

Compositions formulated for parenteral administration by injection, e.g., by bolus injection or continuous infusion can be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Formulations for parenteral administration include aqueous solutions or other compositions in water-soluble form.

Suspensions of the active compounds may also be prepared as appropriate oily injection suspensions. Suitable lipophilic solvents or vehicles include fatty oils such as sesame oil and synthetic fatty acid esters, such as ethyl oleate or triglycerides, or liposomes. Aqueous injection suspensions may contain substances that increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions. Alternatively, the active ingredient may be in powder form for constitution with a suitable vehicle, e.g., sterile pyrogen-free water, before use.

As mentioned above, the compositions of the present invention may also be formulated as a depot preparation. Such long acting formulations may be administered by implantation (for example, subcutaneously or intramuscularly) or by intramuscular injection. Thus, for example, the present compounds may be formulated with suitable polymeric or hydrophobic materials (for example as an emulsion in an acceptable oil) or ion exchange resins, or as sparingly soluble derivatives, for example, as a sparingly soluble salt.

Suitable carriers for the hydrophobic molecules of the invention are well known in the art and include co-solvent systems comprising, for example, benzyl alcohol, a nonpolar surfactant, a water-miscible organic polymer, and an aqueous phase. The co-solvent system may be the VPD co-solvent system. VPD is a solution of 3% w/v benzyl alcohol, 8% w/v of the nonpolar surfactant polysorbate 80, and 65% w/v polyethylene glycol 300, made up to volume in absolute ethanol. The VPD co-solvent system (VPD:5W) consists of VPD diluted 1:1 with a 5% dextrose in water solution. This co-solvent system is effective in dissolving hydrophobic compounds and produces low toxicity upon systemic administration. Naturally, the proportions of a co-solvent system may be varied considerably without destroying its solubility and toxicity characteristics. Furthermore, the identity of the co-solvent components may be varied. For example, other low-toxicity nonpolar surfactants may be used instead of polysorbate 80, the fraction size of polyethylene glycol may be varied, other biocompatible polymers may replace polyethylene glycol, e.g., polyvinyl pyrrolidone, and other sugars or polysaccharides may substitute for dextrose.

Alternatively, other delivery systems for hydrophobic molecules may be employed. Liposomes and emulsions are well known examples of delivery vehicles or carriers for hydrophobic drugs. Liposomal delivery systems are discussed above in the context of gene-delivery systems. Certain organic solvents such as dimethylsulfoxide also may be employed, although usually at the cost of greater toxicity. Additionally, the compounds may be delivered using sustained-release systems, such as semi-permeable matrices of solid hydrophobic polymers containing the effective amount of the composition to be administered. Various sustained-release materials are established and available to those of skill in the art. Sustained-release capsules may, depending on their chemical nature, release the compounds for a few weeks up to over 100 days. Depending on the chemical nature and the biological stability of the therapeutic reagent, additional strategies for protein stabilization may be employed.

For any present composition, a therapeutically effective dose can be estimated initially using a variety of techniques well known in the art. For example, in a cell culture assay, a dose can be formulated in animal models to achieve a circulating concentration range that includes the IC₅₀ as determined in cell culture. Dosage ranges appropriate for human subjects can be determined, for example, using data obtained from cell culture assays and other animal studies.

A therapeutically effective dose of an agent refers to that amount of the agent that results in amelioration of symptoms or a prolongation of survival in a subject. Toxicity and therapeutic efficacy of such molecules can be determined by standard pharmaceutical procedures in cell cultures or experimental animals, e.g., by determining the LD₅₀ (the dose lethal to 50% of the population) and the ED₅₀ (the dose therapeutically effective in 50% of the population). The dose ratio of toxic to therapeutic effects is the therapeutic index, which can be expressed as the ratio LD₅₀/ED₅₀. Agents that exhibit high therapeutic indices are preferred.

Dosages preferably fall within a range of circulating concentrations that includes the ED₅₀ with little or no toxicity. Dosages may vary within this range depending upon the dosage form employed and the route of administration utilized. The exact formulation, route of administration, and dosage should be chosen, according to methods known in the art, in view of the specifics of a subject's condition.

Dosage amount and interval may be adjusted individually to provide plasma levels of the active moiety that are sufficient to modulate endogenous erythropoietin plasma levels as desired, i.e. minimal effective concentration (MEC). The MEC will vary for each compound but can be estimated from, for example, *in vitro* data. Dosages necessary to achieve the MEC will depend on individual characteristics and route of administration. Agents or compositions thereof should be administered using a regimen which maintains plasma levels above the MEC for about 10-90% of the duration of treatment, preferably about 30-90% of the duration of treatment, and most preferably between 50-90%. In cases of local administration or selective uptake, the effective local concentration of the drug may not be related to plasma concentration. Alternatively, stimulation of endogenous erythropoietin may be achieved by 1) administering a loading dose followed by a maintenance dose, 2) administering an induction dose to rapidly achieve erythropoietin levels within a target range, followed by a lower maintenance dose to maintain hematocrit within a desired target range, or 3) repeated intermittent dosing.

The amount of agent or composition administered will, of course, be dependent on a variety of factors, including the sex, age, and weight of the subject being treated, the severity of the affliction, the manner of administration, and the judgment of the prescribing physician.

The present compositions may, if desired, be presented in a pack or dispenser device containing one or more unit dosage forms containing the active ingredient. Such a pack or device may, for example, comprise metal or plastic foil, such as a blister pack. The pack or dispenser device may be accompanied by instructions for administration. Compositions comprising a compound of the invention formulated in a compatible pharmaceutical carrier may also be prepared, placed in an appropriate container, and labeled for treatment of an indicated condition. Suitable conditions indicated on the label may include treatment of conditions, disorders, or diseases in which anemia is a major indication.

### Compound Screening and Identification

Disclosed herein are methods of screening for and identifying additional compounds that increase endogenous erythropoietin. In a particular embodiment, methods of identifying compounds that increase endogenous EPO plasma levels are disclosed. Various assays and screening techniques, including those described below, can be used to identify small molecules that increase the level of endogenous erythropoietin. One assay that is particularly useful involves treating animals with a compound of interest and measuring erythropoietin levels in plasma. (See, e.g., Example 2.) Assays will typically provide for detectable signals associated with the consumption of a reaction substrate or production of a reaction product. Detection can involve, for example, fluorophores, radioactive isotopes, enzyme conjugates, and other detectable labels well known in the art. The results may be qualitative or quantitative. Isolation of the reaction product may be facilitated by a label such as biotin or a histidine tag that allows purification from other reaction components via precipitation or affinity chromatography.

Increasing endogenous erythropoietin expression may involve stabilization of HIFα within cells, and assays used to identify small molecules that modulate (e.g., increase or decrease) the level or activity of HIFα are contemplated herein. Assays for HIFα hydroxylation may involve measuring hydroxylated proline or asparagine residues in HIFα or a fragment thereof, or measuring formation of succinate from 2-oxoglutarate in the presence of enzyme and HIFα or a fragment thereof. (See, e.g., Palmerini et al. (1985) J Chromatogr 339:285-292; Cunliffe et al. (1986) Biochem J 240:617-619.) Exemplary procedures that measure HIFα hydroxylation are described in, e.g., Ivan et al. (*supra*) and Example 9. An exemplary procedure that measures production of succinate from 2-oxoglutarate is described by Kaule and Gunzler. (1990, Anal Biochem 184:291-297.) Substrate molecules may include HLFα or a fragment thereof, e.g., HIF(556-575); for example, an exemplary substrate for use in the assay described in Example 7 is [methoxycoumarin]-DLDLEALAPYIPADDDFQL-amide (SEQ ID NO:5). Enzyme may include, e.g., HIFα prolyl hydroxylase (see, e.g., GenBank Accession No. AAG33965.), obtained from any source. Enzyme may also be present in a crude cell lysate or in a partially purified form. Measuring and comparing enzyme activity in the absence and presence of the compound identifies compounds that inhibit hydroxylation of HIFα.

Additionally, and in combination with the above methods, compounds can be identified by any of a variety of screening techniques known in the art. Such screening methods may allow for target polypeptides or the compounds to be free in solution, affixed to a solid support, borne on a cell surface, located within a cell. For example, test compounds may be arrayed on a surface and analyzed for activity in a manner analogous to array methods currently available in the art. (See, e.g., Shalon et al. (1995) International Publication No. WO 95/35505; Baldeschweiler et al. (1995) International Publication No. WO 95/251116; Brennan et al. (1995) U.S. Patent No. 5,474,796; and Heller et al. (1997) U.S. Patent No. 5,605,662.)

### EXAMPLES

The invention is further understood by reference to the following examples, which are intended to be purely exemplary of the invention. The present invention is not limited in scope by the exemplified embodiments, which are intended as illustrations of single aspects of the invention only. Various modifications of the invention in addition to those described herein will become apparent to those skilled in the art from the foregoing description and accompanying figures. Such modifications fall within the scope of the appended claims.

### Example 1: Increase in Endogenous Erythropoietin Levels in vitro.

Human cells derived from hepatocarcinoma (Hep3B) tissue (see, e.g., American Type Culture Collection, Manassas VA) were seeded into 35 mm culture dishes and grown at 37°C, 20% O₂, 5% CO₂ in Minimal Essential Medium (MEM), Earle's balanced salt solution (Mediatech Inc., Herndon VA), 2 mM L-glutamine, 0.1 mM non-essential amino acids, 1 mM sodium pyruvate, and 10% FBS. When cell layers reached confluence, the media was replaced with OPTI-MEM media (Invitrogen Life Technologies, Carlsbad CA) and cell layers were incubated for approximately 24 hours in 20% O₂, 5% CO₂ at 37°C. A compound of the invention (one of compounds A to I) or 1% DMSO (negative control) was then added to existing media and incubation was continued overnight.

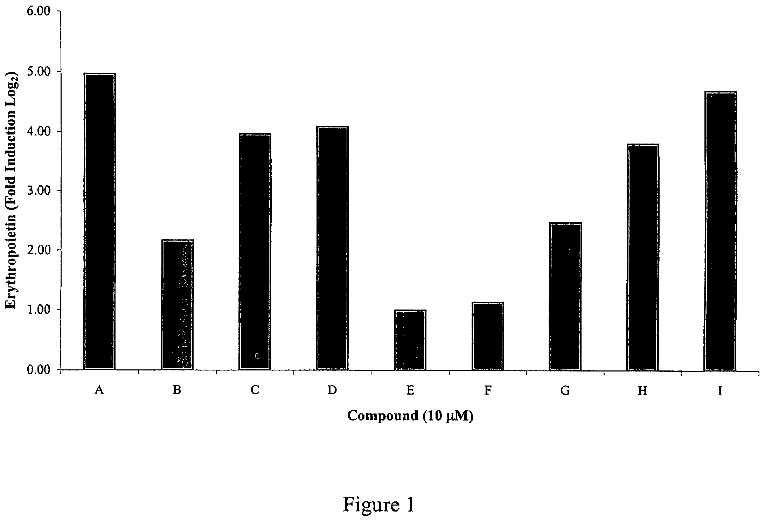

Following incubation, the conditioned media was collected from cell cultures and analyzed for erythropoietin expression using a QUANTIKINE immunoassay (R&D Systems, Inc., Minneapolis MN) according to the manufacturer's instructions. As seen in Figure 1, liver-derived cells (Hep3B) showed significant increase in expression of erythropoietin when treated with compounds of the invention. Thus, compounds of the invention increase erythropoietin expression *in vitro* in cells derived from tissues that normally produce erythropoietin in animals.

### Example 2: Increase in Endogenous Erythropoietin Levels in vivo.

### Experiment I

Twelve Swiss Webster male mice (30-32 g) were obtained from Simonsen, Inc. (Gilroy CA), and treated by oral gavage two times per day for 2.5 days (5 doses) with a 4 ml/kg volume of either 0.5% carboxymethyl cellulose (CMC; Sigma-Aldrich, St. Louis MO) (0 mg/kg/day) or 2.5% Compound C (25 mg/ml in 0.5% CMC) (200 mg/kg/day). Four hours after the final dose, animals were anesthetized with isoflurane and two blood samples were collected from the abdominal vein. One blood sample was collected into a MICROTAINER serum separator tube (Becton-Dickinson, Franklin Lakes NJ), and incubated at room temperature for 30 minutes, centrifuged at 8,000 rpm at 4°C for 10 minutes. The serum fraction was then processed and analyzed for erythropoietin (EPO) expression using a QUANTIKINE immunoassay (R&D Systems) according to the manufacturer's instructions. The second blood sample was collected into a MICROTAINER EDTA-2K tube (Becton-Dickinson) for hematocrit analysis. Hematocrit was measured by drawing EDTA-blood into a 75 mm x 1.1-1.2 mm I.D. capillary tube (Chase Scientific Glass, Inc., Rockwood TN) to approximately ¾ length. One end of the tube was sealed with CRITOSEAL sealant (Sherwood Medical Company) and the tube was centrifuged in a J-503M MICROHEMATOCRIT centrifuge (Jorgensen Laboratories, Inc., Loveland CO) at 12,000 rpm for 5 minutes. Hematocrit was read against a reader card. The mice were then sacrificed and approximately 150 mg of liver and each kidney were isolated and stored in RNALATER solution (Ambion) at -20°C.
RNA isolation was carried out using the following protocol. Tissue slices were cut into small pieces, 1.75 ml of RLT lysis buffer (RNEASY kit; Qiagen Inc., Valencia CA) was added, and the pieces were homogenized for about 20 seconds using a rotor-stator POLYTRON homogenizer (Kinematica, Inc., Cincinnati OH). A 350 µl volume of homogenate was micro-centrifuged for 3 minutes to pellet insoluble material, the supernatant was transferred to a new tube and RNA was isolated using an RNEASY kit (Qiagen) according to the manufacturer's instructions. The RNA was eluted into 80 µL of water and quantitated with RIBOGREEN reagent (Molecular Probes, Eugene OR). Genomic DNA was then removed from the RNA using a DNA-FREE kit (Ambion Inc., Austin TX) according to the manufacturer's instructions. The absorbance at 260 and 280 nm was measured to determine RNA purity and concentration.

cDNA synthesis was performed using 1 µM random hexamer primers, 1 µg of total RNA, and OMNISCRIPT reverse transcriptase (Qiagen), according to the manufacturer's instructions. Resulting cDNA was diluted 5-fold with water to give 100 µL final volume. Analysis of the relative level of erythropoietin gene expression was performed by quantitative PCR using a FASTSTART DNA MASTER SYBR GREEN I kit (Roche) and gene-specific primers, using a LIGHTCYCLER system (Roche), according to manufacturer's instructions. Samples were heated to 94°C for 6 minutes and then cycled through 95°C for 15 seconds, 60°C for 5 seconds, and 72°C for 10 seconds for a total of 42 cycles. Erythropoietin-specific primers were as follows:

| | | |
|---|---|---|
| mEPO-R3 | TTCTGGCCCCGAGGATGTCA | (SEQ ID NO:1) |
| mEPO-F3 | ACGAACTTGCTCCCCGTCACTG | (SEQ ID NO:2) |

The relative level of 18S ribosomal RNA gene expression was measured as a control. Quantitative PCR was performed using a QUANTITECT SYBR GREEN PCR kit (Qiagen) and gene-specific primers, using a LIGHTCYCLER system (Roche), according to manufacturer's instructions. Samples were heated to 95°C for 15 minutes and then cycled through 94°C for 15 seconds, 60°C for 20 seconds, 72°C for 10 seconds for a total of 42 cycles. Ribosomal RNA-specific primers were as follows:

| | | |
|---|---|---|
| 18S-rat-2B | TAGGCACGGCGACTACCATCGA | (SEQ ID NO:3) |
| 18S-rat-2A | CGGCGGCTTTGGTGACTCTAGAT | (SEQ ID NO:4) |

Each PCR run included a standard curve and water blank. In addition, a melt curve was run after completion of each PCR run to assess the specificity of the amplification. Erythropoietin gene expression was normalized relative to the expression level of 18S ribosomal RNA for that sample.

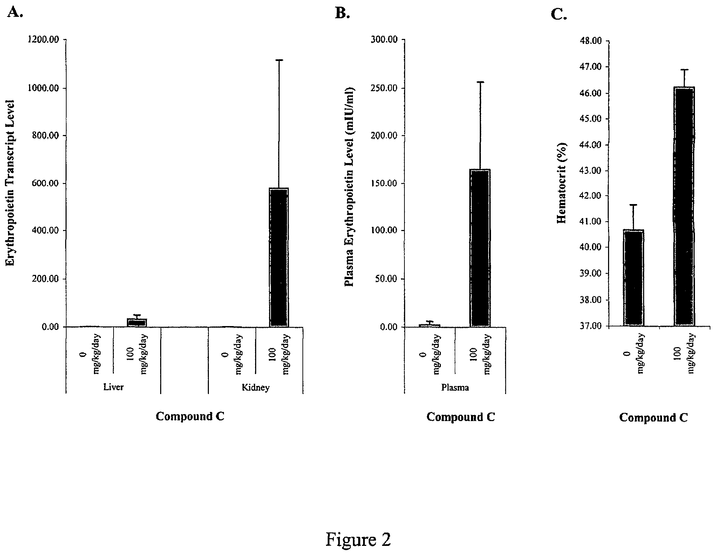

As seen in Figure 2A, erythropoietin gene expression was induced in both the kidney and liver in treated animals relative to untreated controls. The liver showed an approximately 32-fold increase and the kidney showed an approximately 580-fold increase over background in EPO transcript level relative to the untreated liver and kidney, respectively (y-axis units are arbitrary). As seen in Figure 2B, the same animals showed a significant increase in erythropoietin plasma level in the treated group relative to untreated controls. Further, as seen in Figure 2C, the increase in endogenous erythropoietin induced by the compound of the invention significantly increased hematocrit in the treated animals relative to untreated controls.

### Experiment II

Male Swiss Webster mice (29-34 g) were obtained from Simonson, Inc., and were treated by oral gavage once per day for 2.5 days (5 doses) with a 4 ml/kg volume of either 0.5% carboxymethyl cellulose (CMC; Sigma-Aldrich, St. Louis MO) (0 mg/kg/day) or one of compounds E or K at 100 mg/kg/day for 3 days. Blood samples were collected and tissues were processed as for Experiment I (*supra*). Alternatively, mice were treated with 0.5% CMC or one of compounds F or J at 60 mg/kg/day for 5 days. Forty-eight hours after the final treatment, blood samples were collected, mice were sacrificed, and tissues were harvested as described above.

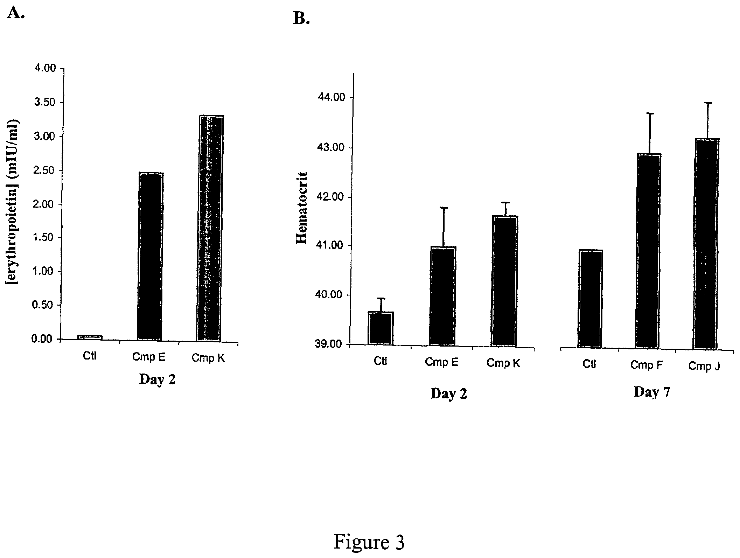

As seen in Figure 3A, plasma erythropoietin levels were increased over controls after 2 days of treatment with compounds of the invention. Also, as seen in Figure 3B, hematocrit was measurably higher after 2 and 7 days in animals treated with compound relative to untreated controls.

### Example 3: Dose Response in vivo.

Twelve Sprague Dawley male rats (approx. 260 g) were obtained from Charles River Laboratories, Inc., and treated by oral gavage as follows: (1) Four rats were dosed daily for days 1 to 7 with 0.5% carboxymethyl cellulose (CMC; Sigma-Aldrich, St. Louis MO) (0 mg/kg), were not treated for days 8 to 14, and then were dosed daily for days 15 to 19 with 0.5% CMC (0 mg/kg); (2) four animals were treated on day 0 and day 3 with a total of 7 ml/kg/day of 5.0% compound C (50 mg/ml in 0.5% CMC) (350 mg/kg), were not treated days 8 to 14, and then were dosed daily for days 15 to 19 with 3% compound C (30 mg/ml in 0.5% CMC) (60 mg/kg/day); and (3) four animals were treated daily for days 1 to 7 with a total volume of 4 ml/kg/day of 2.5% compound C (25 mg/ml in 0.5% CMC) (100 mg/kg), were not treated days 8 to 14, and then were dosed daily for days 15 to 19 with 3% compound C (60 mg/kg/day). Thus, groups (2) and (3) received a total of 700 mg compound/kg body weight over the first 7 days, no treatment from days 8 to 14, and then 60 mg/kg/day for another 5 days. Animals were monitored for change in body weight and signs of toxicity. Blood samples (2 x 0.5 ml) were collected on days 1, 3, 7, 10, 17 and 21 as follows. Animals were anesthetized with isoflurane and 0.5 ml of blood was collected from the tail vein of each animal into each of two MICROTAINER EDTA-2K tubes (Becton-Dickinson). Blood samples were processed for erythropoietin levels, hemoglobin, and hematocrit as described above.

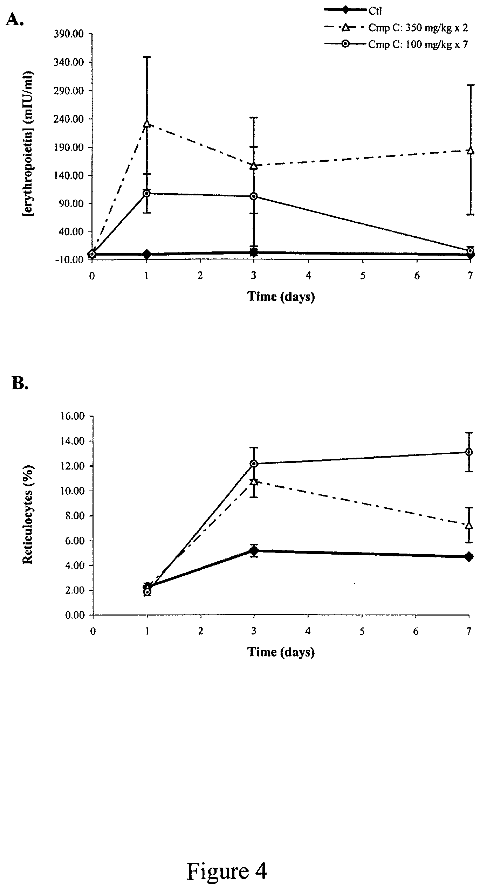

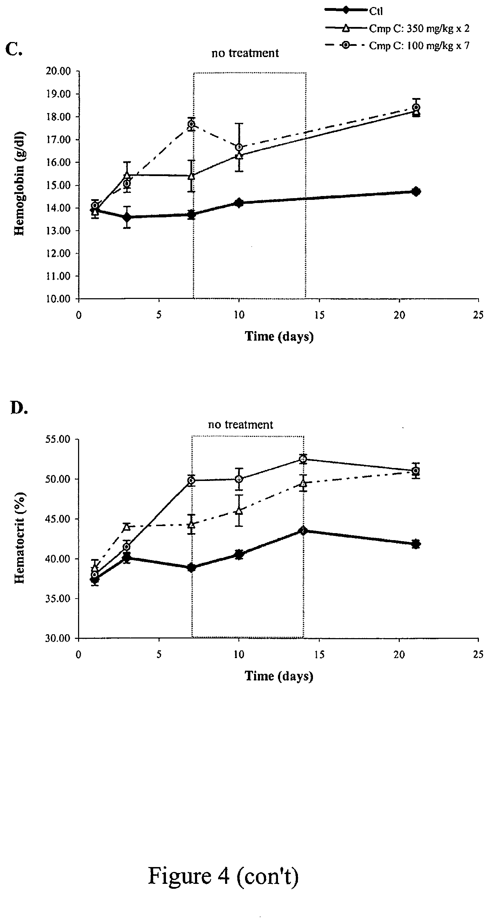

As can be seen in Figure 4A, the compound significantly increased serum erythropoietin level within 1 day of treatment. As seen in Figure 4B, the increase in serum EPO led to a subsequent increase in immature blood cells (reticulocytes), demonstrating that the compound stimulates new red blood cell formation. Additionally, as seen in Figures 4C and 4D, the compound increased blood hemoglobin levels and hematocrit, respectively. Further, the increase in hemoglobin and hematocrit was maintained over an extended period of time with lower doses of the compound. These results demonstrate that a compound of the invention can produce a controlled increase in red blood cells and the animals remain responsive to the compound over an extended period of time.

### Example 4: Treatment of Anemia Induced by Cisplatin.

The ability of a compound of the invention to treat anemia associated with post-ischemic acute renal failure was assayed using a procedure described by Vaziri et al. (1994, Am J Physiol 266(3 Pt 2):F360-6.) Fifteen Sprague Dawley male rats (280-300 g) were obtained from Charles River Laboratories. On day 0, rats were treated by intraperitoneal injection with a single dose of saline (control; n = 3) at 8 ml/kg, or cisplatin (CP; Bedford Laboratories, Bedford OH) at either 7 mg/kg (7 ml/kg; n = 6) or 10 mg/kg (10 ml/kg; n = 6). Blood samples (0.2 ml) were collected on days 5, 9, and 16 as follows. Animals were anesthetized with isoflurane and 0.2 ml of blood was collected from the tail vein into a MICROTAINER EDTA-2K tube (Becton-Dickinson). Blood samples were processed for hematocrit as described above to determine the degree of anemia produced in each animal.

Beginning on day 19, one half of each cisplatin-treated group (n = 3 x 2) and all of the control group were treated by oral gavage once per day for 5 consecutive days with a 2 ml/kg volume of 0.5% CMC (Sigma-Aldrich). The other half of each cisplatin-treated group (n = 3 x 2) was treated by oral gavage once per day for five consecutive days with a 2 ml/kg volume of 2.5% compound C (25 mg/ml in 0.5% CMC). Blood samples (0.5 ml) were collected as described above immediately prior to treatment and 4 days after treatment initiation. Blood samples were analyzed for CBC and reticulocyte counts as described above. On day 9 after initiation of oral treatment, a blood sample (0.1 ml) was collected and processed for hematocrit as described above.

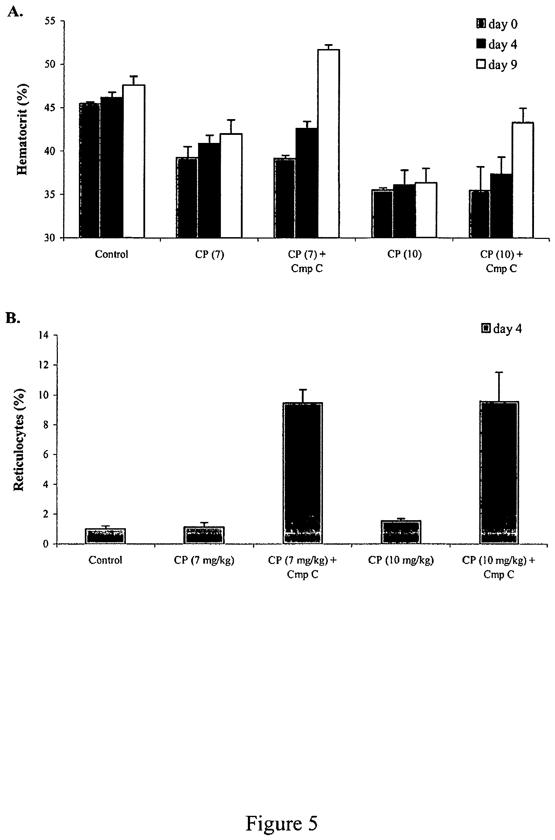

Figure 5A shows that, prior to treatment with the methods of the invention, exposure to 7 and 10 mg/kg CP reduced hematocrit by 14 and 22%, respectively, relative to controls by day 19. The compound of the invention, however, increased hematocrit in the CP-treated animals 4 days after initiating treatment with compound, and hematocrits were significantly higher than non-treated counterparts by day 9 post-treatment. As can be seen in Figure 5A, hematocrit levels in animals initially exposed to 7 mg/kg CP and subsequently treated with the compound of the invention were at or above normal control values by day 9. Figure 5B shows that the increase in hematocrit was due to the formation of new red blood cells, as the number of circulating reticulocytes was also increased in animals treated with the compound.

### Example 5: Treatment of Hemolytic Anemia.

Hemolytic anemia can be caused by numerous factors including exposure to toxins, hemodialysis. The ability of a compound of the invention to treat hemolytic anemia is assayed using a procedure described by Rencricca et al. (1970, Blood 36:764-71.) Briefly, mice are treated by oral gavage 2 times per day for 5 days with a 2 ml/kg volume of either 0.5% CMC (Sigma-Aldrich) (group A) or a compound of the invention. Beginning at day 3, mice are treated with 3 consecutive daily subcutaneous doses of either saline or 60 mg/kg phenylhydrazine (PHZ). On day 8, blood is drawn from each of the experimental animals by cardiac puncture and the hematocrit is measured. Mice that receive PHZ alone should exhibit the lowest hematocrit levels (approximately 50% of control) 4 days after the first PHZ administration. Hematocrit levels return to normal in about 8 days. Treatment with the compound of the invention should minimize the drop in hematocrit levels in mice as compared to the vehicle-treated controls.

Alternatively, rats are treated using a procedure described by Criswell et al. (2000, J Appl Toxicol 20:25-34.) The procedure is essentially as described above for mice, except rats are given 50 mg/kg PHZ by intraperitoneal injection instead of by subcutaneous administration. Maximum decreases (68%) in red blood counts are expected on day three in PHZ-treated animals.

### Example 6: Treatment of Anemia Associated with Renal Failure

The ability of a compound of the invention to treat anemia associated with post-ischemic acute renal failure is assayed using a procedure described by Tan et al. (1996, Kidney Int 50:1958-64.) Briefly, rats are subjected to unilateral clamping of the left renal artery for one hour. The arterial clamp is then removed and the incisional wound is closed. Rats are treated by oral gavage two times per day with a 2 ml/kg volume of either 0.5% CMC (Sigma-Aldrich) (group A) or 5% a compound of the invention. At 2 hours, 24 hours, and 1 week following release of the arterial clamp, blood is drawn for determination of the hematocrit. Hematocrit values in the rats treated with vehicle are expected to be about 85%, 91%, and 93% of sham control rats at the respective time points.

Additionally, the ability of a compound of the invention to treat anemia associated with ischemic acute renal failure is assayed using a procedure described by Nemoto et al. (2001, Kidney Int 59:246-51.) Briefly, rats are treated by oral gavage two times per day with a 2 ml/kg volume of either 0.5% CMC (Sigma-Aldrich) (group A) or 5% a compound of the invention. Rats are subjected to clamping of the right kidney with a vascular clip with simultaneous nephrectomy of the left kidney. After each occlusion, the clip is released at either 30 (moderate) or 45 (severe) minutes, and reperfusion is observed.

### Example 7: Erythropoietin Expression in vivo.

Twenty-five male Swiss Webster mice (35-38 g) were obtained from Simonson, Inc., and treated by oral gavage once per day for 2.5 days (5 doses) with a 4 ml/kg volume of either 0.5% carboxymethyl cellulose (CMC; Sigma-Aldrich, St. Louis MO) (0 mg/kg/day), compound C at 30 or 100 mg/kg/day for 4 days, or blood was collected daily from animals as described above to induce anemia. Blood samples were collected and kidney, liver, brain, lung, heart, and skeletal muscle tissues were harvested and processed as for Example 2, Experiment I (*supra*).

RNA isolation was carried out using the following protocol. A 50 mg section of each organ was diced, 875 µl of RLT buffer (RNEASY kit; Qiagen Inc., Valencia CA) was added, and the pieces were homogenized for about 20 seconds using a rotor-stator POLYTRON homogenizer (Kinematica, Inc., Cincinnati OH). The homogenate was micro-centrifuged for 3 minutes to pellet insoluble material, the supernatant was transferred to a new tube and RNA was isolated using an RNEASY kit (Qiagen) according to the manufacturer's instructions. The RNA was eluted into 80µL of water and quantitated with RIBOGREEN reagent (Molecular Probes, Eugene OR). Genomic DNA was then removed from the RNA using a DNA-FREE kit (Ambion Inc., Austin TX) according to the manufacturer's instructions. The absorbance at 260 and 280 nm was measured to determine RNA purity and concentration.

RNA was precipitated in 0.3 M sodium acetate (pH 5.2), 50 ng/ml glycogen, and 2.5 volumes of ethanol for one hour at -20°C. Samples were centrifuged and pellets were washed with cold 80% ethanol, dried, and resuspend in water. Double stranded cDNA was synthesized using a T7-(dT)24 first strand primer (Affymetrix, Inc., Santa Clara CA) and the SUPERSCRIPT CHOICE system (Invitrogen) according to the manufacturer's instructions. The final cDNA was extracted with an equal volume of 25:24:1 phenol:chloroform:isoamyl alcohol using a PHASE LOCK GEL insert (Brinkman, Inc., Westbury NY). The aqueous phase was collected and cDNA was precipitated using 0.5 volumes of 7.5 M ammonium acetate and 2.5 volumes of ethanol. Alternatively, cDNA was purified using the GENECHIP sample cleanup module (Affymetrix) according to the manufacturer's instructions.

Biotin-labeled cRNA was synthesized from the cDNA in an *in vitro* translation (IVT) reaction using a BIOARRAY HighYield RNA transcript labeling kit (Enzo Diagnostics, Inc., Farmingdale NY) according to the manufacturer's instructions. Final labeled product was purified and fragmented using the GENECHIP sample cleanup module (Affymetrix) according to the manufacturer's instructions.

Hybridization cocktail was prepared by bringing 5 µg probe to 100 µl in 1x hybridization buffer (100 mM MES, 1 M [Na⁺], 20 mM EDTA, 0.01% Tween 20), 100 µg/ml herring sperm DNA, 500 µg/ml acetylated BSA, 0.03 nM contol oligo B2 (Affymetrix), and 1x GENECHIP eukaryotic hybridization control (Affymetrix). The cocktail was sequentially incubated at 99°C for 5 minutes and 45°C for 5 minutes, and then centrifuged for 5 minutes. The Murine genome U74AV2 array (MG-U74Av2; Affymetrix) was brought to room temperature and then prehybridized with 1x hybridization buffer at 45°C for 10 minutes with rotation. The buffer was then replaced with 80 µl hybridization cocktail and the array was hybridized for 16 hours at 45°C at 60 rpm with counter balance. Following hybridization, arrays were washed once with 6x SSPE, 0.1% Tween 20, and then washed and stained using R-phycoerythrin-conjugated streptavidin (Molecular Probes, Eugene OR), goat anti-streptavidin antibody (Vector Laboratories, Burlingame CA), and a GENECHIP Fluidics Station 400 instrument (Affymetrix) according to the manufacturer's micro_1v1 protocol (Affymetrix). Arrays were analyzed using a GENEARRAY scanner (Affymetrix) and Microarray Suite software (Affymetrix).

The Murine Genome U74AV2 array (Affymetrix) represents all sequences (∼6,000) in Mouse UniGene database build 74 (National Center for Biotechnology Information, Bethesda MD) that have been functionally characterized and approximately 6,000 unannotated expressed sequence tag (EST) clusters.

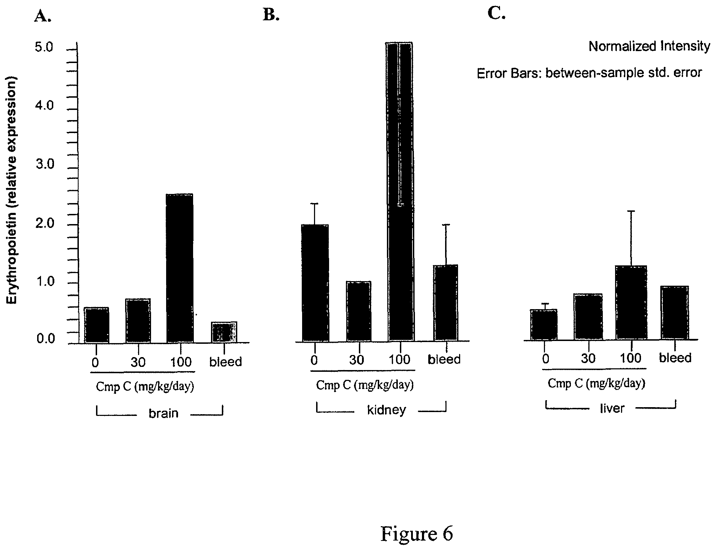

As can be seen in Figures 6A, 6B, and 6C, a dose-dependent increase in erythropoietin transcript was seen in brain, kidney, and liver tissues, respectively. The level of expression was equivalent to or exceeded the level of transcription seen with bleed-induced anemia in the same tisssue. Thus, the compounds used in the methods of the invention can penetrate and induce endogenous erythropoietin production in organs that normally produce erythropoietin in animals.

### Example 8: Erythropoietin production Following Bilateral Nephrectomy

The ability of a compound of the invention to induce endogenous erythropoietin production in the absence of functioning kidney was assayed using a procedure described by Jacobson et al. (1957, Nature 179:633-634.) Briefly, rats were anesthetized under isoflurane and a midline abdominal incision was made under sterile conditions. The kidney capsules were peeled off, the pedicles were ligated, and both kidneys were removed. The abdomen was then closed and the animal was allowed to recover.

Animals were treated by oral gavage at 2 and 20 hours post-surgery with either 0.5% carboxymethyl cellulose (CMC; Sigma-Aldrich, St. Louis MO) or with compound C at 100 or 150 mg/kg. Blood samples (0.6 ml) were collected at 24 hours as follows. Animals were anesthetized with isoflurane and blood was collected from the tail vein into a MICROTAINER EDTA-2K tube (Becton-Dickinson). Blood samples were processed for erythropoietin levels and hematocrit as described in Example 2. Complete Blood Count (CBC) analysis, including blood hemoglobin level, reticulocyte number, and hematocrit, was performed by IDEXX veterinary service (W. Sacramento, CA).

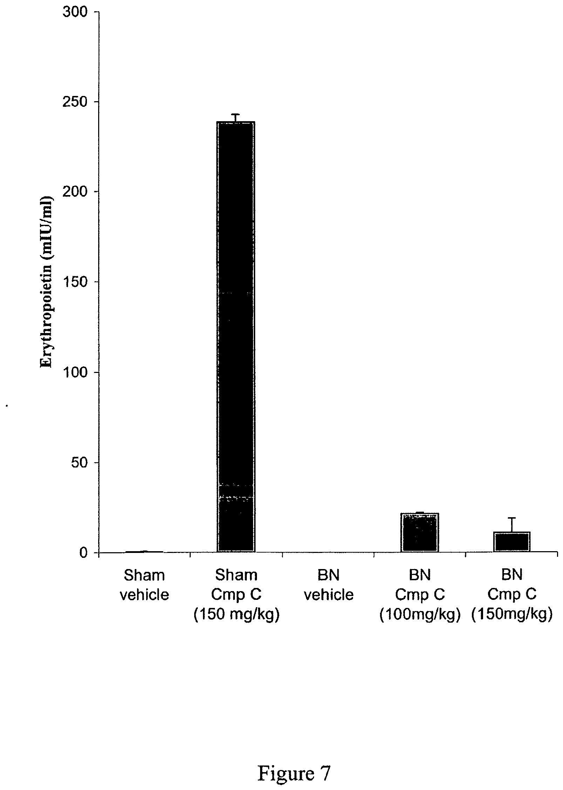

As can be seen in Figure 7, the compound significantly increased serum erythropoietin levels in sham-operated animals. Further, serum erythropoietin levels noticably increased in bilaterally nephrectomized animals treated with the compound relative to untreated BN and sham controls (Figure 7).

### Example 9: Screening Assay

Compounds that increase endogenous erythropoietin levels can be identified using the procedure described in Example 1. Additional compounds that inhibit HIF-specific prolyl hydroxylase activity and stabilize HIFα, increasing endogenous erythropoietin, can be identified and characterized using the following assay. A 50 µl aliquot of a reaction mix containing 4 mg/ml BSA, 0.1 M Tris HCl (pH 7.2), 2 mM ascorbate, 80 µM ferrous sulfate, 0.2 mM 2-oxoglutarate, 600 units/ml catalase, with or without 100 µM HIFα peptide, is mixed with 50 µl HeLa cell extract or purified HIF prolyl hydroxylase and incubated 1.5 hours at 37°C. Following incubation, 50 µl of streptavidin beads are added and the mixture is incubated for 1 hour with agitation at 4°C. The mixture is transferred to tubes and centrifuged at low speed to pellet the beads. The beads are washed three times with 0.5-1 ml 20 mM Tris HCl (pH 7.2). The peptide is then eluted from the beads with 5 µl 2 mM biotin in 20 mM Tris HCl (pH 7.2) for 1 hour. The tubes are centrifuged to pellet the resin and 40-50 µl of supernatant is removed and an equal volume of acetonitrile is added. Alternatively, the peptide is attached to methoxycoumarin, a pH insensitive fluorophore. The fluorophore may provide sensitivity and specificity to enhance detection in assays run with crude cell lysate. An exemplary HIF peptide for use in the screening assay may comprise [methoxycoumarin]-DLDLEALAPYIPADDDFQL-amide (SEQ ID NO:5). The non-hydroxylated and hydroxylated peptides are then separated by reverse-phase HPLC on a C18 column with UV detection at 214 nm.

## Claims

1. A compound that inhibits HIF prolyl hydroxylase enzyme activity for use in increasing endogenous erythropoietin in preventing or treating anemia associated with kidney disease in a subject, wherein the compound is a compound of formula (I): 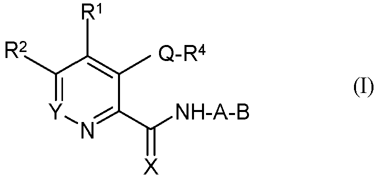 wherein
A is (C₁-C₄)-alkylene;
B is -CO₂H, -NH₂, -NHSO₂CF₃, tetrazolyl, imidazolyl, 3-hydroxyisoxazolyl, -CONHCOR‴, - CONHSOR‴, CONHSO₂R‴, where R‴ is aryl, heteroaryl, (C₃-C₇)-cycloalkyl, or (C₁-C₄)-alkyl, optionally monosubstituted by (C₆-C₁₂)-aryl, heteroaryl, OH, SH, (C₁-C₄)-alkyl, (C₁-C₄)-alkoxy, (C₁-C₄)-thioalkyl, (C₁-C₄)-sulfinyl, (C₁-C₄)-sulfonyl, CF₃, Cl, Br, F, I, NO2, -COOH, (C₂-C₅)-alkoxycarbonyl, NH₂, mono-(C₁-C₄-alkyl)-amino, di-(C₁-C₄-alkyl)-amino, or (C₁-C₄)-perfluoroalkyl; or wherein B is a CO₂-G carboxyl radical, where G is a radical of an alcohol G-OH in which G is selected from (C₁-C₂₀)-alkyl radical, (C₃-C₈) cycloalkyl radical, (C₂-C₂₀)-alkenyl radical, (C₃-C₈)-cycloalkenyl radical, retinyl radical, (C₂-C₂₀)-alkynyl radical, (C₄-C₂₀)-alkenynyl radical, where the alkenyl, cycloalkenyl, alkynyl, and alkenynyl radicals contain one or more multiple bonds; (C₆-C₁₆)-carbocyclic aryl radical, (C₇-C₁₆)-carbocyclic aralkyl radical, heteroaryl radical, or heteroaralkyl radical, wherein a heteroaryl radical or heteroaryl moiety of a heteroaralkyl radical contains 5 or 6 ring atoms; and wherein radicals defined for G are substituted by one or more hydroxyl, halogen, cyano, trifluoromethyl, nitro, carboxyl, (C₁-C₁₂)-alkyl, (C₃-C₈)-cycloalkyl, (C₅-C₈)-cycloalkenyl, (C₆-C₁₂)-aryl, (C₇-C₁₆)-aralkyl, (C₂-C₁₂)-alkenyl, (C₂-C₁₂)-alkynyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, (C₆-C₁₂)-aryloxy, (C₇-C₁₆)-aralkyloxy, (C₁-C₈)-hydroxyalkyl, -O-[CH₂]ₓ-C_{f}H_{(2f+1-g)}-F_{g},-OCF₂Cl, -OCF₂-CHFCl, (C₁-C₁₂)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₂-C₁₂)-alkenylcarbonyl, (C₂-C₁₂)-alkynylcarbonyl, (C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, acyloxy, (C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆) aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy, carbamoyl, N-(C₁-C₁₂)-alkylcarbamoyl, N.N-di(C₁-C₁₂)-alkylcarbamoyl, N-(C₃-C₈)-cycloalkyl-carbamoyl, N-(C₆-C₁₆)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₀)-alkoxy-(C₁-C₁₀)-alkyl)-carbamoyl, N-((C₆-C₁₂)-aryloxy-(C₁-C₁₀)alkyl)-carbamoyl, N-((C₇-C₁₆)-aralkyloxy-(C₁-C₁₀)-alkyl)-carbamoyl, N-(C₁-C₁₀)-alkyl-N-((C₁-C₁₀)-alkoxy-(C₁-C₁₀)-alkyl)-carbamoyl, N-(C₁-C₁₀)-alkyl-N-((C₆-C₁₆)-aryloxy-(C₁-C₁₀)-alkyl)-carbamoyl, N-(C₁-C₁₀)-alkyl-N-((C₇-C₁₆)-aralkyloxy-(C₁-C₁₀)-alkyl)-carbamoyl, carbamoyloxy, N-(C₁-C₁₂)-alkylcarbamoyloxy, N.N-di-(C₁-C₁₂)-alkylcarbamoyloxy, N-(C₃-C₈)-cycloalkylcarbamoyloxy, N-(C₆-C₁₂)-arylcarbamoyloxy, N-(C₇-C₁₆)-aralkylcarbamoyloxy, N-(C₁-C₁₀)-alkyl-N-(C₆-C₁₂)-arylcarbamoyloxy, N(C₁-C₁₀)-alkyl-N-(C₇-C₁₆)-aralkylcarbamoyloxy, N-((C₁-C₁₀)-alkyl)-carbamoyloxy, N-((C₆-C₁₂)-aryloxy-(C₁-C₁₀)-alkyl)-carbamoyloxy, N-((C₇-C₁₆)-aralkyloxy-(C₁-C₁₀)-alkyl)-carbamoyloxy, N-(C₁-C₁₀)-alkyl-N-((C₁-C₁₀)-alkoxy-(C₁-C₁₀)-alkyl)-carbamoyloxy, N-(C₁-C₁₀)-alkyl-N-((C₆-C₁₂)-aryloxy-(C₁-C₁₀)-alkyl)-carbamoyloxy, N-(C₁-C₁₀)-alkyl-N-((C₇-C₁₆)-aralkyloxy-(C₁-C₁₀)-alkyl)-carbamoyloxy, amino, (C₁-C₁₂)-alkylamino, di-(C₁-C₁₂)-alkylamino, (C₃-C₈)-cycloalkylamino, (C₂-C₁₂)-alkenylamino, (C₂-C₁₂)-alkynylamino, N-(C₆-C₁₂)-arylamino, N-(C-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino, (C₁-C₁₂)-alkylcarbonylamino, (C₃-C₈)-cycloalkylcarbonylamino, (C₆-C₁₂) arylcarbonylamino, (C₇-C₁₆)-aralkylcarbonylamino, (C₁-C₁₂)-alkylcarbonyl-N-(C₁-C₁₀)-alkylamino, (C₃-C₈)-cycloalkylcarbonyl-N-(C₁-C₁₀)-alkylamino, (C₆-C₁₂)-arylcarbonyl-N-(C₁-C₁₀)alkylamino, (C₇-C₁₁)-aralkylcarbonyl-N-(C₁-C₁₀)-alkylamino, (C₁-C₁₂)-alkylcarbonylamino-(C₁-C₈)-alkyl, (C₃-C₈)-cycloalkylcarbonylamino-(C₁-C₈)alkyl, (C₆-C₁₂)-arylcarbonylamino-(C₁-C₈)-alkyl, (C₇-C₁₂)-aralkylcarbonylamino(C₁-C₈)-alkyl, amino-(C₁-C₁₀)-alkyl, N-(C₁-C₁₀) alkylamino-(C₁-C₁₀)-alkyl, N.N-di-(C₁-C₁₀)-alkylamino-(C₁-C₁₀)-alkyl, (C₃-C₈)cycloalkylamino-(C₁-C₁₀)-alkyl, (C₁-C₁₂)-alkylmercapto, (C₁-₁₂)-alkylsulfinyl, (C₁-C₁₂)-alkylsulfonyl, (C₆-C₁₆)-arylmercapto, (C₆-C₁₆)-arylsulfinyl, (C₆-C₁₂)-arylsulfonyl, (C₇-C₁₆)-aralkylmercapto, (C₇-C₁₆)-aralkylsulfinyl, (C₇-C₁₆)-aralkylsulfonyl, sulfamoyl, N-(C₁-C₁₀)-alkylsulfamoyl, N.N-di(C₁-C₁₀)-alkylsulfamoyl, (C₃-C₈)-cycloalkylsulfamoyl, N-(C₆-C₁₂)-alkylsulfamoyl, N-(C₇-C₁₆)-aralkylsulfamoyl, N-(C₁-C₁₀)-alkyl-N-(C₆-C₁₂)-arylsulfamoyl, N-(C₁-C₁₀)-alkyl-N-(C₇-C₁₆)-aralkylsulfamoyl, (C₁-C₁₀)-alkylsulfonamido, N-((C₁-C₁₀)-alkyl)-(C₁-C₁₀)-alkylsulfonamido, (C₇-C₁₆)-aralkylsulfonamido, or N-((C₁-C₁₀)-alkyl-(C₇-C₁₆)-aralkylsulfonamido; wherein radicals which are aryl or contain an aryl moiety, may be substituted on the aryl by one to five identical or different hydroxyl, halogen, cyano, trifluoromethyl, nitro, carboxyl, (C₁-C₁₂)-alkyl, (C₃-C₈)-cycloalkyl, (C₆-C₁₂)-aryl, (C₇-C₁₆)-aralkyl, (C₁-C₁₂)-alkoxy, (C₁-C₁₂)-alkoxy-(C₁-C₁₂)alkyl, (C₁-C₁₂)-alkoxy-(C₁ C₁₂)alkoxy, (C₆-C₁₂)-aryloxy, (C₇-C₁₆)-aralkyloxy, (C₁-C₈)-hydroxyalkyl, (C₁-C₁₂)-alkylcarbonyl, (C₃-C₈)-cycloalkyl-carbonyl, (C₆-C₁₂)-arylcarbonyl, (C₇-C₁₆) aralkylcarbonyl, (C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, (C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy, (C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy, carbamoyl, N-(C₁-C₁₂)-alkylcarbamoyl, N.N-di-(C₁-C₁₂)-alkylcarbamoyl, N-(C₃-C₈)-cycloalkylcarbamoyl, N-(C₆-C₁₂)-arylcarbamoyl, N-(C₇-C₁₆)-aralkylcarbamoyl, N-(C₁-C₁₀)-alkyl-N-(C₆-C₁₂)-arylcarbamoyl, N-(C₁-C₁₀)-alkyl-N-(C₇-C₁₆)-aralkylcarbamoyl, N-((C₁-C₁₀)-alkoxy-(C₁-C₁₀)-alkyl)-carbamoyl, N-((C₆-C₁₂)-aryloxy-(C₁-C₁₀)-alkyl)-carbamoyl, N-((C₇-C₁₆)-aralkyloxy-(C₁-C₁₀)-alkyl)-carbamoyl, N-(C₁-C₁₀)-alkyl-N-((C₁-C₁₀)-alkoxy-(C₁-C₁₀)-alkyl)-carbamoyl, N-(C₁-C₁₀)-alkyl-N-((C₆-C₁₂)-aryloxy-(C₁-C₁₀)-alkyl)-carbamoyl, N-(C₁-C₁₀)-alkyl-N-((C₇-C₁₆)-aralkyloxy-(C₁-C₁₀)-alkyl)-carbamoyl, carbamoyloxy, N-(C₁-C₁₂)-alkylcarbamoyloxy, N.N-di-(C₁-C₁₂)-alkylcarbamoyloxy, N-(C₃-C₈)-cycloalkylcarbamoyloxy, N-(C₆-C₁₂)-arylcarbamoyloxy, N-(C₇-C₁₆)-aralkylcarbamoyloxy, N-(C₁-C₁₀)-alkyl-N-(C₆-C₁₂)-arylcarbamoyloxy, N(C₁-C₁₀)-alkyl-N-(C₇-C₁₆)-aralkylcarbamoyloxy, N-((C₁-C₁₀)-alkyl)-carbamoyloxy, N-((C₆-C₁₂)-aryloxy-(C₁-C₁₀)-alkyl)-carbamoyloxy, N-((C₇-C₁₆)-aralkyloxy-(C₁-C₁₀)-alkyl)-carbamoyloxy, N-(C₁-C₁₀)-alkyl-N-((C₁-C₁₀)-alkoxy-(C₁-C₁₀)-alkyl)-carbamoyloxy, N-(C₁-C₁₀)-alkyl-N-((C₆-C₁₂)-aryloxy-(C₁-C₁₀)-alkyl)-carbamoyloxy, N-(C₁-C₁₀)-alkyl-N-((C₇-C₁₆)-aralkyloxy-(C₁-C₁₀)-alkyl)-carbamoyloxy, amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkylaralkylamino, N-alkyl-arylamino, (C₁-C₁₂)-alkoxyamino, (C₁-C₁₂)-alkoxy-N-(C₁-C₁₀)-alkylamino, (C₁-C₁₂)-alkylcarbonylamino, (C₃-C₈)-cycloalkylcarbonylamino, (C₆-C₁₂)-arylcarbonylamino, (C₇-C₁₆)-alkylcarbonylamino, (C₁-C₁₂)-alkylcarbonyl-N-(C₁-C₁₀)-alkylamino, (C₃-C₈)-cycloalkylcarbonyl-N-(C₁-C₁₀)-alkylamino, (C₆-C₁₂)-arylcarbonyl-N-(C₁-C₁₀)-alkylamino, (C₇-C₁₁)-aralkylcarbonyl-N-(C₁-C₁₀)-alkylamino, (C₁-C₁₂)-alkylcarbonylamino-(C₁-C₈)-alkyl, (C₃-C₈)-cycloalkylcarbonylamino-(C₁-C₈)-alkyl, (C₆-C₁₂)-arylcarbonylamino-(C₁-C₈)-alkyl, (C₇-C₁₆)-aralkylcarbonylamino-(C₁-C₈)-alkyl, amino-(Ci-C₁₀)-alkyl, N-(C₁-C₁₀)-alkylamino-(C₁-C₁₀)alkyl, N.N-di-(C₁-C₁₀)-alkylamino-(C₁-C₁₀)-alkyl, (C₃-C₈)-cycloalkylamino-(C₁-C₁₀)-alkyl, (C₁-C₁₂)-alkylmercapto, (C₁-₁₂)-alkylsulfinyl, (C₁-C₁₂)-alkylsulfonyl, (C₆-C₁₂)-arylmercapto, (C₆-C₁₂)-arylsulfinyl, (C₆-C₁₂)-arylsulfonyl, (C₇-C₁₆)-aralkylmercapto, (C₇-C₁₆)-aralkylsulfinyl, or (C₇-C₁₆)-aralkylsulfonyl;
X is O or S;
Q is O, S, NR', or a bond;
where, if Q is a bond, R⁴ is halogen, nitrile, or trifluoromethyl;
or where, if Q is O, S, or NR', R⁴ is hydrogen, (C₁-C₁₀)-alkyl radical, (C₂-C₁₀)-alkenyl radical, (C₂-C₁₀)-alkynyl radical, wherein alkenyl or alkynyl radical contains one or two C-C multiple bonds; unsubstituted fluoroalkyl radical of the formula -[CH₂]ₓ-C_{f}H_{(2f+1-g)}-F_{g}, (C₁-C₈)-alkoxy-(C₁-C₆)-alkyl radical, (C₁-C₆)-alkoxy-(C₁-C₄)-alkoxy-(C₁-C₄)-alkyl radical, aryl radical, heteroaryl radical, (C₇-C₁₁)-aralkyl radical, or a radical of the formula Z
-[CH₂]ᵥ-[O]_{w}-[CH₂]ₜ-E (Z)
where
E is a heteroaryl radical, a (C₃-C₈)-cycloalkyl radical, or a phenyl radical of the formula F 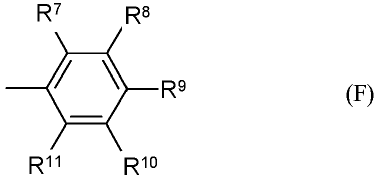
v is 0-6,
w is 0 or 1,
t is 0-3, and
R⁷, R⁸, R⁹, R¹⁰, and R¹¹ are identical or different and are hydrogen, halogen, cyano, nitro, trifluoromethyl, (C₁-C₆)-alkyl, (C₃-C₈)-cycloalkyl, (C₁-C₆)-alkoxy, -O-[CH₂]ₓ-C_{f}H_{(2f+1-g)}-F_{g},-OCF₂-Cl, -O-CF₂-CHFCl, (C₁-C₆)-alkylmercapto, (C₁-C₆)-hydroxyalkyl, (C₁-C₆)-alkoxy-(C₁-C₆)-alkoxy, (C₁-C₆)-alkoxy-(C₁-C₆)-alkyl, (C₁-C₆)-alkylsulfinyl, (C₁-C₆)-alkylsulfonyl, (C₁-C₆)-alkylcarbonyl, (C₁-C₈)-alkoxycarbonyl, carbamoyl, N-(C₁-C₈)-alkylcarbamoyl, N,N-di-(C₁-C₈)-alkylcarbamoyl, or (C₇-C₁₁)-aralkylcarbamoyl, optionally substituted by fluorine, chlorine, bromine, trifluoromethyl, (C₁-C₆)-alkoxy, N-(C₃-C₈)-cycloalkylcarbamoyl, N-(C₃-C₈)-cycloalkyl-(C₁-C₄)-alkylcarbamoyl, (C₁-C₆)-alkylcarbonyloxy, phenyl, benzyl, phenoxy, benzyloxy, NR^{Y}R^{Z} wherein R^{y} and R^{z} are independently selected from hydrogen, (C₁-C₁₂)-alkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₃-C₁₀)-cycloalkyl, (C₃-C₁₂)-alkenyl, (C₃-C₁₂)-alkynyl, (C₆-C₁₂)-aryl, (C₇-C₁₁)-aralkyl, (C₁-C₁₂)-alkoxy, (C₇-C₁₂)aralkoxy, (C₁-C₁₂)-alkylcarbonyl, (C₃-C₈)-cycloalkylcarbonyl, (C₆-C₁₂) arylcarbonyl, (C₇-C₁₆)-aralkylcarbonyl; or further wherein R^{y} and R^{z} together are -[CH2 ]ₕ, in which a CH₂ group can be replaced by O, S, N-(C₁-C₄)-alkylcarbonylimino, or N-(C₁-C₄)-alkoxycarbonylimino; phenylmercapto, phenylsulfonyl, phenylsulfinyl, sulfamoyl, N-(C₁-C₈)-alkylsulfamoyl, or N, N-di-(C₁-C₈)-alkylsulfamoyl; or alternatively R⁷ and R⁸, R⁸ and R⁹, R⁹ and R¹⁰, or R¹⁰ and R¹¹, together are a chain selected from -[CH₂]ₙ- or -CH=CH-CH=CH-, where a CH₂ group of the chain is optionally replaced by O, S, SO, SO₂, or NR^{Y}; and n is 3, 4, or 5; and if E is a heteroaryl radical, said radical can carry 1-3 substituents selected from those defined for R⁷-R¹¹, or if E is a cycloalkyl radical, the radical can carry one substituent selected from those defined for R⁷-R¹¹;
or where, if Q is NR', R⁴ is alternatively R", where R' and R" are identical or different and are hydrogen, (C₆-C₁₂)-aryl, (C₇-C₁₁)-aralkyl, (C₁-C₈)-alkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C8)-alkyl, (C₁-C₁₀)-alkylcarbonyl, optionally substituted (C₇-C₁₆)-aralkylcarbonyl, or optionally substituted C₆-C₁₂)-arylcarbonyl; or R' and R" together are -[CH₂]ₕ, in which a CH₂ group can be replaced by O, S, N-acylimino, or N-(C₁-C₁₀)-alkoxycarbonylimino, and h is 3 to 7;
Y is N or CR³;
R¹, R² and R³ are identical or different and are hydrogen, hydroxyl, halogen, cyano, trifluoromethyl, nitro, carboxyl, (C₁-C₂₀)-alkyl, (C₃-C₈)-cycloalkyl, (C₃-C₈)cycloalkyl-(C₁-C₁₂)-alkyl, (C₃-C₈)-cycloalkoxy, (C₃-C₈)-cycloalkyl-(C₁-C₁₂)-alkoxy, (C₃-C₈)-cycloalkyloxy-(C₁-C₁₂)-alkyl, (C₃-C₈)-cycloalkyloxy-(C₁-C₁₂)-alkoxy, (C₃-C₈)-cycloalkyl-(C₁-C₈)-alkyl-(C₁-C₆)-alkoxy, (C₃-C₈)-cycloalkyl-(C₁-C₈)-alkoxy-(C₁-C₆)-alkyl, (C₃-C₈)-cycloalkyloxy-(C₁-C₈)-alkoxy-(C₁-C₆)-alkyl, (C₃-C₈)-cycloalkoxy-(C₁-C₈)-alkoxy-(C₁-C₈)-alkoxy, (C₆-C₁₂)-aryl, (C₇-C₁₆)-aralkyl, (C₇-C₁₆)-aralkenyl, (C₇-C₁₆)-aralkynyl, (C₂-C₂₀)-alkenyl, (C₂-C₂₀)-alkynyl, (C₁-C₂₀)-alkoxy, (C₂-C₂₀)-alkenyloxy, (C₂-C₂₀)-alkynyloxy, retinyloxy, (C₁-C₂₀)-alkoxy-(C₁-C₁₂)-alkyl, (C₁-C₁₂)-alkoxy-(C₁-C₁₂)-alkoxy, (C₁-C₁₂)-alkoxy-(C₁-C₈)-alkoxy-(C₁-C₈)-alkyl, (C₆-C₁₂)-aryloxy, (C₇-C₁₆)-aralkyloxy, (C₆-C₁₂)-aryloxy-(C₁-C₆)-alkoxy, (C₇-C₁₆)-aralkoxy-(C₁-C₆)-alkoxy, (C₁-C₁₆)-hydroxyalkyl, (C₆-C₁₆)-aryloxy-(C₁-C₈)-alkyl, (C₇-C₁₆)-aralkoxy-(C₁-C₈)-alkyl, (C₆-C₁₂)-aryloxy-(C₁-C₈)-alkoxy-(C₁-C₆)-alkyl, (C₇-C₁₂)-aralkyloxy-(C₁-C₈)-alkoxy-(C₁-C₆)-alkyl, (C₂-C₂₀)-alkenyloxy-(C₁-C₆)-alkyl, (C₂-C₂₀)-alkynyloxy-(C₁-C₆)-alkyl, retinyloxy-(C₁-C₆)-alkyl, -O-[CH₂]ₓCfH_{(2f+1-g)}F_{g}, -OCF₂Cl, -OCF₂-CHFCl, (C₁-C₂₀)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₂-C₂₀)-alkenylcarbonyl, (C₂-C₂₀)-alkynylcarbonyl, (C₁-C₂₀)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₂₀)-alkenyloxycarbonyl, retinyloxycarbonyl, (C₂-C₂₀)-alkynyloxycarbonyl, (C₆-C₁₂)-aryloxy-(C₁-C₆)-alkoxycarbonyl, (C₇-C₁₆)-aralkoxy-(C₁-C₆)-alkoxycarbonyl, (C₃-C₈)-cycloalkyl-(C₁-C₆)-alkoxycarbonyl, (C₃-C₈)-cycloalkoxy-(C₁-C₆)-alkoxycarbonyl, (C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy, (C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy, carbamoyl, N-(C₁-C₁₂)-alkylcarbamoyl, N,N-di-(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)-carbamoyl, N-(C₁-C₆)-alkyl-N-((C₃-C₈)-cycloalkyl-(C₁-C₆)-alkyl)-carbamoyl, N-(+)-dehydroabietylcarbamoyl, N-(C₁-C₆)-alkyl-N-(+)-dehydroabietylcarbamoyl, N-(C₆-C₁₂)-arylcarbamoyl, N-(C₇-C₁₆)-aralkylcarbamoyl, N-(Ci-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₈)-alkoxy-(C₁-C₁₀)-alkyl)-carbamoyl, N-((C₆-C₁₆)-aryloxy-(C₁-C₁₀)-alkyl)-carbamoyl, N-((C₇-C₁₆)-aralkyloxy-(C₁-C₁₀)-alkyl)-carbamoyl, N-(C₁-C₁₀)-alkyl-N-((C₁-C₁₀)-alkoxy-(C₁-C₁₀)-alkyl)-carbamoyl, N-(C₁-C₁₀)-alkyl-N-((C₆-C₁₂)-aryloxy-(C₁-C₁₀)-alkyl)-carbamoyl, N-(C₁-C₁₀)-alkyl-N-((C₇-C₁₆)-aralkyloxy-(C₁-C₁₀)-alkyl)-carbamoyl; CON(CH₂)ₕ, in which a CH₂ group can be replaced by O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino, N-(C₁-C₄)-alkoxy-(C₁-C₆)-alkylimino, and h is from 3 to 7; a carbamoyl radical of the formula R 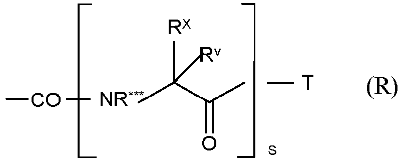 in which
R^{X} and R^{v} are each independently selected from hydrogen, (C₁-C₆)-alkyl, (C₃-C₇)-cycloalkyl, or aryl,
s is 1-5,
T is OH, or NR*R**, and R*, R** and R*** are identical or different and are selected from hydrogen, (C₆-C₁₂)-aryl, (C₇-C₁₁)-aralkyl, (C₁-C₈)-alkyl, (C₃-C₈)-cycloalkyl, (+)-dehydroabietyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkanoyl, optionally substituted (C₇-C₁₆)-aralkanoyl, optionally substituted (C₆-C₁₂)-aroyl; or R* and R** together are -[CH₂]ₕ, in which a CH₂ group can be replaced by O, S, SO, SO₂, N-acylamino, N-(C₁-C₁₀)-alkoxycarbonylimino, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino, N-(C₁-C₄)-alkoxy-(C₁-C₆)-alkylimino, and h is from 3 to 7;
carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₂)-arylcarbamoyloxy, N-(C₇-c₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkyl)-carbamoyloxy, N-((C₆-C₁₂)-aryloxy-(C₁-C₁₀)-alkyl)-carbamoyloxy, N-((C₇-C₁₆)-aralkyloxy-(C₁-C₁₀)-alkyl)-carbamoyloxy, N-(C₁-C₁₀)-alkyl-N-((C₁-C₁₀)-alkoxy-(C₁-C₁₀)-alkyl)-carbamoyloxy, N-(C₁-C₁₀)-alkyl-N-((C₆-C₁₂)-aryloxy-(C₁-C₁₀)-alkyl)-carbamoyloxy, N-(C₁-C₁₀)-alkyl-N-((C₇-C₁₆)-aralkyloxy-(C₁-C₁₀)-alkyl)-carbamoyloxyamino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino, (C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino, (C₁-C₁₂)-alkanoylamino-(C₁-C₈)-alkyl, (C₃-C₈)-cycloalkanoylamino-(C₁-C₈)-alkyl, (C₆-C₁₂)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di(C₁-C₁₀)-alkylamino-(C₁-C₁₀)-alkyl, (C₃-C₈)-cycloalkylamino(C₁-C₁₀)-alkyl, (C₁-C₂₀)-alkylmercapto, (C₁-C₂₀)-alkylsulfinyl, (C₁-C₂₀)-alkylsulfonyl, (C₆-C₁₂)-arylmercapto, (C₆-C₁₂)-arylsulfinyl, (C₆-C₁₂)-arylsulfonyl, (C₇-C₁₆)-aralkylmercapto, (C₇-C₁₆)-aralkylsulfinyl, (C₇-C₁₆)-aralkylsulfonyl, (C₁-C₁₂)-alkylmercapto-(C₁-C₆)-alkyl, (C₁-C₁₂)-alkylsulfinyl-(C₁-C₆)-alkyl, (C₁-C₁₂)-alkylsulfonyl-(C₁-C₆)-alkyl, (C₆-C₁₂)-arylmercapto-(C₁-C₆)-alkyl, (C₆-C₁₂)-arylsulfinyl-(C₁-C₆)-alkyl, (C₆-C₁₂)-arylsulfonyl-(C₁-C₆)-alkyl, (C₇-C₁₆)-aralkylmercapto-(C₁-C₆)-alkyl, (C₇-C₁₆)-aralkylsulfinyl-(C₁-C₆)-alkyl, (C₇-C₁₆)-aralkylsulfonyl-(C₁-C₆)-alkyl, sulfamoyl, N-(C₁-C₁₀)-alkylsulfamoyl, N,N-di-(C₁-C₁₀)-alkylsulfamoyl, (C₃-C₈)-cycloalkylsulfamoyl, N-(C₆-C₁₂)-arylsulfamoyl, N-(C₇-C₁₆)-aralkylsulfamoyl, N-(C₁-C₁₀)-alkyl-N-(C₆-C₁₂)-arylsulfamoyl, N-(C₁-C₁₀)-alkyl-N-(C₇-C₁₆)-aralkylsulfamoyl, (C₁-C₁₀)-alkylsulfonamido, N-((C₁-C₁₀)-alkyl)-(C₁-C₁₀)-alkylsulfonamido, (C₇-C₁₆)-aralkylsulfonamido, and N-((C₁-C₁₀)-alkyl-(C₇-C₁₆)-aralkylsulfonamido; where an aryl radical may be substituted by 1 to 5 substituents selected from hydroxyl, halogen, cyano, trifluoromethyl, nitro, carboxyl, (C₂-C₁₆)-alkyl, (C₃-C₈)-cycloalkyl, (C₃-C₈)-cycloalkyl-(C₁-C₁₂)-alkyl, (C₃-C₈)-cycloalkoxy, (C₃-C₈)-cycloalkyl-(C₁-C₁₂)-alkoxy, (C₃-C₈)-cycloalkyloxy-(C₁-C₁₂)-alky, (C₃-C₈)-cycloalkyloxy-(C₁-C₁₂)-alkoxy, (C₃-C₈)-cycloalkyl-(C₁-C₈)-alkyl-(C₁-C₆)-alkoxy, (C₃-C₈)-cycloalkyl(C₁-C₈)-alkoxy-(C₁-C₆)-alkyl, (C₃-C₈)-cycloalkyloxy-(C₁-C₈)-alkoxy-(C₁-C₆)-alkyl, (C₃-C₈)-cycloalkoxy-(C₁-C₈)-alkoxy-(C₁-C₈)-alkoxy, (C₆-C₁₂)-aryl, (C₇-C₁₆)-aralkyl, (C₂-C₁₆)-alkenyl, (C₂-C₁₂)-alkynyl, (C₁-C₁₆)-alkoxy, (C₁-C₁₆)-alkenyloxy, (C₁-C₁₂)-alkoxy-(C₁-C₁₂)-alkyl, (C₁-C₁₂)-alkoxy-(C₁-C₁₂)-alkoxy, (C₁-C₁₂)-alkoxy(C₁-C₈)-alkoxy-(C₁-C₈)-alkyl, (C₆-C₁₂)-aryloxy, (C₇-C₁₆)-aralkyloxy, (C₆-C₁₂)-aryloxy-(C₁-C₆)-alkoxy, (C₇-C₁₆)-aralkoxy-(C₁-C₆)-alkoxy, (C₁-C₈)-hydroxyalkyl, (C₆-C₁₆)-aryloxy-(C₁-C₈)-alkyl, (C₇-C₁₆)-aralkoxy-(C₁-C₈)-alkyl, (C₆-C₁₂)-aryloxy-(C₁-C₈)-alkoxy-(C₁-C₆)-alkyl, (C₇-C₁₂)-aralkyloxy-(C₁-C₈)-alkoxy-(C₁-C₆)-alkyl, -O-[CH₂]ₓC_{f}H_{(2f+i-g)}Fg, -OCF₂Cl, -OCF₂-CHFCl, (C₁-C₁₂)-alkylcarbonyl, (C₃-C₈)-cycloalkylcarbonyl, (C₆-C₁₂)-arylcarbonyl, (C₇-C₁₆)-aralkylcarbonyl, (C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₁₂)-alkenyloxycarbonyl, (C₂-C₁₂)-alkynyloxycarbonyl, (C₆-C₁₂)-aryloxy-(C₁-C₆)-alkoxycarbonyl, (C₇-C₁₆)-aralkoxy-(C₁-C₆)-alkoxycarbonyl, (C₃-C₈)-cycloalkyl-(C₁-C₆)-alkoxycarbonyl, (C₃-C₈)-cycloalkoxy-(C₁-C₆)-alkoxycarbonyl, (C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₂-C₁₂)-alkenylcarbonyloxy, (C₂-C₁₂)-alkynylcarbonyloxy, (C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₂-C₁₂)-alkenyloxycarbonyloxy, (C₂-C₁₂)-alkynyloxycarbonyloxy, carbamoyl, N-(C₁-C₁₂)-alkylcarbamoyl, N,N-di(C₁-C₁₂)-alkylcarbamoyl, N-(C₃-C₈)-cycloalkylcarbamoyl, N,N-dicyclo-(C₃-C₈)-alkylcarbamoyl, N-(C₁-C₁₀)-alkyl-N-(C₃-C₈)-cycloalkylcarbamoyl, N-((C₃-C₈)-cycloalkyl-(C₁-C₆)-alkyl)carbamoyl, N-(C₁-C₆)-alkyl-N-((C₃-C₈)-cycloalkyl-(C₁-C₆)-alkyl)carbamoyl, N-(+)-dehydroabietylcarbamoyl, N-(C₁-C₆)-alkyl-N-(+)-dehydroabietylcarbamoyl, N-(C₆-C₁₂)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₆)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₂)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)-carbamoyl, CON(CH₂)ₕ, in which a CH₂ group can be replaced by, O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino, N-(C₁-C₄)-alkoxy-(C₁-C₆)-alkylimino, and h is from 3 to 7; carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₆)-arylcarbamoyloxy, N-(C₇-C₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkyl)carbamoyloxy, N-((C₆-C₁₂)-aryloxy-(C₁-C₁₀)-alkyl)carbamoyloxy, N-((C₇-C₁₆)-aralkyloxy-(C₁-C₁₀)-alkyl)carbamoyloxy, N-(C₁-C₁₀)-alkyl-N-((C₁-C₁₀)-alkoxy-(C₁-C₁₀)-alkyl)carbamoyloxy, N-(C₁-C₁₀)-alkyl-N-((C₆-C₁₂)-aryloxy-(C₁-C₁₀)-alkyl)carbamoyloxy, N-(C₁-C₁₀)-alkyl-N-((C₇-C₁₆)-aralkyloxy-(C₁-C₁₀)-alkyl)carbamoyloxy, amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-alkyl-aralkylamino, N-alkyl-arylamino, (C₁-C₁₂)-alkoxyamino, (C₁-C₁₂)-alkoxy-N-(C₁-C₁₀)-alkylamino, (C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino, (C₁-C₁₂)-alkanoylamino-(C₁-C₈)-alkyl, (C₃-C₈)-cycloalkanoylamino-(C₁-C₈)-alkyl, (C₆-C₁₂)-aroylamino- (C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylammo-(C₁-C₁₀)-alkyl, (C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkylmercapto, (C₇-C₁₆)-aralkylsulfinyl, or (C₇-C₁₆)-aralkylsulfonyl;
or wherein R¹ and R², or R² and R³ form a chain [CH₂]ₒ, which is saturated or unsaturated by a C=C double bond, in which 1 or 2 CH₂ groups are optionally replaced by O, S, SO, SO₂, or NR', and R' is hydrogen, (C₆-C₁₂)-aryl, (C₁-C₈)-alkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₁-C₈)-alkyl, (C₁-C₁₀)-alkanoyl, optionally substituted (C₇-C₁₆)-aralkanoyl, or optionally substituted (C6-C12)-aroyl; and o is 3, 4 or 5;
or wherein the radicals R¹ and R², or R² and R³, together with the pyridine or pyridazine carrying them, form a 5,6,7,8-tetrahydroisoquinoline ring, a 5,6,7,8-tetrahydroquinoline ring, or a 5,6,7,8-tetrahydrocinnoline ring;
or wherein R¹ and R², or R² and R³ form a carbocyclic or heterocyclic 5- or 6-membered aromatic ring;
or where R¹ and R², or R² and R³, together with the pyridine or pyridazine carrying them, form an optionally substituted heterocyclic ring systems selected from thienopyridines, furanopyridines, pyridopyridines, pyrimidinopyridines, imidazopyridines, thiazolopyridines, oxazolopyridines, quinoline, isoquinoline, and cinnoline;
or wherein the radicals R¹ and R², together with the pyridine carrying them, form a compound of Formula Id: 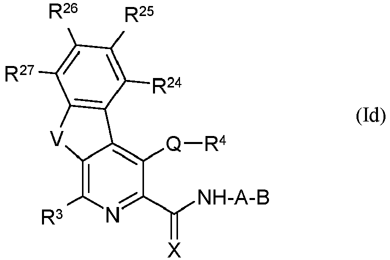
where V is S, O, or NR^{k}, and R^{k} is selected from hydrogen, (C₁-C₆)-alkyl, aryl, or benzyl; where an aryl radical may be optionally substituted by 1 to 5 substituents as defined above; and
R²⁴, R²⁵, R²⁶, and R²⁷ in each case independently of each other have the meaning of R¹, R² and R³;
f is 1 to 8;
g is 0 or 1 to (2f+1);
x is 0 to 3; and
h is 3 to 7;
including physiologically active salts thereof.

2. The compound of claim 1 for the use of that claim, wherein the compound is for oral administration.

## Patentansprüche

1. Verbindung, die die HIF-Prolylhydroxylase-Enzymaktivität inhibiert, zur Verwendung bei der Erhöhung von endogenem Erythropoietin bei der Prävention oder Behandlung von mit Nierenkrankheit assoziierter Anämie bei einem Patienten, wobei es sich bei der Verbindung um eine Verbindung der Formel (I): 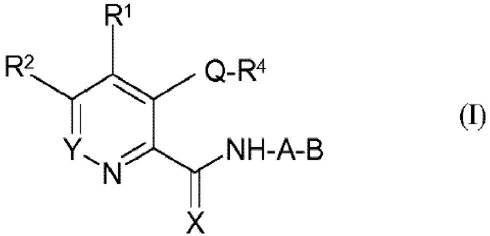 wobei
A für (C₁-C₄) -Alkylen steht;
B für -CO₂H, -NH₂, -NHSO₂CF₃, Tetrazolyl, Imidazolyl, 3-Hydroxyisoxazolyl, -CONHCOR‴, -CONHSOR‴, CONHSO₂R‴ steht, wobei R‴ für Aryl, Heteroaryl, (C₃-C₇)-Cycloalkyl oder (C₁-C₄) -Alkyl, gegebenenfalls monosubstituiert durch (C₆-C₁₂) -Aryl, Heteroaryl, OH, SH, (C₁-C₄) -Alkyl, (C₁-C₄) -Alkoxy, (C₁-C₄) -Thioalkyl, (C₁-C₄) -Sulfinyl, (C₁-C₄) -Sulfonyl, CF₃, Cl, Br, F, I, NO₂, -COOH, (C₂-C₅) -Alkoxycarbonyl, NH₂, Mono- (C₁-C₄-alkyl)-amino, Di-(C₁-C₄-alkyl)-amino oder (C₁-C₄) - Perfluoralkyl, steht; oder wobei B für einen CO₂-G-Carboxylrest steht, wobei G für einen Rest eines Alkohols G-OH steht, wobei G ausgewählt ist aus einem (C₁-C₂₀) -Alkylrest, einem (C₃-C₈) -Cycloalkylrest, einem (C₂-C₂₀) -Alkenylrest, einem (C₃-C₈) -Cycloalkenylrest, einem Retinylrest, einem (C₂-C₂₀)-Alkinylrest, einem (C₄-C₂₀) -Alkeninylrest, wobei die Alkenyl-, Cycloalkenyl-, Alkinyl- und Alkeninylreste eine oder mehrere Mehrfachbindungen enthalten; einem carbocyclischen (C₆-C₁₆) -Arylrest, einem carbocyclischen (C₇-C₁₆) -Aralkylrest, einem Heteroarylrest oder einem Heteroaralkylrest, wobei ein Heteroarylrest oder eine Heteroaryleinheit eines Heteroaralkylrests 5 oder 6 Ringatome enthält; und wobei die für G definierten Reste durch ein oder mehrere Hydroxy, Halogen, Cyano, Trifluormethyl, Nitro, Carboxyl, (C₁-C₁₂) -Alkyl, (C₃-C₈)-Cycloalkyl, (C₅-C₈) -Cycloalkenyl, (C₆-C₁₂) -Aryl, (C₇-C₁₆)-Aralkyl, (C₂-C₁₂) -Alkenyl, (C₂-C₁₂) -Alkinyl, (C₁-C₁₂) - Alkoxy, (C₁-C₁₂)-Alkoxy- (C₁-C₁₂)-alkyl, (C₁-C₁₂) -Alkoxy-(C₁-C₁₂) -alkoxy, (C₆-C₁₂)-Aryloxy, (C₇-C₁₆)-Aralkyloxy, (C₁-C₈) -Hydroxyalkyl, -O-[CH₂]ₓ-C_{f}H_{(2f+1-g)}-F_{g}, -OCF₂Cl, -OCF₂-CHFCl, (C₁-C₁₂) -Alkylcarbonyl, (C₃-C₈) -Cycloalkylcarbonyl, (C₆-C₁₂) -Arylcarbonyl, (C₇-C₁₆) -Aralkylcarbonyl, Cinnamoyl, (C₂-C₁₂) -Alkenylcarbonyl, (C₂-C₁₂) - Alkinylcarbonyl, (C₁-C₁₂) -Alkoxycarbonyl, (C₁-C₁₂) - Alkoxy- (C₁-C₁₂) -alkoxycarbonyl, (C₆-C₁₂) -Aryloxycarbonyl, (C₇-C₁₆) -Aralkoxycarbonyl, (C₃-C₈) -Cycloalkoxycarbonyl, (C₂-C₁₂) -Alkenyloxycarbonyl, (C₂-C₁₂) -Alkinyloxycarbonyl, Acyloxy, (C₁-C₁₂) -Alkoxycarbonyloxy, (C₁-C₁₂) -Alkoxy-(C₁-C₁₂) -alkoxycarbonyloxy, (C₆-C₁₂) -Aryloxycarbonyloxy, (C₇-C₁₆) -Aralkyloxycarbonyloxy, (C₃-C₈) -Cycloalkoxycarbonyloxy, (C₂-C₁₂) -Alkenyloxycarbonyloxy, (C₂-C₁₂) - Alkinyloxycarbonyloxy, Carbamoyl, N-(C₁-C₁₂) -Alkylcarbamoyl, N,N-Di-(C₁-C₁₂)-alkylcarbamoyl, N-(C₃-C₈) - Cycloalkylcarbamoyl, N-(C₆-C₁₆)-Arylcarbamoyl, N-(C₇-C₁₆) -Aralkylcarbamoyl, N-(C₁-C₁₀)-Alkyl-N-(C₆-C₁₆)-arylcarbamoyl, N-(C₁-C₁₀)-Alkyl-N-(C₇-C₁₆) -aralkylcarbamoyl, N-((C₁-C₁₀) -Alkoxy- (C₁-C₁₀) -alkyl) carbamoyl, N-((C₆-C₁₂)-Aryloxy-(C₁-C₁₀)-alkyl) carbamoyl, N-((C₇-C₁₆) - Aralkyloxy-(C₁-C₁₀)-alkyl) carbamoyl, N-(C₁-C₁₀)-Alkyl-N-((C₁-C₁₀)-alkoxy- (C₁-C₁₀)-alkyl) carbamoyl, N-(C₁-C₁₀) - Alkyl-N-((C₆-C₁₆)-aryloxy-(C₁-C₁₀)-alkyl) carbamoyl, N-(C₁-C₁₀)-Alkyl-N-((C₇-C₁₆)-aralkyloxy-(C₁-C₁₀) - alkyl) carbamoyl, Carbamoyloxy, N-(C₁-C₁₂)-Alkylcarbamoyloxy, N,N-Di-(C₁-C₁₂) -alkylcarbamoyloxy, N-(C₃-C₈) -Cycloalkylcarbamoyloxy, N-(C₆-C₁₂) -Arylcarbamoyloxy, N-(C₇-C₁₆) -Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy, N-((C₁-C₁₀)-Alkyl) - carbamoyloxy, N-((C₆-C₁₂)-Aryloxy-(C₁-C₁₀)-alkyl) - carbamoyloxy, N-((C₇-C₁₆)-Aralkyloxy-(C₁-C₁₀)-alkyl) - carbamoyloxy, N-(C₁-C₁₀)-Alkyl-N-((C₁-C₁₀) -alkoxy-(C₁-C₁₀)-alkyl) carbamoyloxy, N-(C₁-C₁₀)-Alkyl-N-((C₆-C₁₂) - aryloxy-(C₁-C₁₀)-alkyl)carbamoyloxy, N-(C₁-C₁₀)-Alkyl-N-((C₇-C₁₆)-aralkyloxy-(C₁-C₁₀)-alkyl)carbamoyloxy, Amino, (C₁-C₁₂)-Alkylamino, Di-(C₁-C₁₂)-alkylamino, (C₃-C₈)-Cycloalkylamino, (C₂-C₁₂) -Alkenylamino, (C₂-C₁₂)-Alkinylamino, N-(C₆-C₁₂) -Arylamino, N-(C-C₁₁)-Aralkylamino, N-Alkylaralkylamino, N-Alkylarylamino, (C₁-C₁₂)-Alkoxyamino, (C₁-C₁₂)-Alkoxy-N-(C₁-C₁₀)-alkylamino, (C₁-C₁₂) -Alkylcarbonylamino, (C₃-C₈) -Cycloalkylcarbonylamino, (C₆-C₁₂)-Arylcarbonylamino, (C₇-C₁₆)-Aralkylcarbonylamino, (C₁-C₁₂)-Alkylcarbonyl-N- (C₁-C₁₀)-alkylamino, (C₃-C₈) -Cycloalkylcarbonyl-N- (C₁-C₁₀) -alkylamino, (C₆-C₁₂)-Arylcarbonyl-N-(C₁-C₁₀)-alkylamino, (C₇-C₁₁)-Aralkylcarbonyl-N-(C₁-C₁₀)-alkylamino, (C₁-C₁₂) - Alkylcarbonylamino-(C₁-C₈)-alkyl, (C₃-C₈)-Cycloalkylcarbonylamino-(C₁-C₈)-alkyl, (C₆-C₁₂)-Arylcarbonylamino-(C₁-C₈) -alkyl, (C₇-C₁₂)-Aralkylcarbonylamino- (C₁-C₈) - alkyl, Amino- (C₁-C₁₀)-alkyl, N-(C₁-C₁₀)-Alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl, (C₁-C₁₂) -Alkylmercapto, (C₁-C₁₂) -Alkylsulfinyl, (C₁-C₁₂) -Alkylsulfonyl, (C₆-C₁₆)-Arylmercapto, (C₆-C₁₆)-Arylsulfinyl, (C₆-C₁₂) - Arylsulfonyl, (C₇-C₁₆)-Aralkylmercapto, (C₇-C₁₆) - Aralkylsulfinyl, (C₇-C₁₆) -Aralkylsulfonyl, Sulfamoyl, N-(C₁-C₁₀)-Alkylsulfamoyl, N,N-Di-(C₁-C₁₀)-alkylsulfamoyl, (C₃-C₈)-Cycloalkylsulfamoyl, N-(C₆-C₁₂)-Alkylsulfamoyl, N-(C₇-C₁₆) -Aralkylsulfamoyl, N-(C₁-C₁₀) -Alkyl-N-(C₆-C₁₂)-arylsulfamoyl, N-(C₁-C₁₀)-Alkyl-N-(C₇-C₁₆) - aralkylsulfamoyl, (C₁-C₁₀)-Alkylsulfonamido, N-((C₁-C₁₀)-Alkyl)-(C₁-C₁₀) -alkylsulfonamido, (C₇-C₁₆)-Aralkylsulfonamido oder N-((C₁-C₁₀)-Alkyl- (C₇-C₁₆) -aralkylsulfonamido substituiert sind; wobei Reste, bei denen es sich um Aryl handelt oder die eine Aryleinheit enthalten, am Aryl durch einen bis fünf gleiche oder verschiedene Hydroxy, Halogen, Cyano, Trifluormethyl, Nitro, Carboxyl, (C₁-C₁₂) -Alkyl, (C₃-C₈) -Cycloalkyl, (C₆-C₁₂)-Aryl, (C₇-C₁₆)-Aralkyl, (C₁-C₁₂)-Alkoxy, (C₁-C₁₂)-Alkoxy-(C₁-C₁₂)-alkyl, (C₁-C₁₂)-Alkoxy-(C₁-C₁₂) -alkoxy, (C₆-C₁₂)-Aryloxy, (C₇-C₁₆)-Aralkyloxy, (C₁-C₈)-Hydroxyalkyl, (C₁-C₁₂)-Alkylcarbonyl, (C₃-C₈)-Cycloalkylcarbonyl, (C₆-C₁₂) -Arylcarbonyl, (C₇-C₁₆) - Aralkylcarbonyl, (C₁-C₁₂) -Alkoxycarbonyl, (C₁-C₁₂) - Alkoxy- (C₁-C₁₂) -alkoxycarbonyl, (C₆-C₁₂) -Aryloxycarbonyl, (C₇-C₁₆)-Aralkoxycarbonyl, (C₃-C₈) -Cycloalkoxycarbonyl, (C₂-C₁₂)-Alkenyloxycarbonyl, (C₂-C₁₂) -Alkinyloxycarbonyl, (C₁-C₁₂) -Alkylcarbonyloxy, (C₃-C₈) -Cycloalkylcarbonyloxy, (C₆-C₁₂) -Arylcarbonyloxy, (C₇-C₁₆) -Aralkylcarbonyloxy, Cinnamoyloxy, (C₂-C₁₂) -Alkenylcarbonyloxy, (C₂-C₁₂) - Alkinylcarbonyloxy, (C₁-C₁₂) -Alkoxycarbonyloxy, (C₁-C₁₂) - Alkoxy-(C₁-C₁₂)-alkoxycarbonyloxy, (C₆-C₁₂) - Aryloxycarbonyloxy, (C₇-C₁₆)-Aralkyloxycarbonyloxy, (C₃-C₈) -Cycloalkoxycarbonyloxy, (C₂-C₁₂) -Alkenyloxycarbonyloxy, (C₂-C₁₂)-Alkinyloxycarbonyloxy, Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₂) - Arylcarbamoyl, N-(C₇-C₁₆) -Aralkylcarbamoyl, N-(C₁-C₁₀)-Alkyl-N-(C₆-C₁₂)arylcarbamoyl, N-(C₁-C₁₀)-Alkyl-N-(C₇-C₁₆)-aralkylcarbamoyl, N-((C₁-C₁₀)-Alkoxy-(C₁-C₁₀) - alkyl) carbamoyl, N-((C₆-C₁₂)-Aryloxy-(C₁-C₁₀)-alkyl) - carbamoyl, N-((C₇-C₁₆)-Aralkyloxy-(C₁-C₁₀)-alkyl) - carbamoyl, N-(C₁-C₁₀)-Alkyl-N-((C₁-C₁₀)-alkoxy-(C₁-C₁₀)-alkyl) carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆) - aralkyloxy-(C₁-C₁₀)-alkyl)carbamoyl, Carbamoyloxy, N-(C₁-C₁₂)-Alkylcarbamoyloxy, N,N-Di-(C₁-C₁₂) -alkylcarbamoyloxy, N-(C₃-C₈) -Cycloalkylcarbamoyloxy, N-(C₆-C₁₂) -Arylcarbamoyloxy, N-(C₇-C₁₆) -Aralkylcarbamoyloxy, N-(C₁-C₁₀)-Alkyl-N- (C₆-C₁₂) -arylcarbamoyloxy, N-(C₁-C₁₀)-Alkyl-N-(C₇-C₁₆)-aralkylcarbamoyloxy, N-((C₁-C₁₀)-Alkyl) carbamoyloxy, N-((C₆-C₁₂)-Aryloxy-(C₁-C₁₀)-alkyl) carbamoyloxy, N-((C₇-C₁₆)-Aralkyloxy-(C₁-C₁₀) - alkyl) carbamoyloxy, N-(C₁-C₁₀) -Alkyl-N-((C₁-C₁₀)-alkoxy-(C₁-C₁₀)-alkyl) carbamoyloxy, N-(C₁-C₁₀)-Alkyl-N- ((C₆-C₁₂) - aryloxy-(C₁-C₁₀) -alkyl) carbamoyloxy, N-(C₁-C₁₀) -Alkyl-N-((C₇-C₁₆)-aralkyloxy-(C₁-C₁₀)-alkyl)carbamoyloxy, Amino, (C₁-C₁₂)-Alkylamino, Di-(C₁-C₁₂) -alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-Alkylaralkylamino, N-Alkylarylamino, (C₁-C₁₂)-Alkoxyamino, (C₁-C₁₂)-Alkoxy-N-(C₁-C₁₀)-alkylamino, (C₁-C₁₂) -Alkylcarbonylamino, (C₃-C₈) - Cycloalkylcarbonylamino, (C₆-C₁₂)-Arylcarbonylamino, (C₇-C₁₆)-Alkylcarbonylamino, (C₁-C₁₂) -Alkylcarbonyl-N-(C₁-C₁₀)-alkylamino, (C₃-C₈) -Cycloalkylcarbonyl-N- (C₁-C₁₀)-alkylamino, (C₆-C₁₂)-Arylcarbonyl-N-(C₁-C₁₀) - alkylamino, (C₇-C₁₁)-Aralkylcarbonyl-N-(C₁-C₁₀)- alkylamino, (C₁-C₁₂)-Alkylcarbonylamino-(C₁-C₈)-alkyl, (C₃-C₈) -Cycloalkylcarbonylamino-(C₁-C₈) -alkyl, (C₆-C₁₂) - Arylcarbonylamino-(C₁-C₈)-alkyl, (C₇-C₁₆) - Aralkylcarbonylamino-(C₁-C₈)-alkyl, Amino-(C₁-C₁₀)-alkyl, N-(C₁-C₁₀)-Alkylamino- (C₁-C₁₀)-alkyl, N,N-Di-(C₁-C₁₀)-alkylamino-(C₁-C₁₀)-alkyl, (C₃-C₈)-Cycloalkylamino-(C₁-C₁₀) -alkyl, (C₁-C₁₂) -Alkylmercapto, (C₁-C₁₂)-Alkylsulfinyl, (C₁-C₁₂)-Alkylsulfonyl, (C₆-C₁₂)-Arylmercapto, (C₆-C₁₂)-Arylsulfinyl, (C₆-C₁₂) - Arylsulfonyl, (C₇-C₁₆)-Aralkylmercapto, (C₇-C₁₆) - Aralkylsulfinyl oder (C₇-C₁₆)-Aralkylsulfonyl substituiert sein können;
X für O oder S steht;
Q für O, S, NR' oder eine Bindung steht;
wobei dann, wenn Q für eine Bindung steht, R⁴ für Halogen, Nitril oder Trifluormethyl steht;
oder wobei dann, wenn Q für O, S oder NR' steht, R⁴ für Wasserstoff, einen (C₁-C₁₀)-Alkylrest, einen (C₂-C₁₀)-Alkenylrest, einen (C₂-C₁₀)-Alkinylrest, wobei der Alkenyl- bzw. Alkinylrest eine oder zwei C-C-Mehrfachbindungen enthält; einen unsubstituierten Fluoralkylrest der Formel -[CH₂]ₓ-C_{f}H_{(2f+1-g)}-F_{g}, einen (C₁-C₈)-Alkoxy- (C₁-C₆)-alkylrest, einen (C₁-C₆)-Alkoxy-(C₁-C₄)-alkoxy-(C₁-C₄)-alkylrest, einen Arylrest, einen Heteroarylrest, einen (C₇-C₁₁)-Aralkylrest oder einen Rest der Formel Z
-[CH₂]ᵥ-[O]_{w}-[CH₂]ₜ-E (Z)
steht, wobei
E für einen Heteroarylrest, einen (C₃-C₈)-Cycloalkylrest oder einen Phenylrest der Formel F 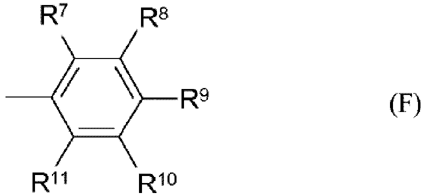 steht,
v für 0-6 steht,
w für 0 oder 1 steht,
t für 0-3 steht und
R⁷, R⁸, R⁹, R¹⁰ und R¹¹ gleich oder verschieden sind und für Wasserstoff, Halogen, Cyano, Nitro, Trifluormethyl, (C₁-C₆) -Alkyl, (C₃-C₈) -Cycloalkyl, (C₁-C₆) -Alkoxy, -O-[CH₂]ₓ-C_{f}H_{(2f+1-g)}-F_{g}, -OCF₂-Cl, -O-CF₂-CHFCl, (C₁-C₆)-Alkylmercapto, (C₁-C₆) -Hydroxyalkyl, (C₁-C₆) -Alkoxy-(C₁-C₆) -alkoxy, (C₁-C₆)-Alkoxy- (C₁-C₆)-alkyl, (C₁-C₆)-Alkylsulfinyl, (C₁-C₆) -Alkylsulfonyl, (C₁-C₆)-Alkylcarbonyl, (C₁-C₈) -Alkoxycarbonyl, Carbamoyl, N-(C₁-C₈)-Alkylcarbamoyl, N,N-Di-(C₁-C₈)-alkylcarbamoyl oder (C₇-C₁₁)-Aralkylcarbamoyl, gegebenenfalls substituiert durch Fluor, Chlor, Brom, Trifluormethyl, (C₁-C₆) -Alkoxy, N-(C₃-C₈)-Cycloalkylcarbamoyl, N-(C₃-C₈)-Cycloalkyl-(C₁-C₄) -alkylcarbamoyl, (C₁-C₆)-Alkylcarbonyloxy, Phenyl, Benzyl, Phenoxy, Benzyloxy, NR^{Y}R^{Z}, wobei R^{Y} und R^{z} unabhängig voneinander aus Wasserstoff, (C₁-C₁₂)-Alkyl, (C₁-C₈)-Alkoxy- (C₁-C₈)-alkyl, (C₇-C₁₂)-Aralkoxy- (C₁-C₈)-alkyl, (C₆-C₁₂)-Aryloxy-(C₁-C₈)-alkyl, (C₃-C₁₀)-Cycloalkyl, (C₃-C₁₂)-Alkenyl, (C₃-C₁₂)-Alkinyl, (C₆-C₁₂)-Aryl, (C₇-C₁₁)-Aralkyl, (C₁-C₁₂)-Alkoxy, (C₇-C₁₂)-Aralkoxy, (C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl ausgewählt sind; oder wobei weiterhin R^{Y} und R^{Z} zusammen für -[CH₂]ₕ stehen, wobei eine CH₂-Gruppe durch O, S, N-(C₁-C₄)-Alkylcarbonylimino oder N-(C₁-C₄)-Alkoxycarbonylimino ersetzt sein kann; Phenylmercapto, Phenylsulfonyl, Phenylsulfinyl, Sulfamoyl, N-(C₁-C₈)-Alkylsulfamoyl oder N,N-Di-(C₁-C₈)-alkylsulfamoyl stehen; oder alternativ dazu R⁷ und R⁸, R⁸ und R⁹, R⁹ und R¹⁰ oder R¹⁰ und R¹¹ zusammen für eine Kette, die aus -[CH₂]ₙ- oder -CH=CH-CH=CH- ausgewählt ist, stehen, wobei eine CH₂-Gruppe der Kette gegebenenfalls durch O, S, SO, SO₂ oder NR^{Y} ersetzt ist; und n für 3, 4 oder 5 steht; und dann, wenn E für einen Heteroarylrest steht, dieser Rest 1-3 aus den für R⁷-R¹¹ definierten Substituenten ausgewählte Substituenten tragen kann, oder dann, wenn E für einen Cycloalkylrest steht, der Rest einen aus den für R⁷-R¹¹ definierten Substituenten ausgewählten Substituenten tragen kann;
oder wobei dann, wenn Q für NR' steht, R⁴ alternativ dazu für R" steht, wobei R' und R" gleich oder verschieden sind und für Wasserstoff, (C₆-C₁₂)-Aryl, (C₇-C₁₁)-Aralkyl, (C₁-C₈)-Alkyl, (C₁-C₈)-Alkoxy- (C₁-C₈)-alkyl, (C₇-C₁₂)-Aralkoxy- (C₁-C₈)-alkyl, (C₆-C₁₂)-Aryloxy-(C₁-C₈)-alkyl, (C₁-C₁₀)-Alkylcarbonyl, gegebenenfalls substituiertes (C₇-C₁₆)-Aralkylcarbonyl oder gegebenenfalls substituiertes (C₆-C₁₂)-Arylcarbonyl stehen; oder R' und R" zusammen für -[CH₂]ₕ stehen, wobei eine CH₂-Gruppe durch O, S, N-Acylimino oder N-(C₁-C₁₀)-Alkoxycarbonylimino ersetzt sein kann, und h für 3 bis 7 steht;
Y für N oder CR³ steht;
R¹, R² und R³ gleich oder verschieden sind und für Wasserstoff, Hydroxy, Halogen, Cyano, Trifluormethyl, Nitro, Carboxyl, (C₁-C₂₀)-Alkyl, (C₃-C₈) -Cycloalkyl, (C₃-C₈) -Cycloalkyl- (C₁-C₁₂) -alkyl, (C₃-C₈) -Cycloalkoxy, (C₃-C₈) -Cycloalkyl- (C₁-C₁₂) -alkoxy, (C₃-C₈) -Cycloalkyloxy-(C₁-C₁₂) -alkyl, (C₃-C₈)-Cycloalkyloxy-(C₁-C₁₂)-alkoxy, (C₃-C₈) -Cycloalkyl- (C₁-C₈) -alkyl- (C₁-C₆) -alkoxy, (C₃-C₈) - Cycloalkyl- (C₁-C₈) -alkoxy- (C₁-C₆) -alkyl, (C₃-C₈) - Cycloalkyloxy-(C₁-C₈)-alkoxy-(C₁-C₆)-alkyl, (C₃-C₈) - Cycloalkoxy- (C₁-C₈) -alkoxy- (C₁-C₈) -alkoxy, (C₆-C₁₂) -Aryl, (C₇-C₁₆) -Aralkyl, (C₇-C₁₆) -Aralkenyl, (C₇-C₁₆) -Aralkinyl, (C₂-C₂₀)-Alkenyl, (C₂-C₂₀)-Alkinyl, (C₁-C₂₀)-Alkoxy, (C₂-C₂₀) -Alkenyloxy, (C₂-C₂₀) -Alkinyloxy, Retinyloxy, (C₁-C₂₀)-Alkoxy-(C₁-C₁₂)-alkyl, (C₁-C₁₂)-Alkoxy-(C₁-C₁₂) - alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy-(C₁-C₆)-alkoxy, (C₁-C₁₆) - Hydroxyalkyl, (C₆-C₁₆)-Aryloxy-(C₁-C₈) -alkyl, (C₇-C₁₆) - Aralkoxy- (C₁-C₈) -alkyl, (C₆-C₁₂) -Aryloxy- (C₁-C₈) -alkoxy-(C₁-C₆) -alkyl, (C₇-C₁₂) -Aralkyloxy- (C₁-C₈) -alkoxy- (C₁-C₆) - alkyl, (C₂-C₂₀)-Alkenyloxy-(C₁-C₆)-alkyl, (C₂-C₂₀) - Alkinyloxy-(C₁-C₆)-alkyl, Retinyloxy-(C₁-C₆)-alkyl, -O--[CH₂] ₓ-C_{f}H(_{2f+1-g}) Fg, -OCF₂Cl, -OCF₂-CHFCl, (C₁-C₂₀)- Alkylcarbonyl, (C₃-C₈) -Cycloalkylcarbonyl, (C₆-C₁₂)-Arylcarbonyl, (C₇-C₁₆) -Aralkylcarbonyl, Cinnamoyl, (C₂-C₂₀) -Alkenylcarbonyl, (C₂-C₂₀) -Alkinylcarbonyl, (C₁-C₂₀)-Alkoxycarbonyl, (C₁-C₁₂)-Alkoxy-(C₁-C₁₂) - alkoxycarbonyl, (C₆-C₁₂) -Aryloxycarbonyl, (C₇-C₁₆) - Aralkoxycarbonyl, (C₃-C₈) -Cycloalkoxycarbonyl, (C₂-C₂₀)-Alkenyloxycarbonyl, Retinyloxycarbonyl, (C₂-C₂₀)-Alkinyloxycarbonyl, (C₆-C₁₂) -Aryloxy- (C₁-C₆) - alkoxycarbonyl, (C₇-C₁₆)-Aralkoxy-(C₁-C₆)-alkoxycarbonyl, (C₃-C₈) -Cycloalkyl- (C₁-C₆) -alkoxycarbonyl, (C₃-C₈)-Cycloalkoxy- (C₁-C₆) -alkoxycarbonyl, (C₁-C₁₂) - Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂) -Alkenylcarbonyloxy, (C₂-C₁₂) - Alkinylcarbonyloxy, (C₁-C₁₂) -Alkoxycarbonyloxy, (C₁-C₁₂) - Alkoxy- (C₁-C₁₂) -alkoxycarbonyloxy, (C₆-C₁₂) - Aryloxycarbonyloxy, (C₇-C₁₆) -Aralkyloxycarbonyloxy, (C₃-C₈)-Cycloalkoxycarbonyloxy, (C₂-C₁₂)-Alkenyloxy-carbonyloxy, (C₂-C₁₂)-Alkinyloxycarbonyloxy, Carbamoyl, N- (C₁-C₁₂)-Alkylcarbamoyl, N,N-Di- (C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N- (C₃-C₈)-cycloalkylcarbamoyl, N- ( (C₃-C₈)-Cycloalkyl- (C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N- (C₁-C₆)-alkyl-N- (+)-dehydroabietylcarbamoyl, N-(C₆-C₁₂)-Arylcarbamoyl, N- (C₇-C₁₆)-Aralkylcarbamoyl, N- (C₁-C₁₀)-Alkyl-N- (C₆-C₁₆)-arylcarbamoyl, N- (C₁-C₁₀)-Alkyl-N-(C₇-C₁₆)-aralkylcarbamoyl, N-((C₁-C₈)-Alkoxy- (C₁-C₁₀)-alkyl) carbamoyl, N-((C₆-C₁₆)-Aryloxy-(C₁-C₁₀)-alkyl) carbamoyl, N-((C₇-C₁₆)-Aralkyloxy-(C₁-C₁₀-alkyl) carbamoyl, N-(C₁-C₁₀)-Alkyl-N-((C₁-C₁₀)-alkoxy-(C₁-C₁₀)-alkyl) carbamoyl, N- (C₁-C₁₀)-Alkyl-N-((C₆-C₁₂)-aryloxy- (C₁-C₁₀)-alkyl) carbamoyl, N- (C₁-C₁₀)-Alkyl-N-((C₇-C₁₆)-aralkyloxy-(C₁-C₁₀)-alkyl) carbamoyl; CON (CH₂) ₕ, wobei eine CH₂-Gruppe durch O, S, N- (C₁-C₈)-Alkylimino, N- (C₃-C₈)-Cycloalkylimino, N-(C₃-C₈)-Cycloalkyl- (C₁-C₄)-Alkylimino, N-(C₆-C₁₂)-Arylimino, N-(C₇-C₁₆)-Aralkylimino, N-(C₁-C₄)-Alkoxy- (C₁-C₆)-alkylimino ersetzt sein kann und h für 3 bis 7 steht; einen Carbamoylrest der Formel R 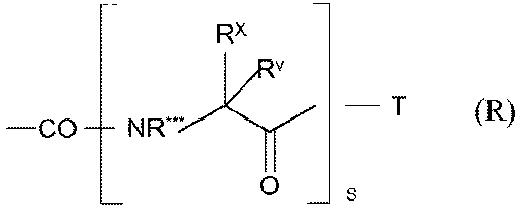 wobei
R^{x} und R^{v} jeweils unabhängig voneinander aus Wasserstoff, (C₁-C₆) -Alkyl, (C₃-C₇) -Cycloalkyl oder Aryl ausgewählt sind,
s für 1-5 steht,
T für OH oder NR*R** steht und R*, R** und R*** gleich oder verschieden sind und aus Wasserstoff, (C₆-C₁₂)-Aryl, (C₇-C₁₁) -Aralkyl, (C₁-C₈) -Alkyl, (C₃-C₈)-Cycloalkyl, (+)-Dehydroabietyl, (C₁-C₈)-Alkoxy- (C₁-C₈)-alkyl, (C₇-C₁₂)-Aralkoxy- (C₁-C₈)-alkyl, (C₆-C₁₂)-Aryloxy- (C₁-C₈)-alkyl, (C₁-C₁₀)-Alkanoyl, gegebenenfalls substituiertem (C₇-C₁₆)-Aralkanoyl, gegebenenfalls substituiertem (C₆-C₁₂)-Aroyl ausgewählt sind; oder R* und R** zusammen für - [CH_{2] h}, wobei eine CH₂-Gruppe durch O, S, SO, SO₂, N-Acylamino, N-(C₁-C₁₀)-Alkoxycarbonylimino, N- (C₁-C₈)-Alkylimino, N- (C₃-C₈)-Cycloalkylimino, N- (C₃-C₈)-Cycloalkyl- (C₁-C₄)-alkylimino, N- (C₆-C₁₂)-Arylimino, N- (C₇-C₁₆) -Aralkylimino, N- (C₁-C₄) -Alkoxy- (C₁-C₆) - alkylimino ersetzt sein kann und h für 3 bis 7 steht; Carbamoyloxy, N- (C₁-C₁₂)-Alkylcarbamoyloxy, N,N-Di-(C₁-C₁₂)-alkylcarbamoyloxy, N-(C₃-C₈)-Cycloalkyl-carbamoyloxy, N- (C₆-C₁₂)-Arylcarbamoyloxy, N- (C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-aralkyl-carbamoyloxy, N-((C₁-C₁₀)-Alkyl) carbamoyloxy, N-((C₆-C₁₂)-Aryloxy- (C₁-C₁₀)-alkyl) carbamoyloxy, N-((C₇-C₁₆)-Aralkyloxy- (C₁-C₁₀)-alkyl) carbamoyloxy, N- (C₁-C₁₀)-Alkyl-N-((C₁-C₁₀)-alkoxy- (C₁-C₁₀)-alkyl) carbamoyloxy, N- (C₁-C₁₀)-Alkyl-N-((C₆-C₁₂)-aryloxy-(C₁-C₁₀)-alkyl)carbamoyloxy, N-(C₁-C₁₀)-Alkyl-N-((C₇-C₁₆)-aralkyloxy- (C₁-C₁₀) - alkyl) carbamoyloxyamino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N- (C₆-C₁₂)-Arylamino, N-(C₇-C₁₁)-Aralkylamino, N-Alkylaralkylamino, N-Alkylarylamino, (C₁-C₁₂)-Alkoxyamino, (C₁-C₁₂)-Alkoxy-N-(C₁-C₁₀)-alkylamino, (C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N- (C₁-C₁₀)-alkylamino, (C₃-C₈)-Cycloalkanoyl-N-(C₁-C₁₀)-alkylamino, (C₆-C₁₂)-Aroyl-N- (C₁-C₁₀)-alkylamino, (C₇-C₁₁)-Aralkanoyl-N- (C₁-C₁₀)-alkylamino, (C₁-C₁₂)-Alkanoylamino- (C₁-C₈)-alkyl, (C₃-C₈)-Cycloalkanoylamino-(C₁-C₈)-alkyl, (C₆-C₁₂) - Aroylamino-(C₁-C₈)-alkyl, (C₇-C₁₆)-Aralkanoylamino- (C₁-C₈)-alkyl, Amino- (C₁-C₁₀)-alkyl, N- (C₁-C₁₀)-Alkylamino-(C₁-C₁₀)-alkyl, N,N-Di- (C₁-C₁₀)-alkylamino- (C₁-C₁₀)-alkyl, (C₃-C₈) -Cycloalkylamino- (C₁-C₁₀) -alkyl, (C₁-C₂₀) - Alkylmercapto, (C₁-C₂₀)-Alkylsulfinyl, (C₁-C₂₀)-Alkylsulfonyl, (C₆-C₁₂)-Arylmercapto, (C₆-C₁₂)-Arylsulfinyl, (C₆-C₁₂)-Arylsulfonyl, (C₇-C₁₆)-Aralkylmercapto, (C₇-C₁₆)-Aralkylsulfinyl, (C₇-C₁₆)-Aralkylsulfonyl, (C₁-C₁₂)-Alkylmercapto- (C₁-C₆)-alkyl, (C₁-C₁₂)-Alkylsulfinyl- (C₁-C₆)-alkyl, (C₁-C₁₂)-Alkylsulfonyl- (C₁-C₆)-alkyl, (C₆-C₁₂)-Arylmercapto- (C₁-C₆)-alkyl, (C₆-C₁₂)-Arylsulfinyl-(C₁-C₆)-alkyl, (C₆-C₁₂)-Arylsulfonyl- (C₁-C₆)-alkyl, (C₇-C₁₆)-Aralkylmercapto- (C₁-C₆)-alkyl, (C₇-C₁₆)-Aralkylsulfinyl-(C₁-C₆)-alkyl, (C₇-C₁₆)-Aralkylsulfonyl-(C₁-C₆)-alkyl, Sulfamoyl, N-(C₁-C₁₀)-Alkylsulfamoyl, N,N-Di-(C₁-C₁₀)-alkylsulfamoyl, (C₃-C₈)-Cycloalkylsulfamoyl, N- (C₆-C₁₂)-Arylsulfamoyl, N-(C₇-C₁₆)-Aralkylsulfamoyl, N- (C₁-C₁₀)-Alkyl-N- (C₆-C₁₂)-arylsulfamoyl, N- (C₁-C₁₀)-Alkyl-N- (C₇-C₁₆)-aralkylsulfamoyl, (C₁-C₁₀)-Alkylsulfonamido, N-((C₁-C₁₀)-Alkyl) - (C₁-C₁₀)-alkylsulfonamido, (C₇-C₁₆)-Aralkylsulfonamido und N- ((C₁-C₁₀)-Alkyl-(C₇-C₁₆)-aralkylsulfonamido; wobei ein Arylrest substituiert sein kann durch 1 bis 5 Substituenten ausgewählt aus Hydroxy, Halogen, Cyano, Trifluormethyl, Nitro, Carboxyl, (C₂-C₁₆)-Alkyl, (C₃-C₈)-Cycloalkyl, (C₃-C₈) - Cycloalkyl-(C₁-C₁₂)-alkyl, (C₃-C₈)-Cycloalkoxy, (C₃-C₈)-Cycloalkyl- (C₁-C₁₂)-alkoxy, (C₃-C₈)-Cycloalkyloxy- (C₁-C₁₂)-alkyl, (C₃-C₈)-Cycloalkyloxy- (C₁-C₁₂)-alkoxy, (C₃-C₈)-Cycloalkyl- (C₁-C₈)-alkyl- (C₁-C₆)-alkoxy, (C₃-C₈)-Cycloalkyl- (C₁-C₈)-alkoxy- (C₁-C₆)-alkyl, (C₃-C₈)-Cycloalkyloxy-(C₁-C₈)-alkoxy-(C₁-C₆)-alkyl, (C₃-C₈)-Cycloalkoxy-(C₁-C₈)-alkoxy- (C₁-C₈)-alkoxy, (C₆-C₁₂)-Aryl, (C₇-C₁₆)-Aralkyl, (C₂-C₁₆)-Alkenyl, (C₂-C₁₂)-Alkinyl, (C₁-C₁₆)-Alkoxy, (C₁-C₁₆)-Alkenyloxy, (C₁-C₁₂)-Alkoxy-(C₁-C₁₂)-alkyl, (C₁-C₁₂)-Alkoxy- (C₁-C₁₂)-alkoxy, (C₁-C₁₂)-Alkoxy-(C₁-C₈)-alkoxy-(C₁-C₈)-alkyl, (C₆-C₁₂)-Aryloxy, (C₇-C₁₆)-Aralkyloxy, (C₆-C₁₂)-Aryloxy-(C₁-C₆)-alkoxy, (C₇-C₁₆)-Aralkoxy- (C₁-C₆)-alkoxy, (C₁-C₈)-Hydroxyalkyl, (C₆-C₁₆)-Aryloxy-(C₁-C₈)-alkyl, (C₇-C₁₆)-Aralkoxy-(C₁-C₈)-alkyl, (C₆-C₁₂)-Aryloxy-(C₁-C₈)-alkoxy-(C₁-C₆)-alkyl, (C₇-C₁₂)-Aralkyloxy-(C₁-C₈)-alkoxy-(C₁-C₆)-alkyl, -O-[CH₂]ₓ-C_{f}H(_{2f+1-g})F_{g}, -OCF₂Cl, -OCF₂-CHFCl, (C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, (C₁-C₁₂)-Alkoxycarbonyl, (C₁-C₁₂)-Alkoxy- (C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₂-C₁₂)-Alkenyloxycarbonyl, (C₂-C₁₂)-Alkinyloxycarbonyl, (C₆-C₁₂)-Aryloxy-(C₁-C₆)-alkoxycarbonyl, (C₇-C₁₆)-Aralkoxy-(C₁-C₆)-alkoxycarbonyl, (C₃-C₈)-Cycloalkyl- (C₁-C₆)-alkoxycarbonyl, (C₃-C₈)-Cycloalkoxy-(C₁-C₆)-alkoxycarbonyl, (C₁-C₁₂) - Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₂-C₁₂)-Alkenylcarbonyloxy, (C₂-C₁₂)-Alkinylcarbonyloxy, (C₁-C₁₂)-Alkoxycarbonyloxy, (C₁-C₁₂)-Alkoxy- (C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₂-C₁₂)-Alkenyloxycarbonyloxy, (C₂-C₁₂) -Alkinyloxycarbonyloxy, Carbamoyl, N- (C₁-C₁₂)-Alkylcarbamoyl, N,N-Di- (C₁-C₁₂)-alkylcarbamoyl, N-(C₃-C₈)-Cycloalkylcarbamoyl, N,N-Dicyclo-(C₃-C₈)-alkylcarbamoyl, N-(C₁-C₁₀)-Alkyl-N- (C₃-C₈)-cycloalkylcarbamoyl, N-((C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietyloarbamoyl, N-(C₁-C₆)-Alkyl-N-(+)-dehydroabietylcarbamoyl, N-(C₆-C₁₂)-Arylcarbamoyl, N- (C₇-C₁₆)-Aralkylcarbamoyl, N- (C₁-C₁₀)-Alkyl-N-(C₆-C₁₆)-Arylcarbamoyl, N- (C₁-C₁₀)-Alkyl-N-(C₇-C₁₆)-aralkylcarbamoyl, N- ( (C₁-C₁₆)-Alkoxy- (C₁-C₁₀)-alkyl) carbamoyl, N-((C₆-C₁₆)-Aryloxy- (C₁-C₁₀)-alkyl) carbamoyl, N((C₇-C₁₆)-Aralkyloxy- (C₁-C₁₀)-alkyl) carbamoyl, N-(C₁-C₁₀)-Alkyl-N-((C₁-C₁₀)-alkoxy- (C₁-C₁₀)-alkyl) carbamoyl, N- (C₁-C₁₀)-Alkyl-N- ( (C₆-C₁₂)aryloxy-(C₁-C₁₀)-alkyl) carbamoyl, N-(C₁-C₁₀)-Alkyl-N-((C₇-C₁₆)-aralkyloxy- (C₁-C₁₀) -alkyl) carbamoyl, CON(CH₂)ₕ, wobei eine CH₂-Gruppe durch O, S, N- (C₁-C₈)-Alkylimino, N-(C₃-C₈)-Cycloalkylimino, N- (C₃-C₈)-Cycloalkyl- (C₁-C₄)-Alkylimino, N-(C₆-C₁₂)-Arylimino, N-(C₇-C₁₆)-Aralkylimino, N- (C₁-C₄)-Alkoxy- (C₁-C₆)-alkylimino ersetzt sein kann und h für 3 bis 7 steht; Carbamoyloxy, N-(C₁-C₁₂)-Alkylcarbamoyloxy, N,N-Di- (C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)- Aralkylcarbamoyloxy, N-(C₁-C₁₀)-Alkyl-N-(C₆-C₁₂) - arylcarbamoyloxy, N-(C₁-C₁₀)-Alkyl-N-(C₇-C₁₆) - aralkylcarbamoyloxy, N-((C₁-C₁₀)-Alkyl)carbamoyloxy, N-((C₆-C₁₂)-Aryloxy-(C₁-C₁₀)-alkyl) carbamoyloxy, N-((C₇-C₁₆) -Aralkyloxy- (C₁-C₁₀) -alkyl) carbamoyloxy, N-(C₁-C₁₀) - Alkyl-N- ((C₁-C₁₀)-alkoxy-(C₁-C₁₀)-alkyl)carbamoyloxy, N-(C₁-C₁₀)-Alkyl-N-((C₆-C₁₂)-aryloxy-(C₁-C₁₀) -alkyl) - carbamoyloxy, N-(C₁-C₁₀)-Alkyl-N-((C₇-C₁₆) -aralkyloxy- (C₁-C₁₀) -alkyl) carbamoyloxy, Amino, (C₁-C₁₂) -Alkylamino, Di-(C₁-C₁₂)-alkylamino, (C₃-C₈)-Cycloalkylamino, (C₃-C₁₂) - Alkenylamino, (C₃-C₁₂) -Alkinylamino, N- (C₆-C₁₂) - Arylamino, N-(C₇-C₁₁)-Aralkylamino, N-Alkylaralkylamino, N-Alkylarylamino, (C₁-C₁₂)-Alkoxyamino, (C₁-C₁₂)-Alkoxy-N-(C₁-C₁₀)-alkylamino, (C₁-C₁₂) -Alkanoylamino, (C₃-C₈) - Cycloalkanoylamino, (C₆-C₁₂)-Aroylamino, (C₇-C₁₆) - Aralkanoylamino, (C₁-C₁₂)-Alkanoyl-N-(C₁-C₁₀)-alkylamino, (C₃-C₈) -Cycloalkanoyl-N- (C₁-C₁₀) -alkylamino, (C₆-C₁₂) - Aroyl-N- (C₁-C₁₀) -alkylamino, (C₇-C₁₁) -Aralkanoyl-N- (C₁-C₁₀) -alkylamino, (C₁-C₁₂) -Alkanoylamino- (C₁-C₈) -alkyl, (C₃-C₈)-Cycloalkanoylamino-(C₁-C₈)-alkyl, (C₆-C₁₂) - Aroylamino-(C₁-C₈)-alkyl, (C₇-C₁₆)-Aralkanoylamino- (C₁-C₈)-alkyl, Amino- (C₁-C₁₀) -alkyl, N- (C₁-C₁₀) -Alkylamino-(C₁-C₁₀) -alkyl, N,N-Di- (C₁-C₁₀) -alkylamino- (C₁-C₁₀) -alkyl, (C₃-C₈)-Cycloalkylamino-(C₁-C₁₀)-alkyl, (C₁-C₁₂) - Alkylmercapto, (C₁-C₁₂) -Alkylsulfinyl, (C₁-C₁₂)-Alkylsulfonyl, (C₆-C₁₆) -Arylmercapto, (C₆-C₁₆) - Arylsulfinyl, (C₆-C₁₆) -Arylsulfonyl, (C₇-C₁₆) - Aralkylmercapto, (C₇-C₁₆) -Aralkylsulfinyl oder (C₇-C₁₆) - Aralkylsulfonyl stehen, stehen;
oder wobei R¹ und R² oder R² und R³ eine Kette [CH_{2]o} bilden, die gesättigt oder durch eine C=C-Doppelbindung ungesättigt ist, wobei 1 oder 2 CH₂-Gruppen gegebenenfalls durch O, S, SO, SO₂ oder NR' ersetzt sind und R' für Wasserstoff, (C₆-C₁₂)-Aryl, (C₁-C₈)-Alkyl, (C₁-C₈) -Alkoxy- (C₁-C₈) -alkyl, (C₇-C₁₂) -Aralkoxy- (C₁-C₈) - alkyl, (C₆-C₁₂) -Aryloxy- (C₁-C₈) -alkyl, (C₁-C₁₀) -Alkanoyl, gegebenenfalls substituiertes (C₇-C₁₆)-Aralkanoyl oder gegebenenfalls substituiertes (C₆-C₁₂)-Aroyl steht; und o für 3, 4 oder 5 steht;
oder wobei die Reste R¹ und R² oder R² und R³ zusammen mit dem Pyridin oder Pyridazin, das sie trägt, einen 5,6,7,8-Tetrahydroisochinolinring, einen 5,6,7,8-Tetrahydrochinolinring oder einen 5,6,7,8-Tetrahydrocinnolinring bilden;
oder wobei R¹ und R² oder R² und R³ einen carbocyclischen oder heterocyclischen 5- oder 6-gliedrigen aromatischen Ring bilden;
oder wobei R¹ und R² oder R² und R³ zusammen mit dem Pyridin oder Pyridazin, das sie trägt, ein gegebenenfalls substituiertes heterocyclisches Ringsystem, das aus Thienopyridinen, Furanopyridinen, Pyridopyridinen, Pyrimidinopyridinen, Imidazopyridinen, Thiazolopyridinen, Oxazolopyridinen, Chinolin, Isochinolin und Cinnolin ausgewählt ist, bilden;
oder wobei die Reste R¹ und R² zusammen mit dem Pyridin, das sie trägt, eine Verbindung der Formel Id bilden: 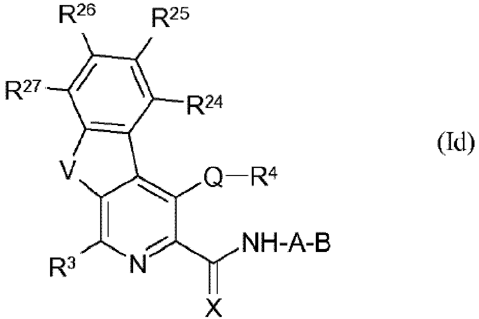
wobei V für S, O oder NR^{k} steht und R^{k} aus Wasserstoff, (C₁-C₆)-Alkyl, Aryl und Benzyl ausgewählt ist; wobei ein Arylrest gegebenenfalls durch 1 bis 5 wie oben definierte Substituenten substituiert sein kann; und
R^{24,} R^{25,} R²⁶ und R²⁷ jeweils unabhängig voneinander die Bedeutung von R¹, R² und R³ haben;
f für 1 bis 8 steht;
g für 0 oder 1 bis (2f+1) steht;
x für 0 bis 3 steht; und
h für 3 bis 7 steht;
einschließlich davon abgeleiteter physiologisch wirksamer Salze handelt.

2. Verwendung nach Anspruch 1 zur Verwendung nach diesem Anspruch, wobei die Verbindung für die orale Verabreichung bestimmt ist.

## Revendications

1. Composé qui inhibe une activité enzymatique de prolyl hydroxylase du HIF pour utilisation dans l'augmentation de l'érythropoïétine endogène dans le traitement prophylactique ou thérapeutique de l'anémie associée à une maladie rénale chez un sujet, où le composé est un composé de formule (I) : 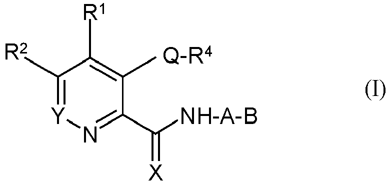 où
A représente un groupement alkylène en C₁-C₄ ;
B représente un groupement -CO₂H, -NH₂, -NHSO₂CF₃, tétrazolyle, imidazolyle, 3-hydroxyisoxazolyle, - CONHCOR"', -CONHSOR'", CONHSO₂R"', où R"' représente un groupement aryle, hétéroaryle, cycloalkyle en C₃-C₇ ou alkyle en C₁-C₄, éventuellement monosubstitué par aryle en C₆-C₁₂, hétéroaryle, OH, SH, alkyle en C₁-C₄, alcoxy en C₁-C₄, thioalkyle en C₁-C₄, sulfinyle en C₁-C₄, sulfonyle en C₁-C₄, CF₃, Cl, Br, F, I, NO₂, -COOH, (alcoxy en C₂-C₅) -carbonyle, NH₂, mono-(alkyle en C₁-C₄)-amino, di-(alkyle en C₁-C₄)-amino ou perfluoroalkyle en C₁-C₄ ; ou où B représente un radical carboxyle CO₂-G, où G représente un radical d'un alcool G-OH dans lequel G est choisi parmi un radical alkyle en C₁-C₂₀, un radical cycloalkyle en C₃-C₈, un radical alcényle en C₂-C₂₀, un radical cycloalcényle en C₃-C₈, un radical rétinyle, un radical alcynyle en C₂-C₂₀, un radical alcénynyle en C₄-C₂₀, où les radicaux alcényle, cycloalcényle, alcynyle et alcénynyle comportent une ou plusieurs liaisons multiples ; un radical aryle carbocyclique en C₆-C₁₆, un radical aralkyle carbocyclique en C₇-C₁₆, un radical hétéroaryle ou un radical hétéroaralkyle, où un radical hétéroaryle ou une entité hétéroaryle d'un radical hétéroaralkyle comporte 5 ou 6 chaînons ; et où les radicaux définis pour G sont substitués par un ou plusieurs groupements hydroxyle, halogène, cyano, trifluorométhyle, nitro, carboxyle, alkyle en C₁-C₁₂, cycloalkyle en C₃-C₈, cycloalcényle en C₅-C₈, aryle en C₆-C₁₂, aralkyle en C₇-C₁₆, alcényle en C₂-C₁₂, alcynyle en C₂-C₁₂, alcoxy en C₁-C₁₂, (alcoxy en C₁-C₁₂)-(alkyle en C₁-C₁₂), (alcoxy en C₁-C₁₂)-(alcoxy en C₁-C₁₂), aryloxy en C₆-C₁₂, aralkyloxy en C₇-C₁₆, hydroxyalkyle en C₁-C₈, -O-[CH₂]ₓ-C_{f}H(_{2f+1-g})-F_{g}, - OCF₂Cl, -OCF₂-CHFCl, (alkyle en C₁-C₁₂)-carbonyle, (cycloalkyle en C₃-C₈)-carbonyle, (aryle en C₆-C₁₂)-carbonyle, (aralkyle en C₇-C₁₆)-carbonyle, cinnamoyle, (alcényle en C₂-C₁₂)-carbonyle, (alcynyle en C₂-C₁₂)-carbonyle, (alcoxy en C₁-C₁₂)-carbonyle, (alcoxy en C₁-C₁₂)- (alcoxy en C₁-C₁₂)-carbonyle, (aryloxy en C₆-C₁₂)-carbonyle, (aralcoxy en C₇-C₁₆)-carbonyle, (cycloalcoxy en C₃-C₈)-carbonyle, (alcényloxy en C₂-C₁₂)-carbonyle, (alcynyloxy C₂-C₁₂)-carbonyle, acyloxy, (alcoxy en C₁-C₁₂)-carbonyloxy, (alcoxy en C₁-C₁₂)-(alcoxy en C₁-C₁₂)-carbonyloxy, (aryloxy en C₆-C₁₂)-carbonyloxy, (aralkyloxy en C₇-C₁₆)-carbonyloxy, (cycloalcoxy en C₃-C₈)-carbonyloxy, (alcényloxy en C₂-C₁₂)-carbonyloxy, (alcynyloxy en C₂-C₁₂)-carbonyloxy, carbamoyle, N-(alkyle en C₁-C₁₂)-carbamoyle, N,N-di-(alkyle en C₁-C₁₂)-carbamoyle, N-(cycloalkyle en C₃-C₈)-carbamoyle, N-(aryle en C₆-C₁₆)-carbamoyle, N-(aralkyle en C₇-C₁₆)-carbamoyle, N-(alkyle en C₁-C₁₀)-N-(aryle en C₆-C₁₆)-carbamoyle, N-(alkyle en C₁-C₁₀)-N-(aralkyle en C₇-C₁₆)-carbamoyle, N-((alcoxy en C₁-C₁₀)-(alkyle en C₁-C₁₀) )-carbamoyle, N-((aryloxy en C₆-C₁₂)-(alkyle en C₁-C₁₀))-carbamoyle, N-((aralkyloxy en C₇-C₁₆) - (alkyle en C₁-C₁₀))-carbamoyle, N-(alkyle en C₁-C₁₀)-N-((alcoxy en C₁-C₁₀)-(alkyle en C₁-C₁₀))-carbamoyle, N-(alkyle en C₁-C₁₀) - N-((aryloxy en C₆-C₁₆) - (alkyle en C₁-C₁₀)) -carbamoyle, N-(alkyle en C₁-C₁₀) -N-((aralkyloxy en C₇-C₁₆) - (alkyle en C₁-C₁₀))-carbamoyle, carbamoyloxy, N-(alkyle en C₁-C₁₂)-carbamoyloxy, N,N-di-(alkyle en C₁-C₁₂)-carbamoyloxy, N-(cycloalkyle en C₃-C₈)-carbamoyloxy, N-(aryle en C₆-C₁₂)-carbamoyloxy, N-(aralkyle en C₇-C₁₆) -carbamoyloxy, N-(alkyle en C₁-C₁₀) -N- (aryle en C₆-C₁₂) -carbamoyloxy, N-(alkyle en C₁-C₁₀)-N-(aralkyle en C₇-C₁₆)-carbamoyloxy, N-((alkyle en C₁-C₁₀)) -carbamoyloxy, N-((aryloxy en C₆-C₁₂)-(alkyle en C₁-C₁₀)) -carbamoyloxy, N-((aralkyloxy en C₇-C₁₆) - (alkyle en C₁-C₁₀)) -carbamoyloxy, N- (alkyle en C₁-C₁₀)-N-((alcoxy en C₁-C₁₀) - (alkyle en C₁-C₁₀))- carbamoyloxy, N-(alkyle en C₁-C₁₀)-N-((aryloxy en C₆-C₁₂)-(alkyle en C₁-C₁₀) ) -carbamoyloxy, N-(alkyle en C₁-C₁₀)-N-((aralkyloxy en C₇-C₁₆) - (alkyle en C₁-C₁₀))-carbamoyloxy, amino, (alkyle en C₁-C₁₂) -amino, di-(alkyle en C₁-C₁₂) -amino, (cycloalkyle en C₃-C₈) -amino, (alcényle en C₂-C₁₂)-amino, (alcynyle en C₂-C₁₂)-amino, N-(aryle en C₆-C₁₂)-amino, N- (aralkyle en C-C₁₁)-amino, N-alkyl-aralkylamino, N-alkyl-arylamino, (alcoxy en C₁-C₁₂)-amino, (alcoxy en C₁-C₁₂)-N-(alkyle en C₁-C₁₀)-amino, (alkyle en C₁-C₁₂) -carbonylamino, (cycloalkyle en C₃-C₈)-carbonylamino, (aryle en C₆-C₁₂) -carbonylamino, (aralkyle en C₇-C₁₆)-carbonylamino, (alkyle en C₁-C₁₂)-carbonyl-N- (alkyle en C₁-C₁₀) -amino, (cycloalkyle en C₃-C₈) -carbonyl-N- (alkyle en C₁-C₁₀) -amino, (aryle en C₆-C₁₂) -carbonyl-N- (alkyle en C₁-C₁₀) -amino, (aralkyle en C₇-C₁₁)-carbonyl-N- (alkyle en C₁-C₁₀)-amino, (alkyle en C₁-C₁₂) -carbonylamino- (alkyle en C₁-C₈), (cycloalkyle en C₃-C₈)-carbonylamino-(alkyle en C₁-C₈), (aryle en C₆-C₁₂)-carbonylamino- (alkyle en C₁-C₈), (aralkyle en C₇-C₁₂)-carbonylamino-(alkyle en C₁-C₈), amino-(alkyle en C₁-C₁₀), N-(alkyle en C₁-C₁₀) -amino- (alkyle en C₁-C₁₀), N,N-di-(alkyle en C₁-C₁₀) -amino- (alkyle en C₁-C₁₀), (cycloalkyle en C₃-C₈) -amino- (alkyle en C₁-C₁₀), (alkyle en C₁-C₁₂) -mercapto, (alkyle en C₁-C₁₂) -sulfinyle, (alkyle en C₁-C₁₂)-sulfonyle, (aryle en C₆-C₁₆)-mercapto, (aryle en C₆-C₁₆)-sulfinyle, (aryle en C₆-C₁₂)-sulfonyle, (aralkyle en C₇-C₁₆)-mercapto, (aralkyle en C₇-C₁₆)-sulfinyle, (aralkyle en C₇-C₁₆)-sulfonyle, sulfamoyle, N-(alkyle en C₁-C₁₀)-sulfamoyle, N,N-di- (alkyle en C₁-C₁₀)-sulfamoyle, (cycloalkyle en C₃-C₈)-sulfamoyle, N-(alkyle en C₆-C₁₂)-suifamoyle, N-(aralkyle en C₇-C₁₆)-sulfamoyle, N-(alkyle en C₁-C₁₀)-N-(aryle en C₆-C₁₂)-sulfamoyle, N-(alkyle en C₁-C₁₀)-N-(aralkyle en C₇-C₁₆)-sulfamoyle, (alkyle en C₁-C₁₀)-sulfonamido, N-((alkyle en C₁-C₁₀))-(alkyle en C₁-C₁₀)-sulfonamido, (aralkyle en C₇-C₁₆)-sulfonamido ou N-((alkyle en C₁-C₁₀)-(aralkyle en C₇-C₁₆)-sulfonamido ; où les radicaux qui sont des groupements aryle ou qui contiennent une entité aryle peuvent être substitués sur le groupement aryle par un à cinq groupements identiques ou différents hydroxyle, halogène, cyano, trifluorométhyle, nitro, carboxyle, alkyle en C₁-C₁₂, cycloalkyle en C₃-C₈, aryle en C₆-C₁₂, aralkyle en C₇-C₁₆, alcoxy en C₁-C₁₂, (alcoxy en C₁-C₁₂)-(alkyle en C₁-C₁₂), (alcoxy en C₁-C₁₂)-(alcoxy en C₁-C₁₂), aryloxy en C₆-C₁₂, aralkyloxy en C₇-C₁₆, hydroxyalkyle en C₁-C₈, (alkyle en C₁-C₁₂)-carbonyle, (cycloalkyle en C₃-C₈)-carbonyle, (aryle en C₆-C₁₂)-carbonyle, (aralkyle en C₇-C₁₆)-carbonyle, (alcoxy en C₁-C₁₂)-carbonyle, (alcoxy en C₁-C₁₂)-(alcoxy en C₁-C₁₂)-carbonyle, (aryloxy en C₆-C₁₂)-carbonyle, (aralcoxy en C₇-C₁₆)-carbonyle, (cycloalcoxy en C₃-C₈)-carbonyle, (alcényloxy en C₂-C₁₂)-carbonyle, (alcynyloxy en C₂-C₁₂)-carbonyle, (alkyle en C₁-C₁₂)-carbonyloxy, (cycloalkyle en C₃-C₈)-carbonyloxy, (aryle en C₆-C₁₂)-carbonyloxy, (aralkyle en C₇-C₁₆)-carbonyloxy, cinnamoyloxy, (alcényle en C₂-C₁₂)-carbonyloxy, (alcynyle en C₂-C₁₂)-carbonyloxy, (alcoxy en C₁-C₁₂)-carbonyloxy, (alcoxy en C₁-C₁₂)-(alcoxy en C₁-C₁₂)-carbonyloxy, (aryloxy en C₆-C₁₂)-carbonyloxy, (aralkyloxy en C₇-C₁₆)-carbonyloxy, (cycloalkyloxy en C₃-C₈)-carbonyloxy, (alcényloxy en C₂-C₁₂)-carbonyloxy, (alcynyloxy en C₂-C₁₂)-carbonyloxy, carbamoyle, N-(alkyle en C₁-C₁₂)-carbamoyle, N,N-di-(alkyle en C₁-C₁₂)-carbamoyle, N-(cycloalkyle en C₃-C₈)-carbamoyle, N-(aryle en C₆-C₁₂)-carbamoyle, N-(aralkyle en C₇-C₁₆) - carbamoyle, N-(alkyle en C₁-C₁₀)-N-(aryle en C₆-C₁₂)-carbamoyle, N-(alkyle en C₁-C₁₀)-N-(aralkyle en C₇-C₁₆) - carbamoyle, N-((alcoxy en C₁-C₁₀)-(alkyle en C₁-C₁₀))-carbamoyle, N-((aryloxy en C₆-C₁₂)-(alkyle en C₁-C₁₀))-carbamoyle, N-((aralkyloxy en C₇-C₁₆)-(alkyle en C₁-C₁₀))-carbamoyle, N-(alkyle en C₁-C₁₀)-N-((alcoxy en C₁-C₁₀)-(alkyle en C₁-C₁₀))-carbamoyle, N-(alkyle en C₁-C₁₀)-N-((aryloxy en C₆-C₁₂)-(alkyle en C₁-C₁₀))-carbamoyle, N-(alkyle en C₁-C₁₀)-N-((aralkyloxy en C₇-C₁₆)-(alkyle en C₁-C₁₀))-carbamoyle, carbamoyloxy, N-(alkyle en C₁-C₁₂)-carbamoyloxy, N,N-di-(alkyle en C₁-C₁₂)-carbamoyloxy, N-(cycloalkyle en C₃-C₈)-carbamoyloxy, N-(aryle en C₆-C₁₂)-carbamoyloxy, N-(aralkyle en C₇-C₁₆)-carbamoyloxy, N-(alkyle en C₁-C₁₀)-N-(aryle en C₆-C₁₂)-carbamoyloxy, N-(alkyle en C₁-C₁₀)-N-(aralkyle en C₇-C₁₆)-carbamoyloxy, N-((alkyle en C₁-C₁₀))-carbamoyloxy, N-((aryloxy en C₆-C₁₂)-(alkyle en C₁-C₁₀))-carbamoyloxy, N-((aralkyloxy en C₇-C₁₆)-(alkyle en C₁-C₁₀))-carbamoyloxy, N-(alkyle en C₁-C₁₀)-N-((alcoxy en C₁-C₁₀)-(alkyle en C₁-C₁₀))-carbamoyloxy, N-(alkyle en C₁-C₁₀)-N-((aryloxy en C₆-C₁₂)-(alkyle en C₁-C₁₀))-carbamoyloxy, N-(alkyle en C₁-C₁₀)-N-((aralkyloxy en C₇-C₁₆)- (alkyle en C₁-C₁₀))-carbamoyloxy, amino, (alkyle en C₁-C₁₂)-amino, di-(alkyle en C₁-C₁₂)-amino, (cycloalkyle en C₃-C₈)-amino, (alcényle en C₃-C₁₂)-amino, (alcynyle en C₃-C₁₂)-amino, N-(aryle en C₆-C₁₂)-amino, N-(aralkyle en C₇-C₁₁) -amino, N-alkylaralkylamino, N-alkyl-arylamino, (alcoxy en C₁-C₁₂)-amino, (alcoxy en C₁-C₁₂)-N-(alkyle en C₁-C₁₀)-amino, (alkyle en C₁-C₁₂)-carbonylamino, (cycloalkyle en C₃-C₈)-carbonylamino, (aryle en C₆-C₁₂)-carbonylamino, (alkyle en C₇-C₁₆)-carbonylamino, (alkyle en C₁-C₁₂)-carbonyl-N-(alkyle en C₁-C₁₀)-amino, (cycloalkyle en C₃-C₈)-carbonyl-N- (alkyle en C₁-C₁₀)-amino, (aryle en C₆-C₁₂)-carbonyl-N- (alkyle en C₁-C₁₀)-amino, (aralkyle en C₇-C₁₁)-carbonyl-N- (alkyle en C₁-C₁₀)-amino, (alkyle en C₁-C₁₂)-carbonylamino- (alkyle en C₁-C₈), (cycloalkyle en C₃-C₈)-carbonylamino- (alkyle en C₁-C₈), (aryle en C₆-C₁₂)-carbonylamino-(alkyle en C₁-C₈), (aralkyle en C₇-C₁₆)-carbonylamino-(alkyle en C₁-C₈), amino-(alkyle en C₁-C₁₀), N-(alkyle en C₁-C₁₀)-amino- (alkyle en C₁-C₁₀), N,N-di-(alkyle en C₁-C₁₀)-amino- (alkyle en C₁-C₁₀), (cycloalkyle en C₃-C₈)-amino- (alkyle en C₁-C₁₀), (alkyle en C₁-C₁₂)-mercapto, (alkyle en C₁-C₁₂)-sulfinyle, (alkyle en C₁-C₁₂)-sulfonyle, (aryle en C₆-C₁₂)-mercapto, (aryle en C₆-C₁₂)-sulfinyle, (aryle en C₆-C₁₂)-sulfonyle, (aralkyle en C₇-C₁₆)-mercapto, (aralkyle en C₇-C₁₆) - sulfinyle ou (aralkyle en C₇-C₁₆)-sulfonyle;
X représente O ou S ;
Q représente O, S, NR' ou une liaison ;
où, si Q représente une liaison, R⁴ représente un atome d'halogène ou un groupement nitrile ou trifluorométhyle ;
ou où, si Q représente O, S ou NR', R⁴ représente un atome d'hydrogène, un radical alkyle en C₁-C₁₀, un radical alcényle en C₂-C₁₀, un radical alcynyle en C₂-C₁₀, où le radical alcényle ou alcynyle comporte une ou deux liaisons multiples C-C ; un radical fluoroalkyle non substitué de la formule -[CH₂]ₓ-C_{f}H_{(2f+1-g)}-F_{g}, un radical (alcoxy en C₁-C₈)-(alkyle en C₁-C₆), un radical (alcoxy en C₁-C₆)-(alcoxy en C₁-C₄)-(alkyle en C₁-C₄), un radical aryle, un radical hétéroaryle, un radical aralkyle en C₇-C₁₁ ou un radical de la formule Z
-[CH₂]ᵥ-[O]_{w}-[CH₂]ₜ-E (Z)
où
E représente un radical hétéroaryle, un radical cycloalkyle en C₃-C₈ ou un radical phényle de la formule F 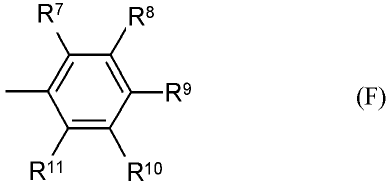
v est compris entre 0 et 6,
w est égal à 0 ou à 1,
t est compris entre 0 et 3, et
R⁷, R⁸, R⁹, R¹⁰ et R¹¹ sont identiques ou différents et représentent un atome d'hydrogène, d'halogène, un groupement cyano, nitro, trifluorométhyle, alkyle en C₁-C₆, cycloalkyle en C₃-C₈, alcoxy en C₁-C₆, -O-[CH₂]ₓ-C_{f}H_{(2f+1-g)}-F_{g}, -OCF₂-Cl, -O-CF₂-CHFCl, (alkyle en C₁-C₆)-mercapto, hydroxyalkyle en C₁-C₆, (alcoxy en C₁-C₆)-(alcoxy en C₁-C₆), (alcoxy en C₁-C₆)-(alkyle en C₁-C₆), (alkyle en C₁-C₆)-sulfinyle, (alkyle en C₁-C₆)-sulfonyle, (alkyle en C₁-C₆)-carbonyle, (alcoxy en C₁-C₈)-carbonyle, carbamoyle, N-(alkyle en C₁-C₈)-carbamoyle, N,N-di-(alkyle en C₁-C₈)-carbamoyle ou (aralkyle en C₇-C₁₁)-carbamoyle, éventuellement substitué par fluor, chlore, brome, trifluorométhyle, alcoxy en C₁-C₆, N-(cycloalkyle en C₃-C₈)-carbamoyle, N-(cycloalkyle en C₃-C₈)-(alkyle en C₁-C₄)-carbamoyle, (alkyle en C₁-C₆)-carbonyloxy, phényle, benzyle, phénoxy, benzyloxy, NR^{Y}R^{Z} où R^{y} et R^{z} sont indépendamment choisis parmi l'atome d'hydrogène, les groupements alkyle en C₁-C₁₂, (alcoxy en C₁-C₈)-(alkyle en C₁-C₈), (aralcoxy en C₇-C₁₂)-(alkyle en C₁-C₈), (aryloxy en C₆-C₁₂)-(alkyle en C₁-C₈), cycloalkyle en C₃-C10, alcényle en C₃-C₁₂, alcynyle en C₃-C₁₂, aryle en C₆-C12, aralkyle en C₇-C₁₁, alcoxy en C₁-C₁₂, aralcoxy en C₇-C₁₂, (alkyle en C₁-C₁₂)-carbonyle, (cycloalkyle en C₃-C₈)-carbonyle, (aryle en C₆-C₁₂)-carbonyle, (aralkyle en C₇-C₁₆)-carbonyle ; ou en outre, où R^{y} et R^{z} forment conjointement -[CH₂]ₕ, dans lequel un groupement CH₂ peut être remplacé par O, S, N-(alkyle en C₁-C₄)-carbonylimino ou N-(alcoxy en C₁-C₄)-carbonylimino; phénylmercapto, phénylsulfonyle, phénylsulfinyle, sulfamoyle, N-(alkyle en C₁-C₈)-sulfamoyle ou N,N-di-(alkyle en C₁-C₈)-sulfamoyle; ou de façon alternative, R⁷ et R⁸, R⁸ et R⁹, R⁹ et R¹⁰ ou R¹⁰ et R¹¹ forment conjointement une chaîne choisie parmi -[CH₂]ₙ- ou - CH=CH-CH=CH-, où un groupement CH₂ de la chaîne est éventuellement remplacé par O, S, SO, SO₂ ou NR^{Y}; et n est égal à 3, 4 ou 5 ; et si E représente un radical hétéroaryle, ledit radical peut porter 1 à 3 substituants choisis parmi ceux définis pour R⁷-R¹¹ ou si E représente un radical cycloalkyle, le radical peut porter un substituant choisi parmi ceux définis pour R⁷-R¹¹;
ou où, si Q représente NR', R⁴ représente alternativement R", où R' et R" sont identiques ou différents et représentent un atome d'hydrogène, un groupement aryle en C₆-C₁₂, aralkyle en C₇-C₁₁, alkyle en C₁-C₈, (alcoxy en C₁-C₈)-(alkyle en C₁-C₈), (aralcoxy en C₇-C₁₂)-(alkyle en C₁-C₈), (aryloxy en C₆-C₁₂)-(alkyle en C₁-C₈), (alkyle en C₁-C₁₀)-carbonyle, (aralkyle en C₇-C₁₆)-carbonyle éventuellement substitué ou (aryle en C₆-C₁₂)-carbonyle éventuellement substitué ; ou R' et R" forment conjointement -[CH₂]ₕ, dans lequel un groupement CH₂ peut être remplacé par O, S, N-acylimino ou N-(alcoxy en C₁-C₁₀)-carbonylimino, et h est compris entre 3 et 7 ;
Y représente N ou CR³;
R¹, R² et R³ sont identiques ou différents et représentent un atome d'hydrogène, groupement hydroxyle, halogène, cyano, trifluorométhyle, nitro, carboxyle, alkyle en C₁-C₂₀, cycloalkyle en C₃-C₈, (cycloalkyle en C₃-C₈)-(alkyle en C₁-C₁₂), cycloalcoxy en C₃-C₈, (cycloalkyle en C₃-C₈)-(alcoxy en C₁-C₁₂), (cycloalcoxy en C₃-C₈)-(alkyle en C₁-C₁₂), (cycloalcoxy en C₃-C₈)-(alcoxy en C₁-C₁₂), (cycloalkyle en C₃-C₈)-(alkyle en C₁-C₈)-(alcoxy en C₁-C₆), (cycloalkyle en C₃-C₈)-(alcoxy en C₁-C₈)-(alkyle en C₁-C₆), (cycloalcoxy en C₃-C₈)-(alcoxy en C₁-C₈)-(alkyle en C₁-C₆), (cycloalcoxy en C₃-C₈)-(alcoxy en C₁-C₈)-(alcoxy en C₁-C₈), aryle en C₆-C₁₂, aralkyle en C₇-C₁₆, aralcényle en C₇-C₁₆, aralcynyle en C₇-C₁₆, alcényle en C₂-C₂₀, alcynyle en C₂-C₂₀, alcoxy en C₁-C₂₀, alcényloxy en C₂-C₂₀, alcynyloxy en C₂-C₂₀, rétinyloxy, (alcoxy en C₁-C₂₀)-(alkyle en C₁-C₁₂), (alcoxy en C₁-C₁₂)-(alcoxy en C₁-C₁₂), (alcoxy en C₁-C₁₂)-(alcoxy en C₁-C₈)-(alkyle en C₁-C₈), aryloxy en C₆-C₁₂, aralkyloxy en C₇-C₁₆, (aryloxy en C₆-C₁₂)-(alcoxy en C₁-C₆), (aralcoxy en C₇-C₁₆) - (alcoxy en C₁-C₆), hydroxyalkyle en C₁-C₁₆, (aryloxy en C₆-C₁₆)-(alkyle en C₁-C₈), (aralcoxy en C₇-C₁₆)-(alkyle en C₁-C₈), (aryloxy en C₆-C₁₂)-(alcoxy en C₁-C₈)-(alkyle en C₁-C₆), (aralkyloxy en C₇-C₁₂)-(alcoxy en C₁-C₈)-(alkyle en C₁-C₆), (alcényloxy en C₂-C₂₀)-(alkyle en C₁-C₆), (alcynyloxy en C₂-C₂₀)-(alkyle en C₁-C₆), rétinyloxy-(alkyle en C₁-C₆), -O-[CH₂]ₓCfH_{(2f+1-g)}F_{g}, -OCF₂Cl, -OCF₂-CHFCl, (alkyle en C₁-C₂₀)-carbonyle, (cycloalkyle en C₃-C₈)-carbonyle, (aryle en C₆-C₁₂)-carbonyle, (aralkyle en C₇-C₁₆)-carbonyle, cinnamoyle, (alcényle en C₂-C₂₀)-carbonyle, (alcynyle en C₂-C₂₀)-carbonyle, (alcoxy en C₁-C₂₀)-carbonyle, (alcoxy en C₁-C₁₂)-(alcoxy en C₁-C₁₂)-carbonyle, (aryloxy en C₆-C₁₂)-carbonyle, (aralcoxy en C₇-C₁₆)-carbonyle, (cycloalcoxy en C₃-C₈)-carbonyle, (alcényloxy en C₂-C₂₀)-carbonyle, rétinyloxycarbonyle, (alcynyloxy en C₂-C₂₀)-carbonyle, (aryloxy en C₆-C₁₂)-(alcoxy en C₁-C₆)-carbonyle, (aralcoxy en C₇-C₁₆)-(alcoxy en C₁-C₆)-carbonyle, (cycloalkyle en C₃-C₈)-(alcoxy en C₁-C₆)-carbonyle, (cycloalcoxy en C₃-C₈)-(alcoxy en C₁-C₆)-carbonyle, (alkyle en C₁-C₁₂)-carbonyloxy, (cycloalkyle en C₃-C₈)-carbonyloxy, (aryle en C₆-C₁₂)-carbonyloxy, (aralkyle en C₇-C₁₆)-carbonyloxy, cinnamoyloxy, (alcényle en C₂-C₁₂)-carbonyloxy, (alcynyle en C₂-C₁₂)-carbonyloxy, (alcoxy en C₁-C₁₂)-carbonyloxy, (alcoxy en C₁-C₁₂)-(alcoxy en C₁-C₁₂)-carbonyloxy, (aryloxy en C₆-C₁₂)-carbonyloxy, (aralkyloxy en C₇-C₁₆)-carbonyloxy, (cycloalcoxy en C₃-C₈)-carbonyloxy, (alcényloxy en C₂-C₁₂)-carbonyloxy, (alcynyloxy en C₂-C₁₂)-carbonyloxy, carbamoyle, N-(alkyle en C₁-C₁₂)-carbamoyle, N,N-di-(alkyle en C₁-C₁₂)-carbamoyle, N-(cycloalkyle en C₃-C₈)-carbamoyle, N,N-di-(cycloalkyle en C₃-C₈)-carbamoyle, N-(alkyle en C₁-C₁₀)-N-(cycloalkyle en C₃-C₈)-carbamoyle, N-((cycloalkyle en C₃-C₈)-(alkyle en C₁-C₆))-carbamoyle, N-(alkyle en C₁-C₆)-N-((cycloalkyle en C₃-C₈)- (alkyle en C₁-C₆))-carbamoyle, N-(+)-déshydroabiétylcarbamoyle, N-(alkyle en C₁-C₆)-N-(+)-déshydroabiétylcarbamoyle, N-(aryle en C₆-C₁₂)-carbamoyle, N-(aralkyle en C₇-C₁₆)-carbamoyle, N-(alkyle en C₁-C₁₀)-N-(aryle en C₆-C₁₆)-carbamoyle, N-(alkyle en C₁-C₁₀)-N-(aralkyle en C₇-C₁₆)-carbamoyle, N-((alcoxy en C₁-C₁₈)-(alkyle en C₁-C₁₀))-carbamoyle, N-((aryloxy en C₆-C₁₆)-(alkyle en C₁-C₁₀))-carbamoyle, N-((aralkyloxy en C₇-C₁₆)-(alkyle en C₁-C₁₀))-carbamoyle, N-(alkyle en C₁-C₁₀)-N-((alcoxy en C₁-C₁₀)-(alkyle en C₁-C₁₀))-carbamoyle, N-(alkyle en C₁-C₁₀)-N-((aryloxy en C₆-C₁₂)-(alkyle en C₁-C₁₀))-carbamoyle, N-(alkyle en C₁-C₁₀)-N-((aralkyloxy en C₇-C₁₆)- (alkyle en C₁-C₁₀))-carbamoyle; CON (CH₂)ₕ, dans lequel un groupement CH₂ peut être remplacé par O, S, N-(alkyle en C₁-C₈)-imino, N-(cycloalkyle en C₃-C₈)-imino, N-(cycloalkyle en C₃-C₈)-(alkyle en C₁-C₄)-imino, N-(aryle en C₆-C₁₂)-imino, N-(aralkyle en C₇-C₁₆)-imino, N-(alcoxy en C₁-C₄)-(alkyle en C₁-C₆)-imino, et h est compris entre 3 et 7 ; un radical carbamoyle de la formule R 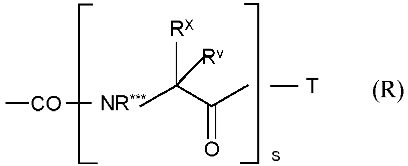 dans laquelle
R^{x} et R^{v} sont chacun indépendamment choisis parmi l'atome d'hydrogène, les groupements alkyle en C₁-C₆, cycloalkyle en C₃-C₇ ou aryle,
s est compris entre 1 et 5,
T représente OH ou NR*R**, et R*, R** et R*** sont identiques ou différents et sont choisis parmi l'atome d'hydrogène, les groupements aryle en C₆-C₁₂, aralkyle en C₇-C₁₁, alkyle en C₁-C₈, cycloalkyle en C₃-C₈, (+)-déshydroabiétyle, (alcoxy en C₁-C₈)-(alkyle en C₁-C₈), (aralcoxy en C₇-C₁₂)-(alkyle en C₁-C₈), (aryloxy en C₆-C₁₂)-(alkyle en C₁-C₈), alcanoyle en C₁-C₁₀, aralcanoyle en C₇-C₁₆ éventuellement substitué, aroyle en C₆-C₁₂ éventuellement substitué ; ou R* et R** forment conjointement -[CH₂]ₕ, dans lequel un groupement CH₂ peut être remplacé par O, S, SO, SO₂, N-acylamino, N-(alcoxy en C₁-C₁₀)-carbonylimino, N-(alkyle en C₁-C₈)-imino, N-(cycloalkyle en C₃-C₈)-imino, N-(cycloalkyle en C₃-C₈)-(alkyle en C₁-C₄)-imino, N-(aryle en C₆-C₁₂)-imino, N-(aralkyle en C₇-C₁₆)-imino, N-(alcoxy en C₁-C₄)-(alkyle en C₁-C₆)-imino, et h est compris entre 3 et 7 ; carbamoyloxy, N-(alkyle en C₁-C₁₂)-carbamoyloxy, N,N-di-(alkyle en C₁-C₁₂)-carbamoyloxy, N-(cycloalkyle en C₃-C₈)-carbamoyloxy, N-(aryle en C₆-C₁₂)-carbamoyloxy, N-(aralkyle en C₇-C₁₆)-carbamoyloxy, N- (alkyle en C₁-C₁₀)-N- (aryle en C₆-C₁₂)-carbamoyloxy, N- (alkyle en C₁-C₁₀)-N-(aralkyle en C₇-C₁₆)-carbamoyloxy, N-((alkyle en C₁-C₁₀))-carbamoyloxy, N-((aryloxy en C₆-C₁₂)-(alkyle en C₁-C₁₀))-carbamoyloxy, N-((aralkyloxy en C₇-C₁₆)-(alkyle en C₁-C₁₀))-carbamoyloxy, N-(alkyle en C₁-C₁₀)-N-((alcoxy en C₁-C₁₀)-(alkyle en C₁-C₁₀))-carbamoyloxy, N-(alkyle en C₁-C₁₀)-N-((aryloxy en C₆-C₁₂)- (alkyle en C₁-C₁₀))-carbamoyloxy, N-(alkyle en C₁-C₁₀)-N- ((aralkyloxy en C₇-C₁₆)-(alkyle en C₁-C₁₀))-carbamoyloxyamino, (alkyle en C₁-C₁₂)-amino, di-(alkyle en C₁-C₁₂)-amino, (cycloalkyle en C₃-C₈)-amino, (alcényle en C₃-C₁₂)-amino, (alcynyle en C₃-C₁₂)-amino, N-(aryle en C₆-C₁₂)-amino, N-(aralkyle en C₇-C₁₁)-amino, N-alkyl-aralkylamino, N-alkyl-arylamino, (alcoxy en C₁-C₁₂)-amino, (alcoxy en C₁-C₁₂)-N-(alkyle en C₁-C₁₀)-amino, (alcanoyle en C₁-C₁₂)-amino, (cycloalcanoyle en C₃-C₈)-amino, (aroyle en C₆-C₁₂)-amino, (aralcanoyle en C₇-C₁₆)-amino, (alcanoyle en C₁-C₁₂)-N-(alkyle en C₁-C₁₀)-amino, (cycloalcanoyle en C₃-C₈)-N-(alkyle en C₁-C₁₀)-amino, (aroyle en C₆-C₁₂)-N-(alkyle en C₁-C₁₀)-amino, (aralcanoyle en C₇-C₁₁)-N-(alkyle en C₁-C₁₀)-amino, (alcanoyle en C₁-C₁₂)-amino-(alkyle en C₁-C₆), (cycloalcanoyle en C₃-C₈)-amino-(alkyle en C₁-C₈), (aroyle en C₆-C₁₂)-amino- (alkyle en C₁-C₈), (aralcanoyle en C₇-C₁₆)-amino- (alkyle en C₁-C₈), amino-(alkyle en C₁-C₁₀), N-(alkyle en C₁-C₁₀)-amino-(alkyle en C₁-C₁₀), N,N-di-(alkyle en C₁-C₁₀)-amino-(alkyle en C₁-C₁₀), (cycloalkyle en C₃-C₈)-amino- (alkyle en C₁-C₁₀), (alkyle en C₁-C₂₀)-mercapto, (alkyle en C₁-C₂₀)-sulfinyle, (alkyle en C₁-C₂₀)-sulfonyle, (aryle en C₆-C₁₂)-mercapto, (aryle en C₆-C₁₂)-sulfinyle, (aryle en C₆-C₁₂)-sulfonyle, (aralkyle en C₇-C₁₆)-mercapto, (aralkyle en C₇-C₁₆)-sulfinyle, (aralkyle en C₇-C₁₆)-sulfonyle, (alkyle en C₁-C₁₂)-mercapto-(alkyle en C₁-C₆), (alkyle en C₁-C₁₂)-sulfinyl-(alkyle en C₁-C₆), (alkyle en C₁-C₁₂)-sulfonyl-(alkyle en C₁-C₆), (aryle en C₆-C₁₂)-mercapto-(alkyle en C₁-C₆), (aryle en C₆-C₁₂)-sulfinyl-(alkyle en C₁-C₆), (aryle en C₆-C₁₂)-sulfonyl-(alkyle en C₁-C₆), (aralkyle en C₇-C₁₆)-mercapto-(alkyle en C₁-C₆), (aralkyle en C₇-C₁₆)-sulfinyl-(alkyle en C₁-C₆), (aralkyle en C₇-C₁₆)-sulfonyl-(alkyle en C₁-C₆), sulfamoyle, N-(alkyle en C₁-C₁₀)-sulfamoyle, N,N-di-(alkyle en C₁-C₁₀)-sulfamoyle, (cycloalkyle en C₃-C₈)-sulfamoyle, N-(aryle en C₆-C₁₂)-sulfamoyie, N-(aralkyle en C₇-C₁₆)-sulfamoyle, N-(alkyle en C₁-C₁₀)-N-(aryle en C₆-C₁₂)-sulfamoyle, N-(alkyle en C₁-C₁₀)-N-(aralkyle en C₇-C₁₆)-sulfamoyle, (alkyle en C₁-C₁₀)-sulfonamido, N-((alkyle en C₁-C₁₀))-(alkyle en C₁-C₁₀)-sulfonamido, (aralkyle en C₇-C₁₆)-sulfonamido et N-((alkyle en C₁-C₁₀)-(aralkyle en C₇-C₁₆)-sulfonamido; où un radical aryle peut être substitué par 1 à 5 substituants choisis parmi les groupements hydroxyle, halogène, cyano, trifluorométhyle, nitro, carboxyle, alkyle en C₂-C₁₆, cycloalkyle en C₃-C₈, (cycloalkyle en C₃-C₈)-(alkyle en C₁-C₁₂), cycloalcoxy en C₃-C₈, (cycloalkyle en C₃-C₈)-(alcoxy en C₁-C₁₂), (cycloalkyloxy en C₃-C₈)-(alkyle en C₁-C₁₂), (cycloalkyloxy en C₃-C₈)-(alcoxy en C₁-C₁₂), (cycloalkyle en C₃-C₈)-(alkyle en C₁-C₆)-(alcoxy en C₁-C₆), (cycloalkyle en C₃-C₈)-(alcoxy en C₁-C₈)-(alkyle en C₁-C₆), (cycloalkyloxy en C₃-C₈)-(alcoxy en C₁-C₈)-(alkyle en C₁-C₆),(cycloalcoxy en C₃-C₈)-(alcoxy en C₁-C₈)-(alcoxy en C₁-C₈), aryle en C₆-C₁₂, aralkyle en C₇-C₁₆, alcényle en C₂-C₁₆, alcynyle en C₂-C₁₂, alcoxy en C₁-C₁₆, alcényloxy en C₁-C₁₆, (alcoxy en C₁-C₁₂)-(alkyle en C₁-C₁₂), (alcoxy en C₁-C₁₂)-(alcoxy en C₁-C₁₂), (alcoxy en C₁-C₁₂)-(alcoxy en C₁-C₈)-(alkyle en C₁-C₈), aryloxy en C₆-C₁₂, aralkyloxy en C₇-C₁₆, (aryloxy en C₆-C₁₂)-(alcoxy en C₁-C₆), (aralcoxy en C₇-C₁₆)-(alcoxy en C₁-C₆), hydroxyalkyle en C₁-C₈, (aryloxy en C₆-C₁₆)-(alkyle en C₁-C₈), (aralcoxy en C₇-C₁₆)-(alkyle en C₁-C₈), (aryloxy en C₆-C₁₂)-(alcoxy en C₁-C₈)-(alkyle en C₁-C₆), (aralkyloxy en C₇-C₁₂)-(alcoxy en C₁-C₈)-(alkyle en C₁-C₆), -O-[CH₂]ₓC_{f}H_{(2f+1-g)}F_{g}, -OCF₂Cl, -OCF₂-CHFCl, (alkyle en C₁-C₁₂)-carbonyle, (cycloalkyle en C₃-C₈)-carbonyle, (aryle en C₆-C₁₂)-carbonyle, (aralkyle en C₇-C₁₆)-carbonyle, (alcoxy en C₁-C₁₂)-carbonyle, (alcoxy en C₁-C₁₂)-(alcoxy en C₁-C₁₂)-carbonyle, (aryloxy en C₆-C₁₂)-carbonyle, (aralcoxy en C₇-C₁₆)-carbonyle, (cycloalcoxy en C₃-C₈)-carbonyle, (alcényloxy en C₂-C₁₂)-carbonyle, (alcynyloxy en C₂-C₁₂)-carbonyle, (aryloxy en C₆-C₁₂)-(alcoxy en C₁-C₆)-carbonyle, (aralcoxy en C₇-C₁₆)-(alcoxy en C₁-C₆)-carbonyle, (cycloalkyle en C₃-C₈)-(alcoxy en C₁-C₆) -carbonyle, (cycloalcoxy en C₃-C₈) -(alcoxy en C₁-C₆)-carbonyle, (alkyle en C₁-C₁₂)-carbonyloxy, (cycloalkyle en C₃-C₈) -carbonyloxy, (aryle en C₆-C₁₂)-carbonyloxy, (aralkyle en C₇-C₁₆) -carbonyloxy, cinnamoyloxy, (alcényle en C₂-C₁₂) -carbonyloxy, (alcynyle en C₂-C₁₂)-carbonyloxy, (alcoxy en C₁-C₁₂)-carbonyloxy, (alcoxy en C₁-C₁₂)-(alcoxy en C₁-C₁₂)-carbonyloxy, (aryloxy en C₆-C₁₂)-carbonyloxy, (aralkyloxy en C₇-C₁₆) -carbonyloxy, (cycloalcoxy en C₃-C₈)-carbonyloxy, (alcényloxy en C₂-C₁₂) -carbonyloxy, (alcynyloxy en C₂-C₁₂) -carbonyloxy, carbamoyle, N-(alkyle en C₁-C₁₂) -carbamoyle, N,N-di-(alkyle en C₁-C₁₂)-carbamoyle, N-(cycloalkyle en C₃-C₈)-carbamoyle, N,N-di-(cycloalkyle en C₃-C₈) -carbamoyle, N-(alkyle en C₁-C₁₀)-N-(cycloalkyle en C₃-C₈)-carbamoyle, N-((cycloalkyle en C₃-C₈)-(alkyle en C₁-C₆)) carbamoyle, N-(alkyle en C₁-C₆)-N-((cycloalkyle en C₃-C₈) -(alkyle en C₁-C₆)) carbamoyle, N-(+)-déshydroabiétylcarbamoyle, N-(alkyle en C₁-C₆) -N-(+)-déshydroabiétylcarbamoyle, N-(aryle en C₆-C₁₂)-carbamoyle, N-(aralkyle en C₇-C₁₆)-carbamoyle, N-(alkyle en C₁-C₁₀)-N-(aryle en C₆-C₁₆)-carbamoyle, N-(alkyle en C₁-C₁₀)-N-(aralkyle en C₇-C₁₆) -carbamoyle, N-((alcoxy en C₁-C₁₆)-(alkyle en C₁-C₁₀)) carbamoyle, N-((aryloxy en C₆-C₁₆)-(alkyle en C₁-C₁₀)) carbamoyle, N-((aralkyloxy en C₇-C₁₆)-(alkyle en C₁-C₁₀)) carbamoyle, N-(alkyle en C₁-C₁₀)-N-((alcoxy en C₁-C₁₀)-(alkyle en C₁-C₁₀)) carbamoyle, N-(alkyle en C₁-C₁₀)-N-((aryloxy en C₆-C₁₂) -(alkyle en C₁-C₁₀)) carbamoyle, N-(alkyle en C₁-C₁₀)-N-((aralkyloxy en C₇-C₁₆) -(alkyle en C₁-C₁₀))-carbamoyle, CON(CH₂)ₕ, dans lequel un groupement CH₂ peut être remplacé par O, S, N-(alkyle en C₁-C₈)-imino, N-(cycloalkyle en C₃-C₈)-imino, N-(cycloalkyle en C₃-C₈)-(alkyle en C₁-C₄)-imino, N-(aryle en C₆-C₁₂)-imino, N-(aralkyle en C₇-C₁₆)-imino, N-(alcoxy en C₁-C₄)-(alkyle en C₁-C₆)-imino, et h est compris entre 3 et 7 ; carbamoyloxy, N-(alkyle en C₁-C₁₂)-carbamoyloxy, N,N-di-(alkyle en C₁-C₁₂) - carbamoyloxy, N-(cycloalkyle en C₃-C₈)-carbamoyloxy, N-(aryle en C₆-C₁₆)-carbamoyloxy, N-(aralkyle en C₇-C₁₆)-carbamoyloxy, N-(alkyle en C₁-C₁₀)-N-(aryle en C₆-C₁₂)-carbamoyloxy, N-(alkyle en C₁-C₁₀)-N-(aralkyle en C₇-C₁₆)-carbamoyloxy, N-((alkyle en C₁-C₁₀))-carbamoyloxy, N-((aryloxy en C₆-C₁₂)-(alkyle en C₁-C₁₀))-carbamoyloxy, N-((aralkyloxy en C₇-C₁₆)- (alkyle en C₁-C₁₀))-carbamoyloxy, N-(alkyle en C₁-C₁₀)-N-((alcoxy en C₁-C₁₀)-(alkyle en C₁-C₁₀))-carbamoyloxy, N-(alkyle en C₁-C₁₀)-N-((aryloxy en C₆-C₁₂)-(alkyle en C₁-C₁₀))-carbamoyloxy, N-(alkyle en C₁-C₁₀)-N-((aralkyloxy en C₇-C₁₆)-(alkyle en C₁-C₁₀))-carbamoyloxy, amino, (alkyle en C₁-C₁₂)-amino, di-(alkyle en C₁-C₁₂)-amino, (cycloalkyle en C₃-C₈)-amino, (alcényle en C₃-C₁₂)-amino, (alcynyle en C₃-C₁₂)-amino, N-(aryle en C₆-C₁₂)-amino, N-(aralkyle en C₇-C₁₁)-amino, N-alkyl-aralkylamino, N-alkyl-arylamino, (alcoxy en C₁-C₁₂)-amino, (alcoxy en C₁-C₁₂)-N-(alkyle en C₁-C₁₀)-amino, (alcanoyle en C₁-C₁₂)-amino, (cycloalcanoyle en C₃-C₈)-amino, (aroyle en C₆-C₁₂)-amino, (aralcanoyle en C₇-C₁₆)-amino, (alcanoyle en C₁-C₁₂)-N-(alkyle en C₁-C₁₀)-amino, (cycloalcanoyle en C₃-C₈)-N-(alkyle en C₁-C₁₀)-amino, (aroyle en C₆-C₁₂)-N-(alkyle en C₁-C₁₀)-amino, (aralcanoyle en C₇-C₁₁)-N-(alkyle en C₁-C₁₀)-amino, (alcanoyle en C₁-C₁₂)-amino- (alkyle en C₁-C₈), (cycloalcanoyle en C₃-C₈)-amino- (alkyle en C₁-C₈), (aroyle en C₆-C₁₂)-amino- (alkyle en C₁-C₈), (aralcanoyle en C₇-C₁₆)-amino- (alkyle en C₁-C₈), amino-(alkyle en C₁-C₁₀), N-(alkyle en C₁-C₁₀)-amino- (alkyle en C₁-C₁₀), N,N-di-(alkyle en C₁-C₁₀)-amino- (alkyle en C₁-C₁₀), (cycloalkyle en C₃-C₈)-amino- (alkyle en C₁-C₁₀), (alkyle en C₁-C₁₂)-mercapto, (alkyle en C₁-C₁₂)-sulfinyle, (alkyle en C₁-C₁₂)-sulfonyle, (aryle en C₆-C₁₆)-mercapto, (aryle en C₆-C₁₆)-sulfinyle, (aryle en C₆-C₁₆)-sulfonyle, (aralkyle en C₇-C₁₆)-mercapto, (aralkyle en C₇-C₁₆)-sulfinyle ou (aralkyle en C₇-C₁₆)-sulfonyle ;
ou où R¹ et R², ou R² et R³ forment une chaîne [CH₂]ₒ, qui est saturée ou insaturée par une double liaison C=C, dans laquelle 1 ou 2 groupements CH₂ sont éventuellement remplacés par O, S, SO, SO₂ ou NR', et R' représente un atome d'hydrogène, un groupement aryle en C₆-C₁₂, alkyle en C₁-C₈, (alcoxy en C₁-C₈)-(alkyle en C₁-C₈), (aralcoxy en C₇-C₁₂)-(alkyle en C₁-C₈), (aryloxy en C₆-C₁₂) -(alkyle en C₁-C₈), alcanoyle en C₁-C₁₀, aralcanoyle en C₇-C₁₆ éventuellement substitué ou aroyle en C₆-C₁₂ éventuellement substitué ; et o représente 3, 4 ou 5 ;
ou où les radicaux R¹ et R², ou R² et R³, forment conjointement avec le groupement pyridine ou pyridazine qui les porte un cycle 5,6,7,8-tétrahydroisoquinoléine, un cycle 5,6,7,8-tétrahydroquinoléine ou un cycle 5,6,7,8-tétrahydrocinnoline ;
ou où R¹ et R², ou R² et R³ forment un cycle aromatique carbocyclique ou hétérocyclique comportant 5 ou 6 chaînons ;
ou où R¹ et R², ou R² et R³, forment conjointement avec le groupement pyridine ou pyridazine qui les porte un système cyclique hétérocyclique éventuellement substitué choisi parmi thiénopyridines, furanopyridines, pyridopyridines, pyrimidinopyridines, imidazopyridines, thiazolopyridines, oxazolopyridines, quinoléine, isoquinoléine et cinnoline ;
ou où les radicaux R¹ et R² forment conjointement avec le groupement pyridine qui les porte un composé de Formule Id : 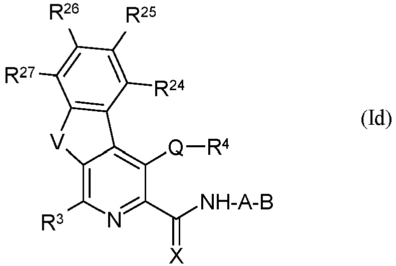
où V représente S, O ou NR^{k}, et R^{k} est choisi parmi l'atome d'hydrogène, les groupements alkyle en C₁-C₆, aryle ou benzyle ; où un radical aryle peut éventuellement être substitué par 1 à 5 substituants comme défini ci-avant ; et
R²⁴, R²⁵, R²⁶ et R²⁷ prennent dans chaque cas indépendamment les uns des autres les valeurs de R¹, R² et R³ ;
f est compris entre 1 et 8 ;
g est égal à 0 ou est compris entre 1 et (2f + 1) ;
x est compris entre 0 et 3 ; et
h est compris entre 3 et 7 ;
y compris les sels physiologiquement actifs de celuici .

2. Composé selon la revendication 1 pour l'utilisation selon cette revendication, où le composé est destiné à une administration orale.
